# EUROPÄISCHE PATENTSCHRIFT

(11) **EP 0 673 931 B1**
(45) Veröffentlichungstag und Bekanntmachung des Hinweises auf die Patenterteilung: **13.08.1997**
(21) Anmeldenummer: 95103581.5
(22) Anmeldetag: 13.03.1995
(51) Int. Cl.: C07D 213/82, A61K 31/44

(54) **Sulfonamidocarbonylpyridin-2-carbonsäureamide sowie ihre Pyridin-N-oxide, Verfahren zu ihrer Herstellung und ihre Verwendung als Arzneimittel**
Sulfonamidocarbonyl-pyridine-2-carboxamides and their pyridine-N-oxides, process for their preparation and their use as medicaments
Sulfonamidocarbonyl-pyridine-2-carboxamides et leur pyridine-N-oxides, procédé pour leur préparation et leur utilisation comme médicament

(30) Priorität: 25.03.1994 DE 4410423
(43) Veröffentlichungstag der Anmeldung: 27.09.1995
(73) Patentinhaber: HOECHST AKTIENGESELLSCHAFT, 65929 Frankfurt am Main (DE)
(72) Erfinder: Weidmann, Klaus, Dr., D-61476 Kronberg (DE); Bickel, Martin, Dr., D-61348 Bad Homburg (DE); Günzler-Pukall, Volkmar, Dr., D-35039 Marburg (DE)

(56) Entgegenhaltungen:
- EP-A- 0 562 512
- Analytical Biochemistry 184,p291-297

## Beschreibung

Die Erfindung betrifft Sulfonamidocarbonylpyridin-2-carbonsäureamide und ihre Pyridin-N-oxide sowie ihre Verwendung als Arzneimittel gegen fibrotische Erkrankungen.

Verbindungen, die die Enzyme Prolin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird proteingebundenes Prolin oder Lysin durch die Enzyme Prolyl- bzw. Lysylhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazallulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Inhibitoren der Prolylhydroxylase sind deshalb geeignete Substanzen in der Therapie von Erkrankungen, in denen die Ablagerung von Kollagenen maßgeblich zum Krankheitsbild beiträgt. Hierzu gehören u. a. Fibrosen der Lunge, Leber und Haut (Skleroderma) sowie die Atherosklerose.

Es ist bekannt, daß das Enzym Prolylhydroxylase durch Pyridin-2,4- und -2,5-dicarbonsäure effektiv gehemmt wird (K. Majamaa et al., Eur. J. Biochem. 138(1984) 239-245). Diese Verbindungen sind in der Zellkultur allerdings nur in sehr hohen Konzentrationen als Hemmstoffe wirksam (Tschank, G. et al., Biochem. J. 238 (1987) 625-633).

In der DE-A 34 32 094 werden Pyridin-2,4- und -2,5-dicarbonsäurediester mit 1-6 C-Atomen im Esteralkylteil als Arzneimittel zur Inhibierung der Prolyl- und Lysylhydroxylase beschrieben.

Diese niedrig-alkylierten Diester haben jedoch den Nachteil, daß sie zu schnell im Organismus zu den Säuren gespalten werden und nicht in genügend hoher Konzentration an ihren Wirkort in der Zelle gelangen und damit für eine eventuelle Verabreichung als Arzneimittel weniger geeignet sind.

Die DE-A 37 03 959, DE-A 37 03 962 und DE-A 37 03 963 beschreiben in allgemeiner Form gemischte Ester/Amide, höher alkylierte Diester und Diamide der Pyridin-2,4- und -2,5-dicarbonsäure, die die Kollagenbiosynthese im Tiermodell wirksam hemmen.

In der EP-A-0 562 512 sind Verbindungen beschrieben, die aufgrund der Hemmung der Prolylhydroxylase antifibrotische Wirkung haben.

In der gleichzeitig eingereichten deutschen Anmeldung P 44 10 480.4 sind die Ester-Prodrugs der entsprechenden Carbonsäuren der Formel I beschrieben.

Es bestand nunmehr die Aufgabe nach Verbindungen zu suchen, die noch stärker die Prolylhydroxylase hemmen als die bisher bekannten Verbindungen und damit zu einer stärkeren Hemmung der Kollagenbiosynthese führen.

Gelöst wird die Aufgabe durch das Bereitstellen von Sulfonamidocarbonylpyridin-2-carbonsäureamiden sowie ihrer Pyridin-N-oxide der allgemeinen Formel I 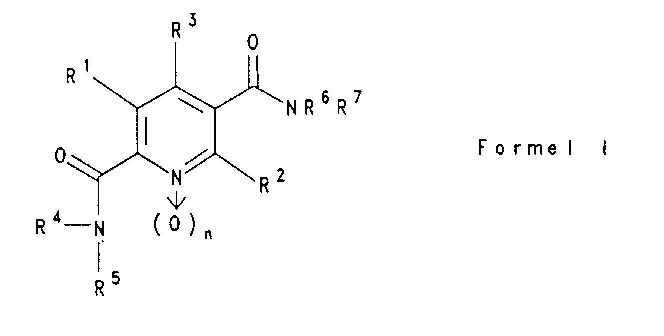 worin
- R¹: Hydroxy oder (C₁-C₆)-Alkoxy,
- R² und R³: gleich oder verschieden sind und Wasserstoff, unsubstituiertes oder substituiertes (C₁-C₆)-Alkyl, (C₁-C₆)-Alkoxy, Halogen, insbesondere Fluor, Chlor oder Brom, Nitril, Hydroxy, Amino,
- R⁶: Wasserstoff, (C₁-C₆)-Alkyl oder eine N-Schutzgruppe wie (C₁-C₈)-Alkanoyl, (C₁-C₆)-Alkylcarbamoyl, (C₁-C₆)-Alkoxycarbonyl, Benzyloxycarbonyl, (C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl oder (C₁-C₆)-Alkoxycarbonyloxy-(C₁-C₆)-alkyl, ein 1, 2, 3 oder 4-wertiges physiologisch verwendbares Kation, insbesondere Na⊕, K⊕, Mg²⊕, Ca²⊕, Al³⊕ oder ein Ammoniumion, ggf. 1-3fach substituiert mit (C₁-C₈)-Alkyl, (C₁-C₈)-Hydroxyalkyl, (C₁-C₄)-Alkoxy-(C₁-C₈)-alkyl, Phenyl, Benzyl oder (C₁-C₈)-Alkyl, welches 1 bis 3-fach substituiert sein kann mit Hydroxy oder (C₁-C₄)-Alkoxy, oder ein Kation eines basischen Aminosäurederivates,
- R⁷: einen Rest der Formel II, ausgenommen -SO₂H, bedeutet

-Y-[C-U]ᵣ-D-W (II)

in welchem
Y -SO₂- oder -CO- bedeutet,
C eine Bindung oder
einen verzweigten oder unverzweigten aliphatischen (C₁-C₁₆)-Alkandiyl oder cycloaliphatischen (C₃-C₁₀)-Alkandiylrest oder einen verzweigten oder unverzweigten (C₂-C₁₆)-Alkendiyl- oder Cycloalkendiyl-Rest, oder einen (C₂-C₁₆)-Alkindiyl-Rest oder einen (C₂-C₁₆)-Alkenindiyl-Rest bedeutet, der jeweils eine oder mehrere C-C-Mehrfachbindungen enthalten kann,
U eine Bindung oder
Wasserstoff oder
einen Rest aus der Reihe folgender Heteroatomgruppierungen -CO-, -O(CO)-, -(CO)-O-, -(CO)NR-, -NR(CO)-, -O-, -SO-, -SO₂-, -NR, worin R (C₁-C₃)-Alkyl, (C₁-C₈)-Alkanoyl, (C₇-C₁₆)-Aralkanoyl, (C₆-C₁₂)-Aroyl, oder Wasserstoff bedeutet,
r 1, 2, 3 oder 4 ist,
D eine Bindung oder Wasserstoff oder
einen verzweigten oder unverzweigten aliphatischen (C₁-C₁₀)-Alkandiyl-Rest, oder
einen verzweigten oder unverzweigten (C₁-C₁₀)-Alkendiyl-Rest, einen (C₂-C₁₀)-Alkindiyl-Rest oder einen (C₂-C₁₀)-Alkenindiyl-Rest, der jeweils eine oder mehrere C-C-Mehrfachbindungen enthalten kann,
W eine Bindung oder
Wasserstoff oder
einen (C₃-C₁₀) cycloaliphatischen Alkyl-, Alkenyl-, Alkinyl- oder Alkeninyl-Rest oder
einen (C₆-C₁₆)-Arylrest oder einen 5- oder 6-gliedrigen Heteroarylrest, wobei mindestens eine der Variablen C oder D oder W nicht eine Bindung bedeuten und U nur dann in der Bedeutung einer Heteratomgruppierung steht, wenn C nicht eine Bindung bedeutet oder wenn D und/oder W nicht eine Bindung bedeuten und
C und/oder W, sofern diese keine Bindung oder Wasserstoff bedeuten und vorzugsweise ihrerseits substituiert sind,
- R⁴: Wasserstoff, (C₁-C₆)-Alkyl, Phenyl, Phenyl-(C₁-C₄)-alkyl,
- R⁵: einen unsubstituierten oder substituierten, verzweigten oder unverzweigten, aliphatischen (C₁-C₈)-Alkylrest bedeutet, der eine saure Gruppe, insbesondere aus der Reihe -CO₂H, -CONHCOR‴, -CONHSOR‴, CONHSO₂R''', -NHSO₂CF₃, Tetrazolyl, Imidazolyl oder 3-Hydroxyisoxazolyl trägt, wobei R''' Aryl, Heteroaryl, (C₃-C₇)-Cycloalkyl oder (C₁-C₄)-Alkyl, gegebenenfalls monosubstituiert mit (C₆-C₁₂)-Aryl, Heteroaryl, OH, SH, (C₁-C₄)-Alkyl, (C₁-C₄)-Alkoxy, (C₁-C₄)-Thioalkyl, -Sulfinyl oder -Sulfonyl, CF₃, Cl, Br, F, I, NO₂, -COOH, (C₂-C₅)-Alkoxycarbonyl, NH₂, Mono- oder Di-(C₁-C₄-alkyl)-amino oder (C₁-C₄)-Perfluoroalkyl bedeutet, trägt und der weiterhin einen oder mehrere Substituenten tragen kann, und
- n: 0 oder 1 bedeutet.

Bevorzugt sind Verbindungen der Formel I, in denen
- R⁶: Wasserstoff, (C₁-C₆)-Alkyl oder eine N-Schutzgruppe wie (C₁-C₈)-Alkanoyl, (C₁-C₆)-Alkylcarbamoyl, (C₁-C₆)-Alkoxycarbonyl, Benzyloxycarbonyl, (C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl oder (C₁-C₆)-Alkoxycarbonyloxy-(C₁-C₆)-alkyl, ein 1, 2, 3 oder 4-wertiges physiologisch verwendbares Kation, insbesondere Na⊕, K⊕, Mg²⊕, Ca²⊕, Al³⊕ oder ein Ammoniumion, ggf. 1-3fach substituiert mit (C₁-C₈)-Alkyl, (C₁-C₈)-Hydroxyalkyl,(C₁-C₄)-Alkoxy-(C₁-C₈)-alkyl, Phenyl, Benzyl oder (C₁-C₈)-Alkyl, welches 1 bis 3-fach substituiert sein kann mit Hydroxy oder (C₁-C₄)-Alkoxy, oder ein Kation eines basischen Aminosäurederivates,
- R⁷: einen Rest der Formel II, ausgenommen -SO₂H, bedeutet

-Y-[C-U]ᵣ-D-W (II)

in welchem
Y -SO₂- oder -CO- bedeutet,
C eine Bindung oder einen verzweigten oder unverzweigten aliphatischen (C₁-C₁₆)-Alkandiyl oder cycloaliphatischen (C₃-C₁₀)-Alkandiylrest oder einen verzweigten oder unverzweigten (C₂-C₁₆)-Alkendiyl- oder Cycloalkendiyl-Rest, oder einen (C₂-C₁₆)-Alkindiyl-Rest oder einen (C₂-C₁₆)-Alkenindiyl-Rest bedeutet, der jeweils eine oder mehrere C-C-Mehrfachbindungen enthalten kann,
U eine Bindung oder
Wasserstoff oder
einen Rest aus der Reihe folgender Heteroatomgruppierungen -CO-, -O(CO)-, -(CO)-O-, -(CO)NR-, -NR(CO)-, -O-, -SO-, -SO₂-F -NR, worin R (C₁-C₃)-Alkyl, (C₁-C₈)-Alkanoyl, (C₇-C₁₆)-Aralkanoyl, (C₆-C₁₂)-Aroyl oder Wasserstoff bedeutet,
r 1, 2, 3 oder 4 ist,
D eine Bindung oder Wasserstoff oder
einen verzweigten oder unverzweigten aliphatischen (C₁-C₁₀)-Alkandiyl-Rest, oder
einen verzweigten oder unverzweigten (C₁-C₁₀)-Alkendiyl-Rest, einen (C₂-C₁₀)-Alkindiyl-Rest oder einen (C₂-C₁₀)-Alkenindiyl-Rest, der jeweils eine oder mehrere C-C-Mehrfachbindungen enthalten kann,
W eine Bindung oder
Wasserstoff oder
einen (C₃-C₁₀) cycloaliphatischen Alkyl-, Alkenyl-, Alkinyl- oder Alkeninyl-Rest oder
einen (C₆-C₁₆)-Arylrest oder einen 5- oder 6-gliedrigen Heteroarylrest, wobei mindestens eine der Variablen C oder D oder W nicht eine Bindung bedeuten und U nur dann in der Bedeutung einer Heteratomgruppierung steht, wenn C nicht eine Bindung bedeutet oder wenn D und/oder W nicht eine Bindung bedeuten und
C und/oder W, sofern diese keine Bindung oder Wasserstoff bedeuten, vorzugsweise ihrerseits substituiert sind durch eine Kombination von bis zu 5 gleichen oder verschiedenen Substituenten aus der Reihe Hydroxy, Halogen, Cyano, Trifluormethyl, Nitro, Carboxyl, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)-alkyl,(C₁-C₁₂)-Alkoxy-(C₁-C₁₂)-alkoxy, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂)-Alkenylcarbonyl,(C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl,(C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy,(C₃-C₈)-Cycloalkoxycarbonyloxy, (C₃-C₁₂)-Alkenyloxycarbonyloxy,(C₃-C₁₂)-Alkinyloxycarbonyloxy, Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N((C₁-C₁₀)-Aryloxy-(C₁-C₁₀)-alkyl)carbamoyl, N-(C₁-C₁₀)-Alkyl-N((C₆-C₁₆)-Aralkyloxy-(C₁-C₁₀)-alkyl)carbamoyl,
N-(C₁-C₁₀)-Alkyl-N-((C₇-C₁₆)-Aralkyloxy-(C₁-C₁₀)-alkyl)carbamoyl, Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy N-((C₁-C₁₀)-Alkoxy-(C₁-C₁₀)-alkyl)carbamoyloxy, N-((C₆-C₁₆)-Aryloxy-(C₁-C₁₀)-alkyl)carbamoyloxy, N-((C₇-C₁₆)-Aralkyloxy-(C₁-C₁₀)-alkyl)carbamoyloxy, N-(C₁-C₁₀)-Alkyl-N-((C₁-C₁₀)-Alkoxy-(C₁-C₁₀)-alkyl)carbamoyloxy), N-(C₁-C₁₀)-Alkyl-N-((C₆-C₁₆)-Aryloxy-(C₁-C₁₀)-alkyl)carbamoyloxy, N-(C₁-C₁₀)-Alkyl-N-((C₇-C₁₆)-Aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-(C₁-C₁₀)-Alkyl-(C₇-C₁₀)-Aralkylamino, N-(C₁-C₁₀)-Alkyl-N-(C₆-C₁₂)-Arylamino, (C₁-C₁₂)-Alkoxy-amino, (C₁-C₁₂)-Alkoxy-(C₁-C₁₀)-alkylamino, (C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino,(C₃-C₈)-Cycloalkanoyl-N-(C₁-C₁₀)-alkylamino,(C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di-(C₁-C₁₀)-alkylamino-(C₁-C₁₀)-alkyl, (C₃-C₈)cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl, (C₃-C₈)-cycloalkylsulfamoyl, N-(C₆-C₁₆)-Arylsulfamoyl, N-(C₇-C₁₆)-Aralkylsulfamoyl, N-(C₁-C₁₀)-Alkyl-N-(C₆-C₁₆)-Arylsulfamoyl, N-(C₁-C₁₀)-Alkyl-N-(C₇-C₁₆)-Aralkylsulfamoyl,
(C₁-C₁₀)-Alkyl-sulfonamido, N-((C₁-C₁₀)-alkyl)-(C₁-C₁₀)-alkylsulfonamido, (C₇-C₁₆)-Aralkylsulfonamido, N-((C₁-C₁₀)-alkyl-(C₇-C₁₆)-Aralkylsulfonamido,
wobei die Reste, die einen Arylrest enthalten, ihrerseits am Aryl substituiert sein können mit 1, 2, 3, 4 oder 5 gleichen oder verschiedenen Substituenten aus der Reihe, Hydroxy, Halogen, Cyano, Trifluormethyl, Nitro, Carboxyl, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)-alkyl, (C₁-C₁₂)-Alkoxy-(C₁-C₁₂)-alkoxy, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂)-Alkenylcarbonyl,(C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl,(C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy,(C₃-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₃-C₁₂)-Alkenyloxycarbonyloxy,(C₃-C₁₂)-Alkinyloxycarbonyloxy,
Carbamoyl, N-(C₁-C₁₂)-Alkylcarbamoyl, Hydroxy-(C₁-C₄)-alkylcarbamoyl, Acyloxy-(C₁-C₄)-alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-Aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-Alkoxy-(C₁-C₁₀)-alkyl)carbamoyl, N-(C₁-C₁₀)-Alkyl-N-((C₆-C₁₆)-Aryloxy-(C₁-C₁₀)-alkyl)carbamoyl, N-(C₁-C₁₀)-Alkyl-N-((C₇-C₁₆)-Aralkyloxy-(C₁-C₁₀)-alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy
   N-((C₁-C₁₀)-Alkoxy-(C₁-C₁₀)-alkyl)carbamoyloxy,
   N-((C₆-C₁₆)-Aryloxy-(C₁-C₁₀)-alkyl)carbamoyloxy,
   N-((C₇-C₁₆)-Aralkyloxy-(C₁-C₁₀)-alkyl)carbamoyloxy,
   N-(C₁-C₁₀)-Alkyl-N-((C₁-C₁₀)-Alkoxy-(C₁-C₁₀)-alkyl)carbamoyloxy,
   N-(C₁-C₁₀)-Alkyl-N-((C₆-C₁₆)-Aryloxy-(C₁-C₁₀)-alkyl)carbamoyloxy,
   N-(C₁-C₁₀)-Alkyl-N-((C₇-C₁₆)-Aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-₁₂)-Alkenylamino, (C₃-C₁₂)-Alkinylamino, N-(C₆-C₁₂)-Arylamino, N-(C₇-C₁₁)-Aralkylamino, N-(C₁-C₁₀)-Alkyl-N-(C₇-C₁₆)-Aralkylamino, N-(C₁-C₁₀)-Alkyl-N-(C₆-C₁₂)-Arylamino, (C₁-C₁₂)-Alkoxy-amino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di-(C₁-C₁₀)-alkylamino-(C₁-C₁₀)-alkyl, (C₃-C₈)-Cycloalkylamino-(C₁-C₁₀)-alkyl, (C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl, (C₃-C₈)-cycloalkylsulfamoyl, N-(C₆-C₁₆)-Arylsulfamoyl, N-(C₇-C₁₆)-Aralkylsulfamoyl, N-(C₁-C₁₀)-Alkyl-N-(C₆-C₁₆)-Arylsulfamoyl, N-(C₁-C₁₀)-Alkyl-N-(C₇-C₁₆)-Aralkylsulfamoyl,
(C₁-C₁₀)-Alkyl-sulfonamido, N-((C₁-C₁₀)-alkyl)-(C₁-C₁₀)-alkylsulfonamido, (C₇-C₁₆)-Aralkylsulfonamido, N-((C₁-C₁₀)-alkyl-(C₇-C₁₆)-Aralkylsulfonamido,
- R⁴: Wasserstoff, (C₁-C₆)-Alkyl, Phenyl, Phenyl-(C₁-C₄)-alkyl,
- R⁵: einen unsubstituierten oder substituierten, verzweigten oder unverzweigten, aliphatischen (C₁-C₈)-Alkylrest, der eine Carboxyl-Gruppe trägt, bedeutet,
der einfach oder mehrfach, vorzugsweise ein- oder zweifach substituiert ist mit einem weiteren Rest aus der Reihe Hydroxy, Halogen, Cyano, Trifluormethyl, (C₁-C₁₂)-Alkyl, (C₃-C₈)-Cycloalkyl, (C₆-C₁₂)-Aryl, (C₇-C₁₂)-Aralkyl, (C₁-C₆)-Alkoxy, (C₆-C₁₂)-Aryloxy, (C₇-C₁₂)-Aralkyloxy, -O-[CH₂₋]xC_{f}H_{(2f+1-g)}F_{g},
wobei die Reste, die einen Arylrest enthalten, ihrerseits am Aryl substituiert sein können mit 1, 2, 3, 4 oder 5 gleichen oder verschiedenen Substituenten aus der Reihe Hydroxy, Halogen, Cyano, Trifluormethyl, Nitro, Carboxyl, (C₁-C₆)-Alkyl, (C₃-C₈)-Cycloalkyl, (C₁-C₆)-Alkoxy, und
- n: 0 oder 1,
- f: 1 bis 8, vorzugsweise 1 bis 5,
- g: 0, 1 bis (2f+1) und
- x: 0 bis 8, vorzugsweise 0 bis 1 bedeuten.

Unter Aryl-, Aryloxy-, Heteroaryl- bzw. Heteroaryloxyverbindungen versteht man insbesondere Phenyl-, Biphenyl- oder Naphthyl- bzw. unsubstituierte 5- und 6-gliedrige heteroaromatische Ringe mit 1, 2 oder 3 Stickstoff- und/oder Sauerstoff und/oder Schwefelatomen, wie Pyridyl-, Pyridazyl-, Pyrimidyl-, Pyrazyl-, Imidazolyl-, Triazolyl-, Thienyl-, Oxazolyl-, und Thiazolyl-Derivate, und deren benzoannellierte Derivate.

Von den genannten Aminosäuren werden insbesondere die natürlichen α-Amino- säuren bevorzugt.

Hierunter sind bevorzugt Verbindungen der allgemeinen Formel I,
worin
- R¹: Hydroxy oder (C₁-C₆)-Alkoxy,
- R² und R³: Wasserstoff,
- R⁶: Wasserstoff, (C₁-C₆)-Alkyl oder eine N-Schutzgruppe wie (C₁-C₈)-Alkanoyl, (C₁-C₆)-Alkylcarbamoyl, (C₁-C₆)-Alkoxycarbonyl, Benzyloxycarbonyl, (C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl oder (C₁-C₆)-Alkoxycarbonyloxy-(C₁-C₆)-alkyl, ein 1, 2, 3 oder 4-wertiges physiologisch verwendbares Kation, insbesondere Na⊕, K⊕, Mg²⊕, Ca²⊕, Al³⊕ oder ein Ammoniumion, ggf. 1-3fach substituiert mit (C₁-C₈)-Alkyl, (C₁-C₈)-Hydroxyalkyl, (C₁-C₄)-Alkoxy-(C₁-C₈)-alkyl, Phenyl, Benzyl oder (C₁-C₈)-Alkyl, welches 1 bis 3-fach substituiert sein kann mit Hydroxy oder (C₁-C₄)-Alkoxy, oder ein Kation eines basischen Aminosäurederivates,
- R⁷: einen Rest der Formel II, ausgenommen -SO₂H, bedeutet

-Y-[C-U]ᵣ-D-W (II)

in welchen
Y -SO₂- bedeutet,
C eine Bindung, oder
einen verzweigten oder unverzweigten aliphatischen (C₁-C₆)-Alkandiyl-rest,
U eine Bindung oder
Wasserstoff oder -O- bedeutet,
r 1 ist,
D eine Bindung oder
Wasserstoff oder
einen verzweigten oder unverzweigten aliphatischen (C₁-C₄)-Alkandiyl-Rest,
W eine Bindung oder
Wasserstoff oder
einen (C₃-C₁₀)-cycloaliphatischen Alkyl-, Alkenyl-, Alkinyl- oder
Alkeninyl-Rest oder
einen (C₆-C₁₆)-Arylrest oder einen 5- oder 6-gliedrigen
Heteroarylrest, wobei mindestens eine der Variablen C oder D oder W nicht eine Bindung bedeuten und U nur dann in der Bedeutung einer Heteratomgruppierung steht, wenn C nicht eine Bindung bedeutet oder wenn D und/oder W nicht eine Bindung bedeuten und
C, D und/oder W, sofern diese keine Bindung oder Wasserstoff bedeuten, vorzugsweise ihrerseits substituiert sind durch eine Kombination von bis zu 5 gleichen oder verschiedenen Substituenten aus der Reihe
Hydroxy, Halogen, Cyano, Trifluormethyl, Nitro, Carboxyl, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)-alkyl,(C₁-C₁₂)-Alkoxy-(C₁-C₁₂)-alkoxy, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂) Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl,(C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-Aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoy, N-((C₇-C₁₆)-Aralkyloxy-(C₁-C₁₀)-alkyl)carbamoyl, N-(C₁-C₁₀)-Alkyl-N-((C₁-C₁₀)-Alkoxy-(C₁-C₁₀)-alkyl)carbamoyl, N-(C₁-C₁₀)-Alkyl-N-((C₆-C₁₆)-Aryloxy-(C₁-C₁₀)-alkyl)carbamoyl, N-(C₁-C₁₀)-Alkyl-N-((C₇-C₁₆)-Aralkyloxy-(C₁-C₁₀)-alkyl)carbamoyl,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂) Alkinylamino, N-(C₆-C₁₂)-Arylamino, N-(C₇-C₁₁)-Aralkylamino, N-(C₁-C₅)-Alkyl-N-(C₇-C₁₀)-Aralkylamino, N-(C₁-C₅)-Alkyl-N-(C₆-C₁₂)-Arylamino,(C₁-C₁₂)-Alkoxy-amino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroyl-amino, (C₇-C₁₆)-Aralkanoylamino, (C₁-C₁₂)-Alkanoyl-N-(C₁-C₁₀)-alkylamino,(C₃-C₈)-Cycloalkanoyl-N-(C₁-C₁₀)-alkylamino,(C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di(C₁-C₁₀)-alkylamino-(C₁-C₁₀)-alkyl, (C₃-C₈)cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkyl-sulfonyl,
wobei die Reste, die einen Arylrest enthalten, ihrerseits am Aryl substituiert sein können mit 1, 2, 3, 4 oder 5 gleichen oder verschiedenen Substituenten aus der Reihe Wasserstoff, Hydroxy, Halogen, Cyano, Trifluormethyl, Nitro, Carboxyl, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)-alkyl,(C₁-C₁₂)-Alkoxy-(C₁-C₁₂)-alkoxy, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂)-Alkenylcarbonyl,(C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl,(C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
Carbamoyl, N-(C₁-C₁₂)-Alkylcarbamoyl, Hydroxy-(C₁-C₄)-alkylcarbamoyl, Acyloxy-(C₁-C₄)-alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-Aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy(C₁-C₁₀)-alkyl)carbamoyl, N-(C₁-C₁₀)-Alkyl-N-((C₁-C₁₀)-Alkoxy-(C₁-C₁₀)-alkyl)carbamoyl, N-(C₁-C₁₀)-Alkyl-N-((C₆-C₁₆)-Aryloxy-(C₁-C₁₀)-alkyl)carbamoyl, N-(C₁-C₁₀)-Alkyl-N((C₇-C₁₆)-Aralkyloxy-(C₁-C₁₀)-alkyl)carbamoyl, Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-(C₁-C₅)-Alkyl-(C₇-C₁₀)-Aralkylamino, N-(C₁-C₅)-Alkyl-(C₆-C₁₂)-Arylamino,(C₁-C₁₂)-Alkoxy-amino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroyl-amino, (C₇-C₁₀)-Aralkanoylamino, (C₁-C₁₂)-Alkanoyl-N-(C₁-C₁₀)-alkylamino, (C₃-C₈)-Cycloalkanoyl-N-(C₁-C₁₀)-alkylamino, (C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di(C₁-C₁₀)-alkylamino-(C₁-C₁₀)-alkyl, (C₃-C₈)cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkyl-sulfonyl,
R⁴ Wasserstoff, (C₁-C₄)-Alkyl,
R⁵ einen verzweigten oder unverzweigten (C₁-C₄)-Alkylrest bedeutet, der eine Carboxylgruppe trägt und der ein- oder zweifach substituiert ist, mit Hydroxy, Fluor, Chlor (C₁-C₆)-Alkyl, (C₃-C₈)-Cycloalkyl, Phenyl, Benzyl,
und
n 0 und 1,
f 1 bis 5,
g 0,1 bis (2f+1) und
x 0 oder 1 bedeuten.

Besonders bevorzugt sind Verbindungen der allgemeinen Formel I, in der
- R¹: Hydroxy, (C₁-C₆)-Alkoxy,
- R² und R³: Wasserstoff,
- R⁶: Wasserstoff oder ein 1 oder 2-wertiges physiologisch verwendbares Kation, insbesondere Na⊕, K⊕, Mg⊕ oder Ca⊕ oder ein Ammoniumion, insbesondere N₃N⊕C(CH₂OH)₃,
- R⁷: einen Rest der Formel II, ausgenommen -SO₂H, bedeutet,

-Y-[C-U]ᵣ-D-W (II),

in welchem
Y -SO₂- bedeutet,
C eine Bindung oder
einen (C₁-C₁₆)-Alkandiyl-Rest,
U eine Bindung,
r 1 ist,
D eine Bindung oder Wasserstoff,
W Wasserstoff oder
einen Phenylrest bedeutet, wobei mindestens eine der Variablen C
oder D oder W nicht eine Bindung bedeuten und
C, D und/oder W substitutiert sein kann durch Fluor, Chlor, Trifluormethyl,
(C₁-C₆)-Alkyl, (C₃-C₈)-Cycloalkyl, Benzyl, Phenyl, (C₁-C₆)-Alkoxy, Phenoxy oder -O-[CH₂]ₓC_{f}H_{(2f+1-g)}F_{g},
Carbamoyl, N-(C₁-C₁₀)-Alkylcarbamoyl, N,N-Di-(C₁-C₈)-alkylcarbamoyl, N-(C₃-C₈)-Cycloalkylcarbamoyl, N-Phenylcarbamoyl, N-(C₇-C₁₁)-Phenylalkylcarbamoyl, N-(C₁-C₈)-Alkyl-N-(C₆-C₁₆)-phenylcarbamoyl, N-(C₁-C₈)-Alkyl-N-(C₇-C₁₁)-Phenylalkylcarbamoyl, N-((C₁-C₁₀)-Alkoxy-(C₁-C₈)-alkyl)carbamoyl, N-Phenoxy-(C₁-C₈)-alkyl)carbamoyl, N-((C₇-C₁₆)-Phenylalkyloxy-(C₁-C₈)-alkyl)carbamoyl, N-(C₁-C₈)-Alkyl-N-((C₁-C₆)-Alkoxy-(C₁-C₈)-alkyl)carbamoyl, N-(C₁-C₈)-Alkyl-N-((C₆-C₁₂)-Phenoxy-(C₁-C₈)-alkyl)carbamoyl, N-(C₁-C₈)-Alkyl-N-((C₇-C₁₆)-Aralkyloxy-(C₁-C₁₀)-alkyl)carbamoyl,
(C₁-C₈)-Alkanoylamino, (C₃-C₈)-Cycloalkanoylamino, Phenylamino, (C₇-C₁₁)-Phenylalkanoylamino, (C₁-C₈)-Alkanoyl-N-(C₁-C₆)-alkylamino,(C₃-C₈)-Cycloalkanoyl-N-(C₁-C₆)-alkylamino, Benzoyl-N-(C₁-C₆)-alkylamino,(C₇-C₁₁)-Phenylalkanoyl-N-(C₁-C₆)-alkylamino,
(C₁-C₁₀)-Alkanoylamino-(C₁-C₄)-alkyl, (C₃-C₈)-Cycloalkanoylamino-(C₁-C₄)-alkyl, Phenoylamino-(C₁-C₆)-alkyl, (C₇-C₁₁)-Phenylalkanoylamino-(C₁-C₄)-alkyl,
wobei die Reste, die einen Arylrest enthalten, ihrerseits am Aryl substitutiert sein können mit 1, 2, 3, 4 oder 5 gleichen oder verschiedenen Substitutenten aus der Reihe Hydroxy, Carboxyl, (C₁-C₄)-Alkoxy, Phenoxy, Benzyloxy, Fluor, Chlor, Trifluormethyl,
(C₁-C₉)-Alkoxycarbonyl, Phenoxycarbonyl, (C₇-C₁₁)-Phenylalkoxycarbonyl,(C₃-C₈)-Cycloalkoxycarbonyl,
(C₁-C₁₂)-Alkylcarbonyloxy, (C₃-C₈)-Cycloalkylcarbonyloxy, Benzoyloxy, (C₇-C₁₁)-Phenylalkylcarbonyloxy,
(C₁-C₈)-Alkoxycarbonyloxy, (C₆-C₁₂)-Phenoxycarbonyloxy (C₇-C₁₁)-Phenylalkyloxycarbonyloxy, (C₃-C₈)-Cycloalkoxycarbonyloxy,
Carbamoyl, N-(C₁-C₆)-Alkylcarbamoyl, N,N-Di-(C₁-C₆)-alkylcarbamoyl, N-(C₃-C₈)-Cycloalkylcarbamoyl, N-Phenylcarbamoyl, N-(C₇-C₁₁)-Phenylalkylcarbamoyl, Hydroxy-(C₁-C₄)-alkylcarbamoyl, Acyloxy-(C₁-C₄)-alkylcarbamoyl,
Carbamoyloxy, N-(C₁-C₆)-Alkylcarbamoyloxy, N,N-Di-(C₁-C₆)-alkylcarbamoyloxy, N-(C₃-C₈)-Cycloalkylcarbamoyloxy
R⁴ Wasserstoff
R⁵ eine Methyl-Gruppe bedeutet, die mit Carboxyl substitutiert ist und einen weiteren Substituenten aus der Reihe (C₁-C₅)-Alkyl, Phenyl, Benzyl, Hydroxy-(C₁-C₄)-alkyl, Hydroxy, (C₁-C₄)-Alkoxy, Phenoxy oder Benzyloxy, trägt, und
n 0 und 1,
f 1 bis 5,
g 0,1 bis (2f+1) und
x 0 oder 1
bedeuten.

Ganz besonders bevorzugt sind Verbindungen der allgemeinen Formel I, in der
- R¹: Hydroxy, (C₁-C₆)-Alkoxy,
- R² und R³: Wasserstoff,
- R⁶: Wasserstoff oder ein 1 oder 2-wertiges physiologisch verwendbares Kation, insbesondere Na⊕, K⊕, Mg²⊕ oder Ca²⊕ oder ein Ammoniumion, insbesondere H₃N⊕C(CH₂OH)₃,
- R⁷: einen Rest der Formel II, ausgenommen -SO₂H, bedeutet

-Y-[C-U]ᵣ-D-W (II),

in welchem
Y -SO₂-,
C eine Bindung oder (C₁-C₁₆)-Alkandiyl,
U eine Bindung,
r 1 ist,
D eine Bindung oder Wasserstoff
W einen Phenylrest bedeutet, wobei W einfach oder zweifach substituiert sein kann durch Fluor, Chlor, (C₁-C₆)-Alkyl, Phenyl, (C₁-C₆)-Alkoxy, und W zusätzlich einfach substituiert ist durch Phenyl, Phenoxy, -O-[CH₂]_{X}C_{f}H_{(2f+1-g)}F_{g},
Carbamoyl, N-(C₁-C₁₀)-Alkylcarbamoyl, N,N-Di-(C₁-C₈)-alkylcarbamoyl, N-(C₃-C₈)-Cycloalkylcarbamoyl, N-Phenylcarbamoyl, N-(C₇-C₁₁)-Phenylalkylcarbamoyl, N-((C₁-C₄)-Alkoxy-(C₁-C₄)-alkyl)carbamoyl, N-Phenoxy-(C₁-C₄)-alkyl)carbamoyl,
(C₁-C₁₀)-Alkanoylamino-(C₁-C₂)-alkyl, (C₃-C₈)-Cycloalkanoylamino-(C₁-C₂)-alkyl, Benzoylamino-(C₁-C₂)-alkyl oder (C₇-C₁₁)-Phenylalkanoylamino-(C₁-C₂)-alkyl,
wobei die Reste, die einen Arylrest enthalten, ihrerseits am Aryl substituiert sein können mit 1, 2, 3, 4 oder 5 gleichen oder verschiedenen Substituenten aus der Reihe Hydroxy, (C₁-C₄)-Alkoxy, Phenoxy, Benzyloxy, Fluor, Chlor, Trifluormethyl,
Carbamoyl, N-(C₁-C₆)-Alkylcarbamoyl, N,N-Di-(C₁-C₆)-alkylcarbamoyl, N-(C₃-C₈)-Cycloalkylcarbamoyl, N-Phenylcarbamoyl, N-(C₇-C₁₁)-Phenalkylcarbamoyl,
R⁴ Wasserstoff,
R⁵ eine Methyl-Gruppe bedeutet, die mit Carboxy substituiert ist, und
n 0,
f 1 bis 5,
g 0,1 bis (2f+1) und
x 0 oder 1
bedeuten.

Weiterhin betrifft die Erfindung die Verwendung von Verbindungen der allgemeinen Formel I sowie die physiologisch verträglichen Salze zur Herstellung eines Arzneimittels zur Hemmung der Kollagenbiosynthese.

Schließlich betrifft die Erfindung die Verbindungen der allgemeinen Formel I zur Verwendung als Arzneimittel.

Insbesondere betrifft die Erfindung die Verbindungen der Formel I zur Anwendung als Inhibitoren der Prolyl-4-hydroxylase.

Insbesondere betrifft die Erfindung die Verbindung der Formel I zur Anwendung der Fibrosesuppressiva.

Die Erfindung umfaßt weiterhin Prodrugs zu den Verbindungen der Formel I, die eine Hemmung der Kollagenbiosynthese in vivo durch Freisetzung von Verbindungen der Formel I oder deren Salze bewirken.

Schließlich umfaßt die Erfindung auch Prodrugs, die in vivo durch Freisetzung vn Verbindungen der Formel I oder deren Salzen eine inhibitorische Wirkung auf die Prolyl-4-hydroxylase bewirken.

Prodrug-Gruppierungen sind chemische Gruppen, die in vivo
- zur Carboxylatgruppe der Verbindungen der Formel I umgewandelt werden und/oder
- vom Amid-N-Atom abgespalten werden können und/oder
- zu einem Pyridinring umgewandelt werden könnne.

Die in Betracht kommenden Prodrug-Gruppen sind dem Fachmann bekannt.

Insbesondere sind folgende Prodrug-Gruppierungen genannt:
für die Carboxylatgruppe Ester-, Amid-, Hydroxymethylgruppen und deren Derivate und Aldehydgruppen und deren Abkömmlinge für das Pyridin-N-Atom N-Oxide und N-Alkylderivate und für den Pyridinring 1,4-Dihydropyridin-Derivate.

Weiterhin betrifft die Erfindung ein Verfahren zur Herstellung von Verbindungen der allgemeinen Formel I.

Verbindungen der Formel I, in denen
- Y =: SO₂ bedeutet,
wurden hergestellt, indem
i) die Pyridin-2-carbonsäure-Derivate bzw. die entsprechenden Ester der Formel 11 mit den Aminen der Formel 5 umgesetzt wurden, oder
ii) die Pyridin-5-carbonsäure-Derivate der Formel 12 mit den Sulfonamid-Derivaten der Formel 9 umgesetzt wurden, oder
iii) die Pyridin-5-carbonsäureamid-Derivate der Formel 13 mit den Sulfonsäure-Derivaten der Formel 2 umgesetzt wurden, vgl. Schema 2,
wobei die Verbindungen der Formeln 12 bzw. 13 ihrerseits aus den Verbindungen der Formel 7 nach den bekannten Methoden hergestellt wurden.

Schema 1 verdeutlicht die Herstellung von Verbindungen der Formel Ia oder Ib, in denen Y = SO₂ bedeutet:

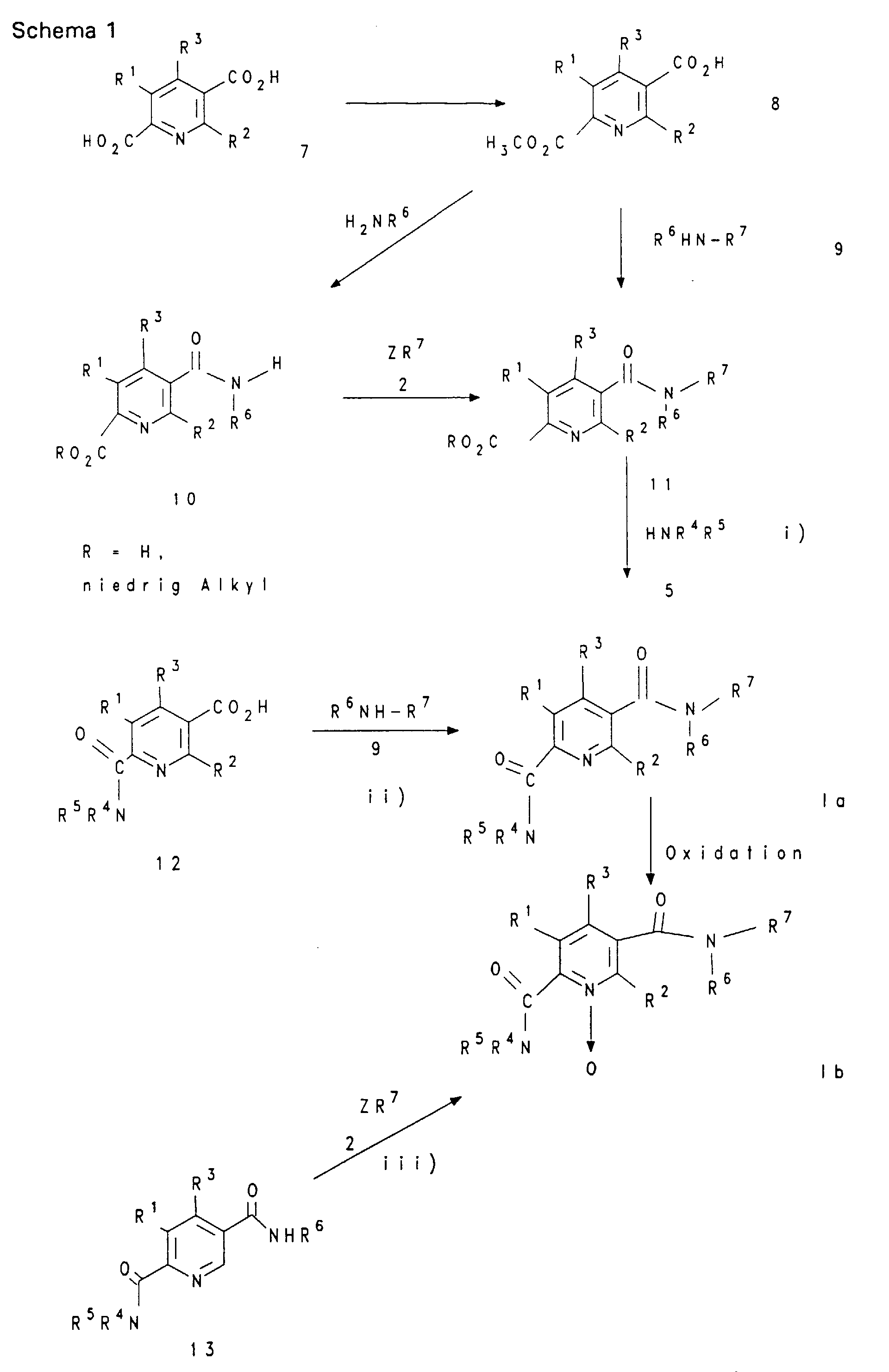

Entsprechend CA: Vol 68, 1968, 68840 h können aus den substituierten Pyridin-2,5-dicarbonsäuren der Formel 7 unter Veresterungsbedingungen die Pyridin-2-carbonsäureester-5-carboxylate der Formel 8 hergestellt werden. Geeignete Bedingungen sind z.B. die Veresterung mit Methanol in Gegenwart von Schwefelsäure, wobei die Reaktionszeit so zu wählen ist, daß die vollständige Veresterung zum Diesterprodukt nur untergeordnet stattfindet, bzw. die Diesterprodukte als Nebenprodukte abgetrennt werden können.

Die Herstellung der Verbindungen der Formel 11 erfolgt aus den Verbindungen der Formel 8 und den Sulfonamidderivaten der Formel 9 (Y = SO₂), wobei es zweckmäßig sein kann, beide Reaktanden mit Hilfsreagenzien zu aktivieren (Houben-Weyl: Methoden der Organischen Chemie, Band IX, Kapitel 19, Seiten 636-637).

An Reagenzien zur Carbonsäurereaktivierung können die dem Fachmann bekannten Substanzen, wie Thionylchlorid, Oxalylchlorid, Pivaloylchlorid oder Chlorameisensäureester-Derivate Verwendung finden. Es ist nicht immer notwendig, diese aktivierten Derivate dem Verbindungen der Formel 8 zu isolieren. Meist ist es zweckmäßig, sie nach Herstellung in situ oder als Rohprodukt mit den Sulfonamidderivaten der Formel 9 umzusetzen.

Zweckmäßigerweise werden die Verbindungen der Formel 9 zunächst mit einer anorganischen oder organischen Base, wie z.B. Natrium- oder Kaliumhydroxid, -carbonat, -alkoxid, -hydrid, -amid, Ammoniak, Triethylamin, Tributylamin, Pyridin bei -20° bis + 150° C, vorzugsweise bei 0° - 80° C zur Reaktion gebracht und dieses Reaktionsgemisch mit einer Verbindung der Formel 8 oder dessen aktivierter Form umgesetzt. Die Umsetzung erfolgt in einem inerten Lösungsmittel, wie z.B. Methylenchlorid, Methanol, Ethanol, Aceton, Essigsäureethylester, Toluol, Tetrahydrofuran, Acetonitril, N,N-Dimethylformamid, N,N-Dimethylacetatamid, Nitromethan, Dimethylsulfoxid oder Gemischen dieser Lösungsmittel. Alternativ können die Ester der Formel 11 mit Hilfe der üblichen Kondensationsreagenzien (wie z.B. N,N'-Dicyclohexylcarbodiimid/4-N,N-Dimethylaminopyridin) hergestellt werden.

Die Reaktion der Pyridin-2-carbonsäureester 11 mit Aminen HNR⁴R⁵ führt zu den erfindungsgemäßen Verbindungen der Formel Ia.

Alternativ können zur Herstellung der Verbindungen der Formel Ia die Verbindungen 11 (R = niederes Alkyl) zu den Pyridin-2-carbonsäure-Derivaten 11 (R = H) verseift werden und diese anschließend mit den Aminen HNR⁴R⁵ nach den üblichen Methoden der Peptidchemie zu den erfindungsgemäßen Verbindungen der Formel Ia gekuppelt werden.

Die weitere Umsetzung zu den Pyridin-N-oxiden der Formel Ib erfolgt durch Oxidation von Verbindungen der Formel Ia.

Eine allgemeine Vorschrift dieser Oxidationsmethode ist auch beispielsweise in "E. Klinsberg, Pyridine and its Derivatives, Interscience Publishers, New York, 1961, Part 2, 93" beschrieben.

Die Oxidation mit Wasserstoffperoxid ist beispielsweise in "E. Ochiai, J. Org. Chem. 18, 534 (1953)" beschrieben.

Die Verfahrensbedingungen können im Detail der deutschen Patentanmeldung P 38 26 471.4, 38 28 140.6, 39 24 093.2, 40 01 002.3 sowie den DE-A-37 03 959, 37 03 962 und 37 03 963 entnommen werden.

Zur Herstellung der Verbindungen der Formel I, in denen NR⁴R⁵ einen Carboxymethyl-Rest (NR⁴R⁵=NH-CH₂-CO₂H) bedeutet, wurden die Verbindungen der Formel 11 (R = niederes Alkyl) zu den Pyridin-2-carbonsäure-Derivaten der Formeln 11 (R = H) verseift und diese mit den entsprechenden Glycinesterderivaten kondensiert. Die Carboxymethyl-Verbindungen wurden durch Verseifung der vorstehenden Esteramide oder durch katalytische Hydrierung der Benzylesteramide erhalten.

Zur Herstellung von Verbindungen gemäß der allgemeinen Formel I (Ia, Ib) nach dem Schema 1 werden Verbindungen eingesetzt, in denen R⁶ Wasserstoff bedeutet. Die Salzbildung, nach der R⁶ ein physiologisch verwendbares Kation bedeutet, erfolgt bevorzugt anschließend. Als Salzbildner kommen bevorzugt N-Alkylamine, (Hydroxyalkyl)-amine und (Alkoxyalkyl)-amine, wie z.B. 2-Ethanolamin, 3-Propanolamin, 2-Methoxyethylamin, 2-Ethoxyethylamin und α,α,α-Tris-(hydroxymethyl)methylamin (= Trispuffer, Tromethan) oder auch basische Aminosäuren, wie z.B. Histidin, Arginin und Lysin in Frage.

Die Einführung des Substituenten R¹ wird in Schema 2 verdeutlicht.

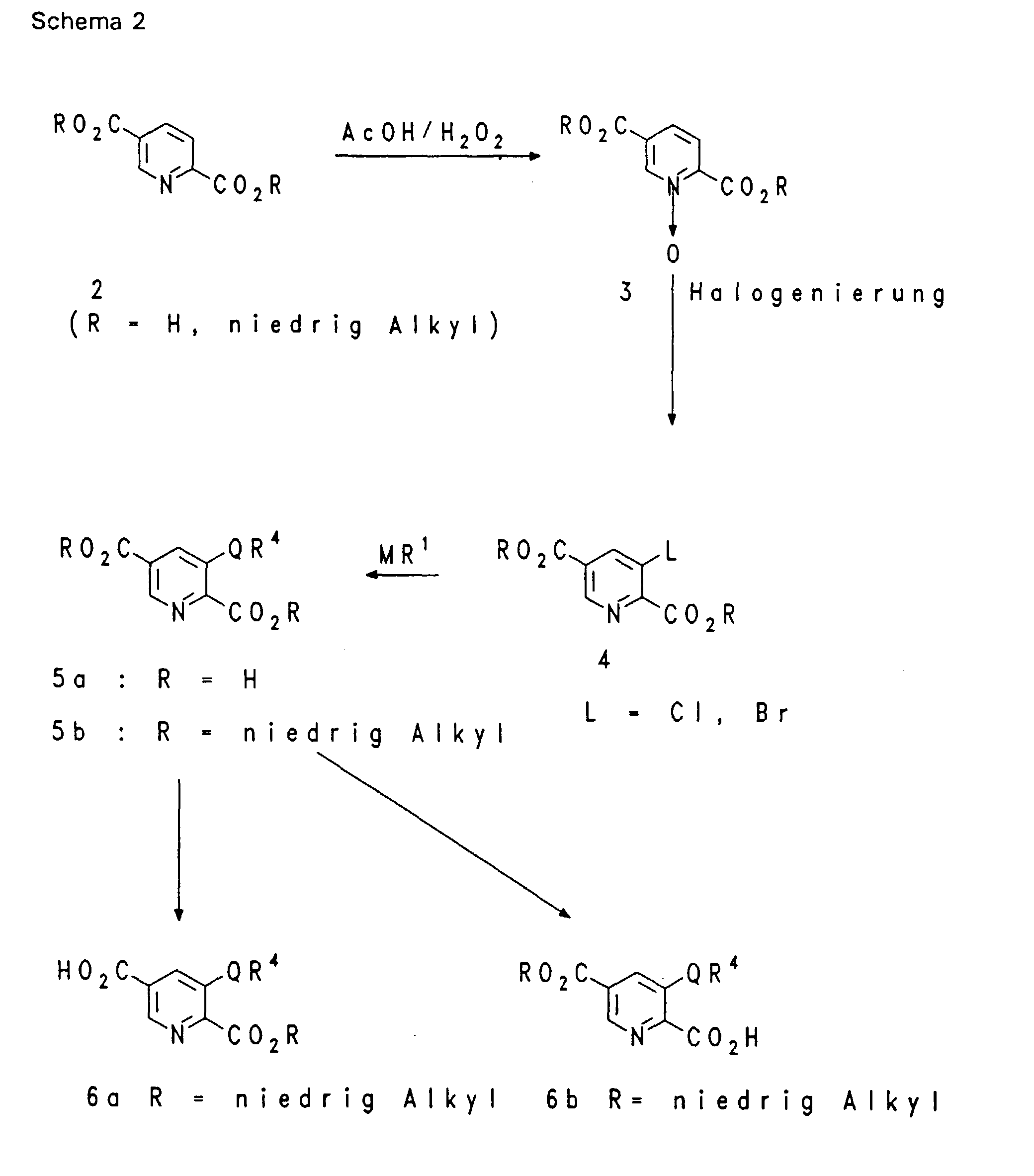

Die 3-substituierten 5-Carboxypyridin-2-carbonsäureester der Formel 6a und 6b werden aus den Pyridin-2,5-dicarbonsäurediestern der Formel 2 hergestellt.

Die Oxidation der Pyridin-2,5-dicarboxylate der Formel 2 ist in J. Chem. Soc. Perkin Trans. 2, 1978, 34-38 und in J. Org. Chem. 25 (1960) 565-568 (M.L. Peterson) beschrieben.

Die Halogenierung (Chlorierung) der Pyridin-N-oxide der Formel 3 und die Reaktion des 3-Chlorpyridin-2,5-dicarbonsäurediesters (Formel 4) mit Alkoholaten MR¹
(M = Metall; z.B. Alkali, Erdalkali) kann in Analogie zu dem in der Patentschrift CH 658 651 (LONZA) beschriebenen Verfahren durchgeführt werden.

Aus substituierten Pyridin-2,5-dicarbonsäuren (siehe CA: Vol. 68, 1968, 68840 h) können unter Veresterungsbedingungen die Pyridin-2-carbonsäureester-5-carboxylate der Formel 6a und 6b hergestellt werden. Geeignete Bedingungen sind z.B. die Veresterung mit Methanol in Gegenwart von Schwefelsäure, wobei die Reaktionszeit so zu wählen ist, daß die vollständige Veresterung zum Diesterprodukt nur untergeordnet stattfindet, bzw. die Diesterprodukte als Nebenprodukte abgetrennt werden können.

Die Verbindungen der Formel I sind Inhibitoren der Prolyl-4-hydroxylase. Die Hemmungen dieses Enzyms würde, wie von Kaule und Günzler in Anal. BioChem. 184, 291-297 (1990) beschrieben, bestimmt.

Die erfindungsgemäßen Verbindungen der Formel I besitzen wertvolle pharmakologische Eigenschaften und zeigen insbesondere antifibrotische Wirksamkeit, insbesondere in der Leber, in der Lunge und auf der Haut (Skleroderma).

Die antifibrotische Wirkung kann im Modell der Tetrachlorkohlenstoff-induzierten Leberfibrose bestimmt werden. Dazu werden Ratten mit CCl₄ (1 ml/kg) - gelöst in Olivenöl - zweimal wöchentlich behandelt. Die Prüfsubstanz wird täglich, gegebenenfalls sogar zweimal täglich per os oder intraperitoneal - gelöst in einem geeigneten verträglichen Lösungsmittel - verabreicht. Das Ausmaß der Leberfibrose wird histologisch bestimmt und der Anteil Kollagen in der Leber per Hydroxyprolinbestimmung - wie bei Kivirikko et al. (Anal. Biochem. 19, 249 f. (1967)) beschrieben- analysiert. Die Aktivität der Fibrogenese kann durch radioimmunologische Bestimmung von Kollagenfragmenten und Prokollagenpeptiden im Serum bestimmt werden. Die erfindungsgemäßen Verbindungen sind in diesem Modell in Konzentrationen 1 - 100 mg/kg wirksam.

Die Aktivität der Fibrogenese kann durch radioimmunologische Bestimmung des N-terminalen Propeptids des Kollagens Typ-III oder der N- bzw. C-terminalen Quervernetzungsdomäne des Kollagens-Typ-IV (7s-Kollagen bzw. Typ-IV-Kollagen NC₁) im Serum bestimmt werden.

Zu diesem Zweck wurden die Hydroxyprolin-, Prokollagen-III-Peptid-, 7s-Kollagen- und Typ-IV-Kollagen-NC-Konzentrationen in der Leber von
a) unbehandelten Ratten (Kontrolle)
b) Ratten, denen Tetrachlorkohlenstoff verabreicht wurde (CCl₄-Kontrolle)
c) Ratten, denen zunächst CCl₄ und anschließend eine erfindungsgemäße Verbindung verabreicht wurde
gemessen (diese Testmethode wird beschrieben von Rouiller, C., experimental toxic injury of the liver; in The Liver, C. Rouiller, Vol. 2, 5. 335-476, New York, Academic Press, 1964).

Die Verbindungen der Formel I können als Medikamente in Form pharmazeutischer Präparate Verwendung finden, welche sie gegebenenfalls zusammen mit verträglichen pharmazeutischen Trägern enthalten. Die Verbindungen können als Heilmittel, z.B. in Form pharmazeutischer Präparate Verwendung finden, welche diese Verbindungen in Mischung mit einem für die enterale, perkutane oder parenterale Applikation geeigneten pharmazeutischen, organischen oder anorganischen Träger, wie z.B. Wasser, Gummi arabicum, Gelatine, Milchzucker, Stärke, Magnesiumstearat, Talk, pflanzliche Öle, Polyalkylenglykole, Vaseline usw. enthalten.

Sie können zu diesem Zweck oral in Dosen von 0,1 - 25 mg/kg/Tag, vorzugsweise 1 - 5 mg/kg/Tag oder parenteral in Dosen von 0,01 - 5 mg/kg/Tag, vorzugsweise 0,01 - 2,5 mg/kg/Tag, insbesondere 0,5 - 1,0 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Die Erfindung umfaßt weiterhin die Verwendung der erfindungsgemäßen Verbindungen bei der Herstellung von Arzneimitteln, die zur Behandlung und Prophylaxe der vorstehend genannten Stoffwechselstörungen eingesetzt werden.

Ein weiterer Gegenstand der Erfindung sind Arzneimitel, die ein oder mehrere erfindungsgemäße Verbindungen der Formel I und/oder deren physiologisch verträgliche Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 % beträgt.

Geeignete Hilfs- bzw. Trägestoffe für die gewünschte Arzneimittelformulierung sind beispielsweise neben Lösemitteln, Gelbildner, Suppositoriengrundlagen, Tabletten-Hilfsstoffen und anderen Wirkstoffträgern auch Antioxidantien, Dispergiermittel, Emulgatoren, Entschäumer, Geschmachskorrigentien, Konservierungsmittel, Lösungsvermittler oder Farbstoffe.

Die folgenden Beispiele sollen die Erfindung erläutern.

Die Verbindungen werden im folgenden als substituierte Pyridin-2-carbonsäureamide bezeichnet.

Sie können ebenso als N-substituierte Glycinderivate klassifiziert werden. Es handelt sich folglich um substituierte N-(Pyridyl-2-carbonyl)glycine.

In diesem Fall lautet z.B. der Name der Titelverbindung des Beispiels 1

N-(3-Methoxy-5-[((phenylsulfonyl)-amino)carbonyl]pyridyl-2-carbonyl)glycin.

### Beispiel 1

### N-Carboxymethyl-3-methoxy-5-[((phenylsulfonyl)-amino)carbonyl]pyridin-2-carbonsäureamid

a) 5-Methoxycarbonylpyridin-2-carbonsäure-1-oxid (vgl. J. Org. Chem. 25 (1960) 565)
   12 g (60 mMol) Pyridin-2,5-dicarbonsäure-dimethylester wurden in 30 ml Eisessig suspendiert und bei 20°C unter Rühren mit 13 ml Wasserstoffperoxid (35 %) versetzt. Unter Rühren wurde sodann auf 100°C (Innentemperatur) erwärmt, wobei sich bei 50°C eine klare Lösung bildete. Nachdem 90 min bei 100°C gerührt worden war, ließ man auf 20°C abkühlen, saugte die kristalline Fällung ab, wusch mit Wasser und nach dem Trocknen erhielt man 7,5 g Produkt, Fp. 160°C (Zers.)
b) 3-Chlorpyridin-2,5-dicarbonsäure-dimethylester
   Es wurden 17 ml Thionylchlorid, 35 ml wasserfreies Chloroform und 1,5 ml N,N-Dimethylformamid unter Rühren auf 60°C erwärmt und bei dieser Temperatur portionsweise mit 7,5 g des vorstehenden Produkts versetzt. Dann wurde weitere 60 min bei 60°C gerührt, das Lösungsmittel und überschüssiges Reagenz nach dem Abkühlen im Vakuum abdestilliert, der Rückstand mit Dichlormethan versetzt, der N,N-Dimethylformamid x HCI-Komplex abgesaugt und mit Dichlormethan gewaschen. Zur Mutterlauge gab man unter Kühlung ca. 15 ml Triethylamin und 10 ml Methanol und rührte 30 min. Nach dem Eindampfen im Vakuum wurde der Rückstand in 50 ml Wasser gelöst, 3 x mit Dichlormethan extrahiet, die organische Phase getrocknet, eingeengt und der Rückstand mit n-Heptan und n-Heptan:Ethylacetat (3:1) an Kieselgel chromatographiert. Aus entsprechenden Fraktionen wurden 5,3 g Produkt mit Petrolether zur Kristallisation gebracht, Fp. 36-38°C.
c) 3-Methoxypyridin-2,5-dicarbonsäure
   53 g (0,231 mol) des vorstehenden Diesters wurden in 500 ml Methanol gelöst und unter Rühren bei 20°C mit 150 ml (0,81 mol) Natriummethylat-Lösung (30 % in Methanol) versetzt, wobei die Temperatur auf 30°C anstieg. Man erhitzte 4,5 h unter Rückfluß, versetzte bei 20°C mit 300 ml Wasser und rührte 30 min bei 35°C. Das überschüssige Methanol wurde im Vakuum abdestilliert, die wäßrige Phase unter Kühlung mit halbkonzentrierter wäßriger Salzsäure auf pH 2 gebracht, das farblose kristalline Produkt abgesaugt und getrocknet. Man erhielt 49 g, Fp. 185 ° (Gasentwicklung); 255°C (Zers.)
d) 5-Carboxy-3-methoxypyridin-2-carbonsäuremethylester
   10 g (50,7 mmol) der vorstehenden Pyridindicarbonsäure wurden in 150 ml wasserfreiem Methanol suspendiert, mit 2 ml konzentrierter Schwefelsäure versetzt und 3 h rückfließend erhitzt. Dann wurde die Hälfte des Methanols im Vakuum abdestilliert, der Rückstand in 400 ml Eiswasser eingetragen, der kristalline Rückstand abgesaugt, mit Wasser gewaschen, der Rückstand in 150 ml gesättigter wäßriger Na-bicarbonat-Lösung gelöst, zweimal mit je 80 ml Dichlormethan extrahiert, die Bicarbonat-Phase unter Kühlung mit halbkonzentrierter wäßriger Salzsäure auf pH 1 gebracht, das ausgefallene Produkt abgesaugt und getrocknet. Man erhielt 5 g farblose, kristalline Substanz, Fp. 196-197°C. Aus der Dichlormethan-Phase wurden 1,7 g Dimethylester erhalten, Fp. 53-55°C (aus Petrolether).
e) 3-Methoxy-5-[((phenylsulfonyl)-amino)carbonyl]pyridin-2-carbonsäuremethylester
   2,4 g (15 mmol) Benzolsulfonsäureamid wurden in 80 ml wasserfreiem Tetrahydrofuran suspendiert, bei 20°C unter Rühren mit 1,85 g (16,5 mmol) Kalium-tert.Butylat versetzt und 30 min bei 50-60°C gerührt (Lösung A). In einem zweiten Kolben wurden 3,2 g (15 mmol) der vorstehenden Pyridincarbonsäure in 80 ml wasserfreiem Tetrahydrofuran gelöst, bei 20°C unter Rühren 2,7 g (16,5 mmol) N,N'-Carbonyldiimidazol zugegeben, 30 min bei 60°C gerührt und die abgekühlte Lösung sodann zur Lösung A getropft. Man rührte 1 h bei 60°C, engte nach dem Abkühlen im Vakumm ein, versetzte den Rückstand mit 200 ml gesättigter, wäßriger Na-bicarbonat-Lösung und extrahierte mit Dichlormethan. Die Bicarbonat-Phase wurde mit halbkonzentrierter wäßriger Salzsäure auf pH 3 gebracht und viermal mit Dichlormethan extrahiert. Nach dem Trocknen wurde Dichlormethan abdestilliert und der Rückstand mit Diethylether/Ethylacetat zur Kristallisation gebracht. Man erhielt 4,0 g Produkt, Fp. 160-163°C (ab 150°C Sintern).
f) 3-Methoxy-5-[((phenylsulfonyl)-amino)carbonyl]pyridin-2-carbonsäure.
   4,0 g (11,4 mmol) des vorstehenden Esters wurden bei 20°C unter Rühren in 150 ml 1,5 N methanolische Natronlauge eingetragen. Nach dem kurzzeitig eine klare Lösung entstand, fiel das Na-Salz aus. Man gab 20 ml Wasser hinzu, rührte 30 min nach, engte im Vakuum ein, löste den Rückstand in 100 ml Wasser, extrahierte mit Dichlormethan, brachte die wäßrige Phase mit konzentrierter wäßriger Salzsäure auf pH 1 und saugte 3,7 g Produkt als kristalline Fällung ab, Fp. 176-178°C.
g) N-Ethoxycarbonylmethyl-3-methoxy-5-[((phenylsulfonyl)amino)carbonyl]pyridin-2-carbonsäureamid
   3,7 g (10 mmol) der vorstehenden Carbonsäure wurden in 300 ml Dichlormethan suspendiert, unter Rühren nacheinander mit 1,4 g (10 mmol) Glycinethylester-Hydrochlorid, 3,9 ml (30 mmol) N-Ethylmorpholin, 1,5 g (11 mmol) 1-Hydroxy-1H-benztriazol und 4,2 g (10 mmol) N-Cyclohexyl-N'-(2-morpholinoethyl)-carbodiimid-methyl-p-toluolsulfonat versetzt und 2 Tage bei 20°C gerührt. Dann wurde vom Ungelöstem abgesaugt, mit Wasser, sodann mit 1 N wäßriger Salzsäure extrahiert, die organische Phase getrocknet, eingeengt und der Rückstand mit Diethylether zur Kristallisation gebracht. Man erhielt 1,5 g Produkt, Fp. ab 100°C unter Schäumen. Aus der wäßrigen Phase wurde nach Ansäuern mit Salzsäure auf pH 3 und Extraktion mit Dichlormethan, Trocknen und Einengen weitere 1,6 g Produkt isoliert.
   h) Die Titelverbindung wurde erhalten, indem 700 mg (1,66 mmol) des vorstehenden Glycinethylesters bei 20°C unter Rühren in 70 ml 1,5 N methanolische Natronlauge eingetragen und 1 Stunde gerührt wurden. Dann wurde im Vakuum eingeengt, der Rückstand in 50 ml Wasser aufgenommen, mit konzentrierter wäßriger Salzsäure auf pH 1 gebracht, das auskristallisierte Produkt abgesaugt und getrocknet. Man erhielt 550 mg der farblos kristallinen Titelverbindung, Fp. 186-188°C.

### Beispiel 2

### N-Carboxymethyl-5-[((4-(((2-(4-fluorphenyl)ethyl)-amino)carbonyl)phenylsulfonyl)-amino)carbonyl]-3-methoxypyridin-2-carbonsäureamid

### Beispiel 3

### N-Carboxymethyl-5-[((4-(((2-(4-fluorphenyl)ethyl)-amino)carbonyl)phenylsulfonyl)-amino)carbonyl]-3-(2-methyl-1-propyloxy)pyridin-2-carbonsäureamid

### Beispiel 4

### N-Carboxymethyl-5-[((4-(((2-(4-fluorphenyl)ethyl)-aminolcarbonyl)phenylsulfonyl)-amino)carbonyl]-3-hydroxypyridin-2-carbonsäureamid

### Beispiel 5

### 5-[((4-((Cyclohexylamino)carbonyl)phenylsulfonyl)amino)carbonyl]-3-methoxypyridin-2-carbonsäure-N-(carboxymethyl)amid

a) 5-[((4-((Cyclohexylamino)carbonyl)phenylsulfonyl)amino)carbonyl]-3-methoxypyridin-2-carbonsäure
   4,2 g (20 mmol) 5-Carboxy-3-methoxypyridin-2-carbonsäuremethylester (vgl. Beispiel 1d)) wurden analog Beipsiel le) mit 5,6 g (20 mmol) 4-((Cyclohexylamino)carbonyl)benzolsulfonamid umgesetzt und das Produkt anschließend verseift. Man erhielt 7 g Produkt, Fp. ab 235°C (Sintern bei 205°C, aus wäßriger Salzsäure).
b) 5-[((4-((Cyclohexylamino)carbonyl)phenysulfonyl)amino)carbonyl]-3-methoxypyridin-2-carbonsäure-N-((ethoxycarbonyl)methyl)amid wurde analog Beispiel 1g) aus 1,85 g (4 mmol) der vorstehenden Verbindung erhalten; 1,3 g Produkt, Fp. 225°C (unter Schäumen, Sintern bei 185°C, aus Diisopropylether).
c) Die Titelverbindung wurde erhalten, indem 0,55 g des vorstehenden Esters in 50 ml 1,5 n methanolischer NaOH verseift wurden. Man erhielt 0,43 g Produkt, Fp. 240°C (unter Schäumen, Sintern ab 150°C, aus wäßriger Salzsäure)

### Beispiel 6

### 5-[((4-((Cyclohexylamino)carbonyl)phenylsulfonyl)amino)carbonyl]-3-(2-methyl-1-propyloxy)pyridin-2-carbonsäure-N-(carboxymethyl)amid

a) 3-(2-Methyl-1-propyloxy)-pyridin-2,5-dicarbonsäure
   Analog Beispiel 1c) wurden 3,5 g (146 mmol) Natrium in 350 ml 2-Methyl-1-propanol (iso-Butylalkohol) gelöst und unter Rühren bei 20°C mit 13,7 g (55 (mmol) 3-Chlorpyridin-2,5-dicarbonsäure-dimethylester (wie in Beispiel 1b) hergestellt) versetzt. Dann wurde 90 Minuten bei 80°C gerührt, nach dem Abkühlen im Vakuum eingeengt, der Rückstand in 200 ml 1 N methanolischer NaOH aufgenommen und bei 20°C gerührt. Nach 15 Minuten trübte sich die Lösung. Man setzte Wasser hinzu bis eine klare Lösung entstand, rührte 1 Stunde, engte im Vakuum ein, säuerte die wäßrige Lösung mit wäßriger Salzsäure an, saugte das kristalline Produkt ab, wusch, trocknete und erhielt 10,6 g Dicarbonsäure, Fp. 192°C (Zers.).
b) 5-Carboxy-3-(2-methyl-1-propyloxy)pyridin-2-carbonsäuremethylester wurden analog Beispiel 1d) aus der obigen Dicarbonsäure durch Veresterung in Methanol/Schwefelsäure erhalten, Fp. 158 bis 160°C (aus wäßriger Salzsäure).
c) 5-[((4-((Cyclohexylamino)carbonyl)phenylsulfonyl)amino)carbonyl]-3-(2-methyl-1-propyloxy)pyridin-2-carbonsäuremethylester wurde aus 1,5 g (6 mmol) des vorstehenden Produkts mit N,N'-Carbonyldiimidazol und 4-((Cyclohexylamino)carbonyl)benzolsulfonamid (Fp. 268 bis 269°C, aus Ethanol/Wasser (1:1))/Kalium-tert.-butylat erhalten, Fp. 270°C (aus Diisopropylether).
d) 5-[((4-((Cyclohexylamino)carbonyl)phenylsulfonyl)amino)carbonyl]-3-(2-methyl-1-propyloxy)pyridin-2-carbonsäure wurde durch Verseifung des obigen Esters erhalten, Fp. > 150°C (unter Schäumen, aus wäßriger Salzsäure).
e) 5-[((4-((Cyclohexylamino)carbonyl)phenylsulfonyl)amino)carbonyl]-3-(2-methyl-1-propyloxy)pyridin-2-carbonsäure-N-((ethoxycarbonyl)methyl)amid
   wurde analog Beispiel 1g) erhalten.
   Aus 1,55 g (3,1 mmol) der vorstehenden Carbonsäure und 0,43 g (3,1 mmol) Glycinethylester-Hydrochlorid wurden 1,46 g Produkt gewonnen, Fp. > 280°C (unter Gasentwicklung, sintern bei 135°C, aus Diethylether).
f) Die Titelverbindung wurde erhalten, indem der vorstehende Ester in methanolischer Natronlauge verseift wurde. Aus 0,7 g Ester wurden 0,63 g der Titelverbindung erhalten, Fp. 240°C (unter Schäumen, sintern bei 150°C, aus wäßriger Salzsäure).

### Beispiel 7

### N-Carboxymethyl-5-[((4-((cylcohexylamino)carbonyl)phenylsulfonyl)-amino)carbonyl]-3-hydroxypyridin-2-carbonsäureamid

### Beispiel 8

### N-Carboxymethyl-5-[((4-(N,N-diethylaminocarbonyl)phenylsulfonyl)-amino)carbonyl]-3-methoxypyridin-2-carbonsäureamid

### Beispiel 9

### N-Carboxymethyl-5-[((4-(N,N-diethylaminocarbonyl)phenylsulfonyl)-amino)-carbonyl]-3-(2-methyl-1-propyloxy)pyridin-2-carbonsäureamid

### Beispiel 10

### N-Carboxymethyl-5-[((4-(N,N-diethylaminocarbonyl)phenylsulfonyll-amino)-carbonyl]-3-hydroxypyridin-2-carbonsäureamid

### Beispiel 11

### N-Carboxymethyl-5-[((4-(N,N-dipropylaminocarbonyl)phenylsulfonyl)-amino)-carbonyl]-3-methoxypyridin-2-carbonsäureamid

### Beispiel 12

### N-Carboxymethyl-5-[((4-(4,4-dibutylaminocarbonyl)phenylsulfonyl)-amino)-carbonyl]-3-methoxypyridin-2-carbonsäureamid

### Beispiel 13

### N-Carboxymethyl-5-[((4-(2-((2-chlor-5-methoxybenzoyl)-amino)ethyl)-phenylsulfonyl)-amino)-carbonyl]-3-methoxypyridin-2-carbonsäureamid

### Beispiel 14

### N-Carboxymethyl-5-[((4-(2-((2-chlor-5-methoxybenzoyl)-amino)ethyl)-phenylsulfonyl)-amino)-carbonyl]-3-(2-methyl-1-propyloxy)pyridin-2-carbonsäureamid

### Beispiel 15

### N-Carboxymethyl-5-[((4-(2-((2-chlor-5-methoxybenzoyl)-amino)ethyl)-phenylsulfonyl)-amino)-carbonyl]-3-hydroxypyridin-2-carbonsäureamid

### Beispiel 16

### N-Carboxymethyl-5-[((4-n-butyloxyphenyl)sulfonyl)-amino)carbonyl]-3-methoxypyridin-2-carbonsäureamid

### Beispiel 17

### N-Carboxymethyl-5-[((n-butylsulfonyl)-amino)carbonyl]-3-methoxypyridin-2-carbonsäureamid

### Beispiel 18

### N-Carboxymethyl-5-[((4-fluorphenyl)sulfonyl)-amino)carbonyl]-3-methoxypyridin-2-carbonsäureamid

### Beispiel 19

### N-Carboxymethyl-3-methoxy-5-[((4-propylphenyl)sulfonyl)-amino)carbonyl]-pyridin-2-carbonsäureamid

### Beispiel 20

### N-Carboxymethyl-3-hydroxy-5-[((4-propylphenyl)sulfonyl)-amino)carbonyl]pyridin-2-carbonsäureamid

### Beispiel 21

### N-Carboxymethyl-5-[((n-butylsulfonyl)-amino)carbonyl]-3-(3-methyl-1-butyloxy)-pyridin-2-carbonsäureamid pyridin-2-carbonsäureamid

### Beispiel 22

### N-Carboxymethyl-5-[((benzylsulfonyl)-amino)carbonyl]-3-methoxypyridin-2-carbonsäureamid

### Beispiel 23

### 5-[((1-Decylsulfonyl)amino)carbonyl]-3-methoxypyridin-2-carbonsäure-N-(carboxymethyl)amid

a) 5-[((1-Decylsulfonyl)amino)carbonyl]-3-methoxypyridin-2-carbonsäure
   2,1 g (10 mmol) 5-Carboxy-3-methoxypyridin-2-carbonsäuremethylester (vgl. Beispiel 1d)) wurden analog wie in Beispiel le) beschrieben mit N,N'-Carbonyldiimidazol und 1-Decylsulfonsäureamid/Kalium-tert.-butylat umgesetzt und der so erhaltene Pyridin-2-carbonsäuremethylester mit 1 n methanolischer Natronlauge verseift. Nachdem die wäßrige Lösung unter Kühlung angesäuert wurde, erhielt man 1,4 g Produkt, Fp. 145°C (unter Zers.).
b) 5-[((1-Decylsulfonyl)amino)carbonyl]-3-methoxypyridin-2-carbonsäure-N-((ethoxycarbonyl)methyl)amid
   1 g (2,5 mmol) der vorstehenden Pyridin-2-carbonsäure wurden analog wie in Beispiel 1g) beschrieben mit Glycinethylester-Hydrochlorid kondensiert. Nach Aufarbeitung wurde der Rückstand mit Diisopropylether zur Kristallisation gebracht. Man erhielt 1,1 g des Produkts, Fp. 70°C.
c) Die Titelverbindung wurde erhalten, indem 0,8 g des vorstehenden Esters in 100 ml 1,5 n methanolischer Natronlauge 1 Stunde bei Raumtemperatur verseift wurden. Dann wurde im Vakuum eingeengt, der Rückstand in Wasser aufgenommen und unter Eiskühlung mit Salzsäure angesäuert, die Fällung abgesaugt, mit Wasser gewaschen und getrocknet. Man erhielt 0,52 g der Titelverbindung, Fp. 90°C.

### Beispiel 24

### 5-[((1-Hexadecylsulfonyl)amino)carbonyl]-3-methoxypyridin-2-carbonsäure-N-(carboxymethyl)amid

a) 5-[((1-Hexadecylsulfonyl)amino)carbonyl]-3-methoxypyridin-2-carbonsäuremethylester wurde analog Beispiel 1e) aus 3,2 g (15 mmol) 5-Carboxy-3-methoxypyridin-2-carbonsäuremethylester (vgl. 1d) mit N,N'-Carbonyldiimidazol, 4,8 g (15 mmol) 1-Hexadecylsulfonsäure-amid (Fp. 98 bis 100°C, aus wäßriger Salzsäure) und Kalium-tert.-butylat erhalten. 6,1 g Produkt, Fp. 75 bis 78°C (aus wäßriger Salzsäure).
b) 5-[((1-Hexadecylsulfonyl)amino)carbonyl]-3-methoxypyridin-2-carbonsäure wurde durch Verseifung des obigen Esters erhalten, Fp. 152°C (unter Zers., aus Ethylacetat).
c) 5-[((1-Hexadecylsulfonyl)amino)carbonyl]-3-methoxypyridin-2-carbonsäure-N-((ethoxycarbonyl)methyl)amid wurde analog Beispiel 1g) erhalten, Fp. 127 bis 130°C (unter Schäumen, aus Diisopropylether).
d) Die Titelverbindung wurde durch Verseifung von 1,5 g des obigen Esters analog Beispiel 23c) erhalten. Der Rückstand wurde in wäßrigem Tetrahydrofuran mit Salzsäure auf pH 1 gebracht, im Vakuum eingeengt, das kristalline, etwas schmierige Produkt abgesaugt und getrocknet, 1,38 g Produkt, Fp. ca. 180°C.

## Patentansprüche

1. Sulfonamidocarbonylpyridin-2-carbonsäureamide sowie ihre Pyridin-N-oxide der allgemeinen Formel I 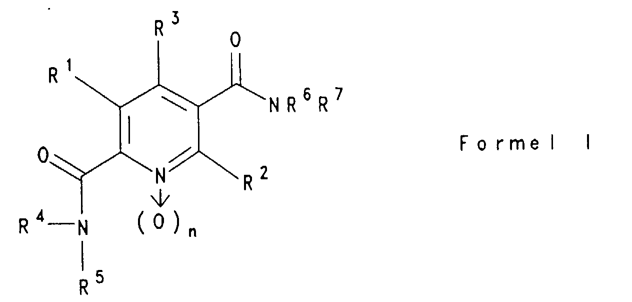 worin
bedeutet und
R¹ Hydroxy oder (C₁-C₆)-Alkoxy,
R² und R³ gleich oder verschieden sind und Wasserstoff, unsubstituiertes oder substituiertes (C₁-C₆)-Alkyl, (C₁-C₆)-Alkoxy, Halogen, insbesondere Fluor, Chlor oder Brom, Nitril, Hydroxy, Amino,
R⁶ Wasserstoff, (C₁-C₆)-Alkyl oder eine N-Schutzgruppe wie (C₁-C₈)-Alkanoyl, (C₁-C₆)-Alkylcarbamoyl, (C₁-C₆)-Alkoxycarbonyl, Benzyloxycarbonyl, (C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl oder (C₁-C₆)-Alkoxycarbonyloxy-(C₁-C₆)-alkyl, ein 1, 2, 3 oder 4-wertiges physiologisch verwendbares Kation, insbesondere Na⊕, K⊕, Mg²⊕, Ca²⊕, Al³⊕ oder ein Ammoniumion, ggf. 1-3fach substituiert mit (C₁-C₈)-Alkyl, (C₁-C₈)-Hydroxyalkyl,(C₁-C₄)-Alkoxy-(C₁-C₈)-alkyl, Phenyl, Benzyl oder (C₁-C₈)-Alkyl, welches 1 bis 3-fach substituiert sein kann mit Hydroxy oder (C₁-C₄)-Alkoxy, oder ein Kation eines basischen Aminosäurederivates,
R⁷ einen Rest der Formel II, ausgenommen -SO₂HF bedeutet
-Y-[C-U]ᵣ-D-W (II)
in welchem
Y -SO₂- oder -CO- bedeutet,
C eine Bindung oder
einen verzweigten oder unverzweigten aliphatischen (C₁-C₁₆)-Alkandiyl oder cycloaliphatischen (C₃-C₁₀)-Alkandiylrest oder einen verzweigten oder unverzweigten (C₂-C₁₆)-Alkendiyl- oder Cycloalkendiyl-Rest, oder einen (C₂-C₁₆)-Alkindiyl-Rest oder einen (C₂-C₁₆)-Alkenindiyl-Rest bedeutet, der jeweils eine oder mehrere C-C-Mehrfachbindungen enthalten kann,
U eine Bindung oder
Wasserstoff oder
einen Rest aus der Reihe folgender Heteroatomgruppierungen -CO-, -O(CO)-, -(CO)-O-, -(CO)NR-, -NR(CO)-, -O-, -SO-, -SO₂-, -NR, worin R (C₁-C₃)-Alkyl, (C₁-C₈)-Alkanoyl, (C₇-C₁₆)-Aralkanoyl, (C₆-C₁₂)-Aroyl, oder Wasserstoff bedeutet,
r 1, 2, 3 oder 4 ist,
D eine Bindung oder Wasserstoff oder
einen verzweigten oder unverzweigten aliphatischen (C₁-C₁₀)-Alkandiyl-Rest, oder
einen verzweigten oder unverzweigten (C₁-C₁₀)-Alkendiyl-Rest, einen (C₂-C₁₀)-Alkindiyl-Rest oder einen (C₂-C₁₀)-Alkenindiyl-Rest, der jeweils eine oder mehrere C-C-Mehrfachbindungen enthalten kann,
W eine Bindung oder
Wasserstoff oder
einen (C₃-C₁₀) cycloaliphatischen Alkyl-, Alkenyl-, Alkinyl- oder
Alkeninyl-Rest oder
einen (C₆-C₁₆)-Ary)rest oder einen 5- oder 6-gliedrigen Heteroarylrest, wobei mindestens eine der Variablen C oder D oder W nicht eine Bindung bedeuten und U nur dann in der Bedeutung einer Heteratomgruppierung steht, wenn C nicht eine Bindung bedeutet oder wenn D und/oder W nicht eine Bindung bedeuten und
C und/oder W, sofern diese keine Bindung oder Wasserstoff bedeuten und vorzugsweise ihrerseits substituiert sind,
R⁴ Wasserstoff, (C₁-C₆)-Alkyl, Phenyl, Phenyl-(C₁-C₄)-alkyl,
R⁵ einen unsubstituierten oder substituierten verzweigten oder unverzweigten, aliphatischen (C₁-C₈)-Alkyrest bedeutet, der eine saure Gruppe trägt und weiterhin einen oder mehrere Substituenten tragen kann, und
n 0 oder 1 bedeutet.

2. Verbindungen gemäß Anspruch 1 nach der allgemeinen Formel I 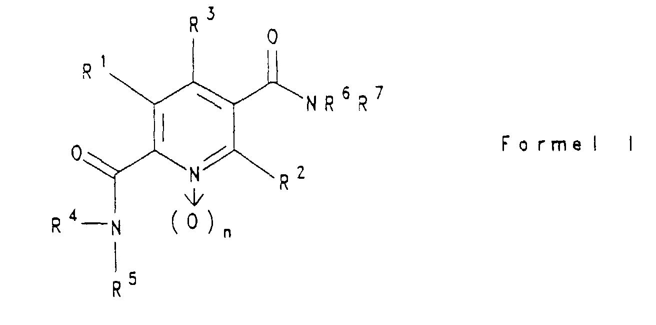 worin
R¹ Hydroxy oder C₁-C₆-Alkoxy,
R² und R³ gleich oder verschieden sind und Wasserstoff, unsubstituiertes oder substituiertes (C₁-C₆)-Alkyl, (C₁-C₆)-Alkoxy, Halogen, insbesondere Fluor, Chlor oder Brom, Nitril, Hydroxy, Amino,
R⁶ Wasserstoff, (C₁-C₆)-Alkyl oder eine N-Schutzgruppe wie (C₁-C₈)-Alkanoyl, (C₁-C₆)-Alkylcarbamoyl, (C₁-C₆)-Alkoxycarbonyl, Benzyloxycarbonyl, (C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl oder (C₁-C₆)-Alkoxycarbonyloxy-(C₁-C₆)-alkyl, ein 1, 2, 3 oder 4-wertiges physiologisch verwendbares Kation, insbesondere Na⊕, K⊕, Mg²⊕, Ca²⊕, Al³⊕ oder ein Ammoniumion, ggf. 1-3fach substituiert mit (C₁-C₈)-Alkyl, (C₁-C₈)-Hydroxyalkyl, (C₁-C₄)-Alkoxy-(C₁-C₈)-alkyl, Phenyl, Benzyl oder (C₁-C₈)-Alkyl, welches 1 bis 3-fach substituiert sein kann mit Hydroxy oder (C₁-C₄)-Alkoxy, oder ein Kation eines basischen Aminosäurederivates,
R⁷ einen Rest der Formel II, ausgenommen -SO₂H, bedeutet
-Y-[C-U]ᵣ-D-W (II)
in welchem
Y -SO₂- oder -CO- bedeutet,
C eine Bindung oder
einen verzweigten oder unverzweigten aliphatischen (C₁-C₁₆)-Alkandiyl oder cycloaliphatischen (C₃-C₁₀)-Alkandiylrest oder einen verzweigten oder unverzweigten (C₂-C₁₆)-Alkendiyl- oder
Cycloalkendiyl-Rest, oder einen (C₂-C₁₆)-Alkindiyl-Rest oder einen (C₂-C₁₆)-Alkenindiyl-Rest bedeutet, der jeweils eine oder mehrere C-C-Mehrfachbindungen enthalten kann,
U eine Bindung oder
Wasserstoff oder
einen Rest aus der Reihe folgender Heteroatomgruppierungen -CO-, -O(CO)-, -(CO)-O-, -(CO)NR-, -NR(CO)-, -O-, -SO-, -SO₂-, -NR, worin R (C₁-C₃)-Alkyl, (C₁-C₈)-Alkanoyl, (C₇-C₁₆)-Aralkanoyl, (C₆-C₁₂)-Aroyl oder Wasserstoff bedeutet,
r 1, 2, 3 oder 4 ist,
D eine Bindung oder Wasserstoff oder
einen verzweigten oder unverzweigten aliphatischen (C₁-C₁₀)-Alkandiyl-Rest, oder
einen verzweigten oder unverzweigten (C₁-C₁₀)-Alkendiyl-Rest, einen (C₂-C₁₀)-Alkindiyl-Rest oder einen (C₂-C₁₀)-Alkenindiyl-Rest, der jeweils eine oder mehrere C-C-Mehrfachbindungen enthalten kann,
W eine Bindung oder
Wasserstoff oder
einen (C₃-C₁₀) cycloaliphatischen Alkyl-, Alkenyl-, Alkinyl- oder Alkeninyl-Rest oder
einen (C₆-C₁₆)-Arylrest oder einen 5- oder 6-gliedrigen Heteroarylrest, wobei mindestens eine der Variablen C oder D oder W nicht eine Bindung bedeuten und U nur dann in der Bedeutung einer Heteratomgruppierung steht, wenn C nicht eine Bindung bedeutet oder wenn D und/oder W nicht eine Bindung bedeuten und
C und/oder W, sofern diese keine Bindung oder Wasserstoff bedeuten und vorzugsweise ihrerseits substituiert sind durch eine Kombination von bis zu 5 gleichen oder verschiedenen Substituenten aus der Reihe Hydroxy, Halogen, Cyano, Trifluormethyl, Nitro, Carboxyl, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)-alkyl,(C₁-C₁₂)-Alkoxy-(C₁-C₁₂)-alkoxy, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂)-Alkenylcarbonyl,(C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl,(C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy, (C₃-C₈)-Cycloalkoxycarbonyloxy, (C₃-C₁₂)-Alkenyloxycarbonyloxy, (C₃-C₁₂)-Alkinyloxycarbonyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-Aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-Alkoxy-(C₁-C₁₀)-alkyl)carbamoyl, N-(C₁-C₁₀)-Alkyl-N-((C₆-C₁₆)-Aryloxy(C₁-C₁₀)-alkyl)carbamoyl, N-(C₁-C₁₀)-Alkyl-N-((C₇-C₁₆)-Aralkyloxy-(C₁-C₁₀)-alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy N-((C₁-C₁₀)-Alkoxy-(C₁-C₁₀)-alkyl)carbamoyloxy, N-((C₆-C₁₆)-Aryloxy-(C₁-C₁₀)-alkyl)carbamoyloxy, N-((C₇-C₁₆)-Aralkyloxy-(C₁-C₁₀)-alkyl)carbamoyloxy, N-(C₁-C₁₀)-Alkyl-N-((C₁-C₁₀)-Alkoxy-(C₁-C₁₀)-alkyl)carbamoyloxy, N-(C₁-C₁₀)-Alkyl-N-((C₆-C₁₆)-Aryloxy-(C₁-C₁₀)-alkyl)carbamoyloxy,
N-(C₁-C₁₀)-Alkyl-N-((C₇-C₁₆)-Aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-(C₁-C₁₀)-Alkyl-(C₇-C₁₀)-Aralkylamino, N-(C₁-C₁₀)-Alkyl-N-(C₆-C₁₂)-Arylamino, (C₁-C₁₂)-Alkoxy-amino, (C₁-C₁₂)-Alkoxy-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino,(C₃-C₈)-Cycloalkanoyl-N-(C₁-C₁₀)-alkylamino,(C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di(C₁-C₁₀)-alkylamino-(C₁-C₁₀)-alkyl,(C₃-C₈)cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl,(C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl,(C₃-C₈)-cycloalkylsulfamoyl, N-(C₆-C₁₆)-Arylsulfamoyl, N-(C₇-C₁₆)-Aralkylsulfamoyl, N-(C₁-C₁₀)-Alkyl-N-(C₆-C₁₆)-Arylsulfamoyl, N-(C₁-C₁₀)-Alkyl-N-(C₇-C₁₆)-Aralkylsulfamoyl,
(C₁-C₁₀)-Alkyl-sulfonamido,
N-((C₁-C₁₀)-alkyl)-(C₁-C₁₀)-alkylsulfonamido, (C₇-C₁₆)-Aralkylsulfonamido,
N-((C₁-C₁₀)-alkyl-(C₇-C₁₆)-Aralkylsulfonamido,
wobei die Reste, die einen Arylrest enthalten, ihrerseits am Aryl substituiert sein können mit 1, 2, 3, 4 oder 5 gleichen oder verschiedenen Substituenten aus der Reihe, Hydroxy, Halogen, Cyano, Trifluormethyl, Nitro, Carboxyl, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy(C₁-C₁₂)-alkyl, (C₁-C₁₂)-Alkoxy-(C₁-C₁₂)-alkoxy, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂)-Alkenylcarbonyl,(C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl,(C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy,(C₃-C₁₂)-Alkinylcarbonyloxy,
(C₁-C₁₂)-Alkoxycarbonyloxy, (C₁-C₁₂)-Alkoxy-(C₁-C₁₂)-alkoxycarbonyloxy, (C₆-C₁₂)-Aryloxycarbonyloxy, (C₇-C₁₆)-Aralkyloxycarbonyloxy,(C₃-C₈)-Cycloalkoxycarbonyloxy, (C₃-C₁₂)-Alkenyloxycarbonyloxy,(C₃-C₁₂)-Alkinyloxycarbonyloxy, Carbamoyl, N-(C₁-C₁₂)-Alkylcarbamoyl, Hydroxy-(C₁-C₄)-alkylcarbamoyl, Acyloxy-(C₁-C₄)-alkylcarbamoyl, N,N-Di(C₁-C₁₂-alkylcarbamoyl, N- N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-Aralkylcarbamoyl, N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-Alkoxy-(C₁-C₁₀)-alkyl)carbamoyl, N-(C₁-C₁₀)-Alkyl-N-((C₆-C₁₆)-Aryloxy-(C₁-C₁₀)-alkyl)carbamoyl, (N-(C₁-C₁₀)-Alkyl-N-((C₇-C₁₆)-Aralkyloxy-(C₁-C₁₀)-alkyl)carbamoyl,
Carbamoyloxy, N-(C₁-C₁₂)-Alkylcarbamoyloxy, N,N-Di-(C₁-C₁₂)-alkylcarbamoyloxy, N-(C₃-C₈)-Cycloalkylcarbamoyloxy, N-(C₆-C₁₆)-Arylcarbamoyloxy, N-(C₇-C₁₆)-Aralkylcarbamoyloxy, N-(C₁-C₁₀)-Alkyl-N-(C₆-C₁₂)-arylcarbamoyloxy, N-(C₁-C₁₀)-Alkyl-N-(C₇-C₁₆)-Aralkylcarbamoyloxy N-((C₁-C₁₀)-Alkoxy-(C₁-C₁₀)-alkyl)carbamoyloxy, N-((C₆-C₁₆)-Aryloxy-(C₁-C₁₀)-alkyl)carbamoyloxy. N-((C₇-C₁₆)-Aralkyloxy-(C₁-C₁₀)-alkyl)carbamoyloxy, N-(C₁-C₁₀)-Alkyl-N-((C₁-C₁₀)-Alkoxy-(C₁-C₁₀)-alkyl)carbamoyloxy, N-(C₁-C₁₀)-Alkyl-N-((C₆-C₁₆)-Aryloxy-(C₁-C₁₀)-alkyl)carbamoyloxy, N-(C₁-C₁₀)-Alkyl-N-((C₇-C₁₆)-Aralkyloxy-(C₁-C₁₀)-alkyl)carbamoyloxy,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino N-(C₆-C₁₂)-Arylamino, N-(C₇-C₁₁)-Aralkylamino, N-(C₁-C₁₀)-Alkyl-N-(C₇-C₁₆)-Aralkylamino, N-(C₁-C₁₀)-Alkyl-N-(C₆-C₁₂)-Arylamino, (C₁-C₁₂)-Alkoxy-amino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroylamino, (C₇-C₁₆)-Aralkanoylamino, (C₁-C₁₂)-Alkanoyl-N-(C₁-C₁₀)-alkylamino,(C₃-C₈)-Cycloalkanoyl-N-(C₁-C₁₀)-alkylamino, (C₆-C₁₂)-Aroyl-N-(C₁-C₁₀)-alkylamino, (C₇-C₁₆)-Aralkanoylamino, -N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di(C₁-C₁₀)-alkylamino-(C₁-C₁₀)-alkyl, (C₃-C₈)cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkylsulfonyl,
Sulfamoyl, N-(C₁-C₁₀)-Alkylsulfamoyl, N,N-Di-(C₁-C₁₀)-alkylsulfamoyl, (C₃-C₈)-cycloalkylsulfamoyl, N-(C₆-C₁₆)-Arylsulfamoyl, N-(C₇-C₁₆)-Aralkylsulfamoyl, N-(C₁-C₁₀)-Alkyl-N-(C₆-C₁₆)-Arylsulfamoyl, N-(C₁-C₁₀)-Alkyl-N-(C₇-C₁₆-Aralkylsulfamoyl,
(C₁-C₁₀)-Alkyl-sulfonamido, N-((C₁-C₁₀)-alkyl)-(C₁-C₁₀)-alkylsulfonamido, (C₇-C₁₆)-Aralkylsulfonamido, N-((C₁-C₁₀)-alkyl-(C₇-C₁₆)-Aralkylsulfonamido,
R⁴ Wasserstoff, (C₁-C₆)-Alkyl, Phenyl, Phenyl-(C₁-C₄)-alkyl,
R⁵ einen unsubstituierten oder substituierten, verzweigten oder unverzweigten, aliphatischen (C₁-C₈)-Alkylrest, der eine Carboxylgruppe trägt, bedeutet, der einfach oder mehrfach, vorzugsweise ein- oder zweifach substituiert sind mit
Hydroxy Fluor, Chlor, Hydroxy-(C₁-C₄)-alkyl, (C₁-C₁₂)-Alkyl, (C₃-C₈)-Cycloalkyl, (C₆-C₁₂)-Aryl, (C₇-C₁₆)-Aralkyl, (C₁-C₆)-Alkoxy, (C₆-C₁₂)-Aryloxy, (C₇-C₁₆)-Aralkyloxy, -O-[CH₂₋]xC_{f}H_{(2f+1-g)}F_{g},
wobei die Reste, die einen Arylrest enthalten, ihrerseits am Aryl substituiert sein können mit 1, 2, 3, 4 oder 5 gleichen oder verschiedenen Substituenten aus der Reihe
Hydroxy, Halogen, Cyano, Trifluormethyl, Nitro, Carboxyl, (C₁-C₆)-Alkyl, (C₃-C₈)-Cycloalkyl, (C₁-C₆)-Alkoxy,
und
n 0 und 1,
f 1 bis 8, vorzugsweise 1 bis 5,
g 0, 1 bis (2f + 1) und
x 0 bis 8, vorzugsweise 0 bis 1 bedeuten.

3. Verbindungen gemäß den Ansprüchen 1 oder 2 nach der allgemeinen Formel I,
worin
R¹ Hydroxy, (C₁-C₆)-Alkoxy,
R² und R³ Wasserstoff,
R⁶ Wasserstoff, (C₁-C₆)-Alkyl oder eine N-Schutzgruppe wie (C₁-C₈)-Alkanoyl, (C₁-C₆)-Alkylcarbamoyl, (C₁-C₆)-Alkoxycarbonyl, Benzyloxycarbonyl, (C₁-C₁₀)-Acyloxy-(C₁-C₆)-alkyl, vorzugsweise (C₁-C₁₀)-Alkanoyloxy-(C₁-C₆)-alkyl, Benzoyloxy-(C₁-C₆)-alkyl, Benzyloxycarbonyloxy-(C₁-C₆)-alkyl oder (C₁-C₆)-Alkoxycarbonyloxy-(C₁-C₆)-alkyl, ein 1, 2, 3 oder 4-wertiges physiologisch verwendbares Kation, insbesondere Na⊕, K⊕, Mg²⊕, Ca²⊕, Al³⊕ oder ein Ammoniumion, ggf. 1-3fach substituiert mit (C₁-C₈)-Alkyl, (C₁-C₈)-Hydroxyalkyl, (C₁-C₄)-Alkoxy-(C₁-C₈)-alkyl, Phenyl, Benzyl oder (C₁-C₈)-Alkyl, welches 1 bis 3-fach substituiert sein kann mit Hydroxy oder (C₁-C₄)-Alkoxy, oder ein Kation eines basischen Aminosäurederivates,
R⁷ einen Rest der Formel II, ausgenommen -SO₂H, bedeutet
-Y-[C-U]ᵣ-D-W (II)
in welchen
Y -SO₂- bedeutet,
C eine Bindung, oder
einen verzweigten oder unverzweigten aliphatischen (C₁-C₁₆)-Alkandiyl-rest,
U eine Bindung oder
Wasserstoff oder -O- bedeutet,
r 1 ist,
D eine Bindung oder
Wasserstoff oder
einen verzweigten oder unverzweigten aliphatischen (C₁-C₄)-Alkandiyl-Rest,
W eine Bindung oder
Wasserstoff oder
einen (C₃-C₁₀)-cycloaliphatischen Alkyl-, Alkenyl-, Alkinyl- oder
Alkeninyl-Rest oder
einen (C₆-C₁₆)-Arylrest oder einen 5- oder 6-gliedrigen Heteroarylrest, wobei mindestens eine der Variablen C oder D oder W nicht eine Bindung bedeuten und U nur dann in der Bedeutung einer Heteratomgruppierung steht, wenn C nicht eine Bindung bedeutet oder wenn D und/oder W nicht eine Bindung bedeuten
und
C, D und/oder W, sofern diese keine Bindung oder Wasserstoff bedeuten, vorzugsweise ihrerseits substituiert sind durch eine Kombination von bis zu 5 gleichen oder verschiedenen Substituenten aus der Reihe
Hydroxy, Halogen, Cyano, Trifluormethyl, Nitro, Carboxyl, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)-alkyl,(C₁-C₁₂)-Alkoxy-(C₁-C₁₂)-alkoxy, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g})F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂)-Alkenylcarbonyl,(C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl,(C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
Carbamoyl, N-(C₁-C₁₂)-Alkylcarbamoyl, N,N-Di-(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-Aralkylcarbamoyl, N-((C₁-C₁₀)-Alkoxy-(C₁-C₁₀)-alkyl)carbamoyl,
N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-Alkoxy-(C₁-C₁₀)-alkyl)carbamoyl, N-(C₁-C₁₀)-Alkyl-N-((C₆-C₁₆)-Aryloxy-(C₁-C₁₀)-alkyl)carbamoyl, N-(C₁-C₁₀)-Alkyl-N-((C₇-C₁₆)-Aralkyloxy-(C₁-C₁₀)-alkyl)carbamoyl,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-(C₁-C₅)-Alkyl-N-(C₇-C₁₀)-Aralkylamino, N-(C₁-C₅)-Alkyl-N-(C₆-C₁₂)-Arylamino, (C₁-C₁₂)-Alkoxy-amino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroyl-amino, (C₇-C₁₆)-Aralkanoylamino, (C₁-C₁₂)-Alkanoyl-N-(C₁-C₁₀)-alkylamino,(C₃-C₈)-Cycloalkanoyl-N-(C₁-C₁₀)-alkylamino,(C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di(C₁-C₁₀)-alkylamino-(C₁-C₁₀)-alkyl,(C₃-C₈)cycloalkylamino-(C₁-C₁₀)-alkyl, (C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkyl-sulfonyl,
wobei die Reste, die einen Arylrest enthalten, ihrerseits am Aryl substituiert sein können mit 1, 2, 3, 4 oder 5 gleichen oder verschiedenen Substituenten aus der Reihe Wasserstoff, Hydroxy,
Halogen, Cyano, Trifluormethyl, Nitro, Carboxyl, (C₁-C₁₂)-Alkyl, (C₃-C₈)-Cycloalkyl, (C₆-C₁₂)-Aryl, (C₇-C₁₆)-Aralkyl, (C₃-C₁₂)-Alkenyl, (C₃-C₁₂)-Alkinyl, (C₁-C₁₂)-Alkoxy, (C₁-C₁₂)-Alkoxy-(C₁-C₁₂)-alkyl, (C₁-C₁₂)-Alkoxy-(C₁-C₁₂)-alkoxy, (C₆-C₁₂)-Aryloxy, (C₇-C₁₆)-Aralkyloxy, (C₁-C₈)-Hydroxyalkyl, -O-[CH₂₋]_{X}C_{f}H_{(2f+1-g)}F_{g}, -OCF₂Cl, -OCF₂-CHFCl,
(C₁-C₁₂)-Alkylcarbonyl, (C₃-C₈)-Cycloalkylcarbonyl, (C₆-C₁₂)-Arylcarbonyl, (C₇-C₁₆)-Aralkylcarbonyl, Cinnamoyl, (C₃-C₁₂)-Alkenylcarbonyl, (C₃-C₁₂)-Alkinylcarbonyl,
(C₁-C₁₂)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₃-C₁₂)-Alkenyloxycarbonyl, (C₃-C₁₂)-Alkinyloxycarbonyl,
(C₁-C₁₂)-Alkylcarbonyloxy, (C₃-C₈)-Cycloalkylcarbonyloxy, (C₆-C₁₂)-Arylcarbonyloxy, (C₇-C₁₆)-Aralkylcarbonyloxy, Cinnamoyloxy, (C₃-C₁₂)-Alkenylcarbonyloxy, (C₃-C₁₂)-Alkinylcarbonyloxy,
Carbamoyl, N-(C₁-C₁₂)-Alkylcarbamoyl, Hydroxy-(C₁-C₄)-alkylcarbamoyl, Acyloxy-(C₁-C₄)-alkylcarbamoyl, N,N-Di(C₁-C₁₂)-alkylcarbamoyl, N-(C₃-C₈)-Cycloalkylcarbamoyl, N-(C₆-C₁₆)-Arylcarbamoyl, N-(C₇-C₁₆)-Aralkylcarbamoyl,N-(C₁-C₁₀)-Alkyl-N-(C₆-C₁₆)-arylcarbamoyl, N-(C₁-C₁₀)-Alkyl-N-(C₇-C₁₆)-Aralkylcarbamoyl,
N-((C₁-C₁₀)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl, N-(C₁-C₁₀)-Alkyl-N-((C₁-C₁₀)-Alkoxy(C₁-C₁₀)-alkyl)carbamoyl, N-(C₁-C₁₀)-Alkyl-N-((C₆-C₁₆)-Aryloxy-(C₁-C₁₀)-alkyl)carbamoyl, N-(C₁-C₁₀)-Alkyl-N-((C₇-C₁₆)-Aralkyloxy-(C₁-C₁₀)-alkyl)carbamoyl,
Amino, (C₁-C₁₂)-Alkylamino, Di-(C₁-C₁₂)-alkylamino, (C₃-C₈)-Cycloalkylamino, (C₃-C₁₂)-Alkenylamino, (C₃-C₁₂)-Alkinylamino, N-(C₆-C₁₂)-Arylamino, N-(C₇-C₁₁)-Aralkylamino, N-(C₁-C₅)-Alkyl-(C₇-C₁₀)-Aralkylamino, N-(C₁-C₅)-Alkyl-(C₆-C₁₂)-Arylamino, (C₁-C₁₂)-Alkoxy-amino, (C₁-C₁₂)-Alkoxy-N-(C₁-C₁₀)-alkylamino,
(C₁-C₁₂)-Alkanoylamino, (C₃-C₈)-Cycloalkanoylamino, (C₆-C₁₂)-Aroyl-amino, (C₇-C₁₆)-Aralkanoylamino, (C₁-C₁₂)-Alkanoyl-N-(C₁-C₁₀)-alkylamino,(C₃-C₈)-Cycloalkanoyl-N-(C₁-C₁₀)-alkylamino,(C₆-C₁₂)-Aroyl-N-(C₁-C₁₀)-alkylamino, (C₇-C₁₁)-Aralkanoyl-N-(C₁-C₁₀)-alkylamino,
(C₁-C₁₂)-Alkanoylamino-(C₁-C₈)-alkyl, (C₃-C₈)-Cycloalkanoylamino-(C₁-C₈)-alkyl, (C₆-C₁₆)-Aroylamino-(C₁-C₈)-alkyl, (C₇-C₁₆)-Aralkanoylamino-(C₁-C₈)-alkyl, Amino-(C₁-C₁₀)-alkyl, N-(C₁-C₁₀)-alkylamino-(C₁-C₁₀)-alkyl, N,N-Di(C₁-C₁₀)-alkylamino-(C₁-C₁₀)-alkyl, (C₃-C₈)cycloalkylamino-(C₁-C₁₀)-alkyl,
(C₁-C₁₂)-Alkylmercapto, (C₁-C₁₂)-Alkylsulfinyl, (C₁-C₁₂)-Alkylsulfonyl, (C₆-C₁₆)-Arylmercapto, (C₆-C₁₆)-Arylsulfinyl, (C₆-C₁₆)-Arylsulfonyl, (C₇-C₁₆)-Aralkylmercapto, (C₇-C₁₆)-Aralkylsulfinyl, (C₇-C₁₆)-Aralkyl-sulfonyl,
R⁴ Wasserstoff, (C₁-C₄)-Alkyl,
R⁵ einen verzweigten oder unverzweigten (C₁-C₄)-Alkylrest bedeutet, der eine Carboxylgruppe trägt und der ein- oder zweifach substituiert ist, mit
Hydroxy, Fluor, Chlor, (C₁-C₆)-Alkyl, (C₃-C₈)-Cycloalkyl, Phenyl, Benzyl und
n 0 und 1,
f 1 bis 5,
g 0,1 bis (2f + 1) und
x 0 oder 1 bedeuten.

4. Verbindungen nach einem oder mehreren der Ansprüche 1 bis 3 nach der allgemeinen Formel I, in der
R¹ Hydroxy, (C₁-C₆)-Alkoxy,
R² und R³ Wasserstoff,
R⁶ Wasserstoff oder ein 1 oder 2-wertiges physiologisch verwendbares Kation, insbesondere Na⊕, K⊕, Mg⊕ oder Ca⊕ oder ein Ammoniumion,
R⁷ einen Rest der Formel II, ausgenommen -SO₂H, bedeutet,
-Y-[C-U]ᵣ-D-W (II),
in welchem
Y -SO₂- bedeutet,
C eine Bindung oder
einen (C₁-C₆)-Alkandiyl-Rest,
U eine Bindung
r 1 ist,
D eine Bindung oder
Wasserstoff
W Wasserstoff oder einen Phenylrest bedeutet, wobei mindestens eine der Variablen C oder D oder W nicht eine Bindung bedeuten und
C, D und/oder W substituiert sein kann durch Fluor, Chlor, Trifluormethyl, (C₁-C₁₆)-Alkyl, (C₃-C₈)-Cycloalkyl, Benzyl, Phenyl, (C₁-C₆)-Alkoxy, Phenoxy oder -O-[CH₂₋]_{X}C_{f}H_{(2f +1-g)}F_{g},
Carbamoyl, N-(C₁-C₁₀)-Alkylcarbamoyl, N,N-Di-(C₁-C₈)-alkylcarbamoyl,
N-(C₃-C₈)-Cycloalkylcarbamoyl, N-Phenylcarbamoyl, N-(C₇-C₁₁)-Phenylalkylcarbamoyl, N-(C₁-C₈)-Alkyl-N-(C₆-C₁₆)phenylcarbamoyl, N-(C₁-C₈)-Alkyl-N-(C₇-C₁₁)-phenylalkylcarbamoyl, N-((C₁-C₁₀)-Alkoxy-(C₁-C₁₀)-alkylcarbamoyl, N-Phenoxy-(C₁-C₈)-alkylcarbamoyl, N-((C₇-C₁₆)-Phenylalkyloxy-(C₁-C₈)-alkylcarbamoyl, N-(C₁-C₈)-Alkyl-N-((C₁-C₆)-Alkoxy-(C₁-C₈)-alkylcarbamoyl, N-(C₁-C₈)-Alkyl-N-Phenoxy-(C₁-C₈)-alkylcarbamoyl, N-(C₁-C₈)-Alkyl-N-((C₇-C₁₆)-Aralkyloxy-(C₁-C₁₀)-alkylcarbamoyl,
(C₁-C₁₀)-Alkanoylamino, (C₃-C₈)-Cycloalkanoylamino, Phenylamino, (C₇-C₁₁)-Phenylalkanoylamino, (C₁-C₈)-Alkanoyl-N-(C₁-C₁₀)-alkylamino, (C₃-C₈)-Cycloalkanoyl-N-(C₁-C₆)-alkylamino, Benzoyl-N-(C₁-C₆)-alkylamino, (C₇-C₁₁)-Phenylalkanoyl-N-(C₁-C₁₀)-alkylamino,
(C₁-C₁₀)-Alkanoylamino-(C₁-C₄)-alkyl, (C₃-C₈)-Cycloalkanoylamino-(C₁-C₄)-alkyl, Phenoylamino-(C₁-C₆)-alkyl, (C₇-C₁₁)-Phenylalkanoylamino-(C₁-C₆)-alkyl,
wobei die Reste, die einen Arylrest enthalten, ihrerseits am Aryl substituiert sein können mit 1, 2, 3, 4 oder 5 gleichen oder verschiedenen Substituenten aus der Reihe Hydroxy, Fluor, Chlor, Trifluormethyl, Carboxyl, (C₁-C₄)-Alkoxy, Benzyloxy, Phenoxy,
(C₁-C₁₂)-Alkoxycarbonyl, (C₃-C₈)-Cycloalkylcarbonyl, Phenoxycarbonyl, (C₇-C₁₁)-Phenylalkoxycarbonyl,
(C₁-C₁₂)-Alkylcarbonyloxy, (C₃-C₈)-Cycloalkylcarbonyloxy, Benzoyloxy, (C₇-C₁₁)-Phenylalkylcarbonyloxy,
Carbamoyl, N-(C₁-C₆)-Alkylcarbamoyl, N,N-Di-(C₁-C₆)-alkylcarbamoyl, N-(C₃-C₈)-Cycloalkylcarbamoyl, N-Phenylcarbamoyl, N-(C₇-C₁₁)-Phenylalkylcarbamoyl, Hydroxy- Hydroxy-(C₁-C₄)-alkylcarbamoyl, Acyloxy-(C₁-C₄)-alkylcarbamoyl,
Carbamoyloxy, N- N-(C₁-C₆)-Alkylcarbamoyloxy, N,N-Di-(C₁-C₆)-alkylcarbamoyloxy, N-(C₃-C₈)-cycloalkylcarbamoyloxy
R⁴ Wasserstoff,
R⁵ eine Methyl-Gruppe bedeutet, die mit Carboxyl substituiert ist und einen weiteren Substituenten hat aus der Reihe
Hydroxy, Hydroxy-(C₁-C₄)-alkyl, Phenyl, Benzyl, (C₁-C₄)-Alkoxy, (C₁-C₅)-Alkyl, Benzyloxy, Phenoxy, und
n 0 und 1,
f 1 bis 5,
g 0,1 bis (2f + 1) und
x 0 oder 1
bedeuten.

5. Verbindungen gemäß einem oder mehreren der Ansprüche 1 bis 4 der allgemeinen Formel I, in der
R¹ Hydroxy, (C₁-C₆)-Alkoxy,
R² und R³ Wasserstoff,
R⁶ Wasserstoff oder ein 1 oder 2-wertiges physiologisch verwendbares Kation, insbesondere Na⊕, K⊕, Mg²⊕ oder Ca²⊕ oder ein Ammoniumion, insbesondere H₃N⊕C(CH₂OH)₃,
R⁷ einen Rest der Formel II, ausgenommen -SO₂H, bedeutet
-Y-[C-U]ᵣ-D-W (II),
in welchem
Y -SO₂-,
C eine Bindung oder
(C₁-C₁₆)-Alkandiyl,
U eine Bindung,
r 1 ist,
D eine Bindung oder Wasserstoff
W einen Phenylrest bedeutet, wobei W einfach oder zweifach substituiert sein kann durch Fluor, Chlor, (C₁-C₆)-Alkyl, (C₇-C₁₆)-Aralkyl, Phenyl, (C₁-C₆)-Alkoxy, und W zusätzlich einfach substituiert ist durch Phenyl, Phenoxy, -O-[CH₂]_{X}C_{f}H_{(2f+1-g)}F_{g},
Carbamoyl, N-(C₁-C₁₀)-Alkylcarbamoyl, N,N-Di-(C₁-C₈)-alkylcarbamoyl, N-(C₃-C₈)-Cycloalkylcarbamoyl, N-Phenylcarbamoyl, N-(C₇-C₁₁)-Phenylalkylcarbamoyl, N-((C₁-C₄)-Alkoxy-(C₁-C₄)-alkyl)carbamoyl, N-Phenoxy-(C₁-C₄)-alkyl)carbamoyl,
(C₁-C₁₀)-Alkanoylamino-(C₁-C₂)-alkyl, (C₃-C₈)-Cycloalkanoylamino-(C₁-C₂)-alkyl, Benzoylamino-(C₁-C₂)-alkyl oder (C₇-C₁₁)-Phenylalkanoylamino-(C₁-C₂)-alkyl,
wobei die Reste, die einen Arylrest enthalten, ihrerseits am Aryl substituiert sein können mit 1, 2, 3, 4 oder 5 gleichen oder verschiedenen Substituenten aus der Reihe Hydroxy, Carboxyl, (C₁-C₄)-Alkoxy, Phenoxy, Benzyloxy, Fluor, Chlor, Trifluormethyl, Carbamoyl, N-(C₁-C₆)-Alkylcarbamoyl, N,N-Di-(C₁-C₆)-alkylcarbamoyl, N-(C₃-C₈)-Cycloalkylcarbamoyl, N-Phenylcarbamoyl, N-(C₇-C₁₁)-Phenalkylcarbamoyl,
R⁴ Wasserstoff,
R⁵ eine Methyl-Gruppe bedeutet, die mit Carboxyl substituiert ist,
und
n 0,
f 1 bis 5,
g 0,1 bis (2f+1) und
x 0 oder 1
bedeuten.

6. Verfahren zur Herstellung von Verbindungen nach einem oder mehreren der Ansprüche 1 bis 5, in denen gemäß Formel I R¹ bis R⁷ die Bedeutung gemäß der Ansprüche 1 bis 5 aufweisen, dadurch gekennzeichnet, daß man Pyridin-2-carbonsäureestercarboxylate der Formel 7 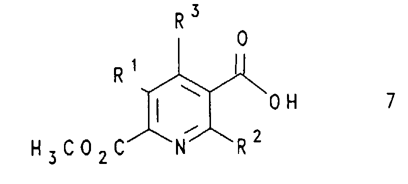 mit einem Sulfonsäureamidderivat der Formel 8
R⁶HN-R⁷ 8
und die hieraus entstehenden Verbindungen mit einem Amin der Formel 5
HNR⁴R⁵ 5
zu den Verbindungen der Formel Ia' umsetzt, 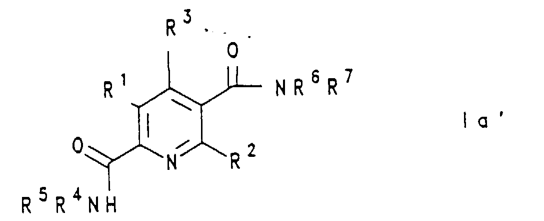 wobei man ggf. eine Oxidation zum Pyridin-N-oxid gemäß Formel Ib' anschließt

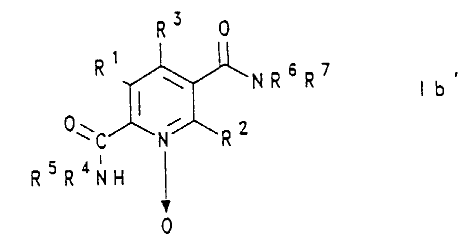

7. Verbindungen nach den Ansprüchen 1 bis 5 zur Anwendung gegen fibrotische Erkrankungen.

8. Verbindungen nach den Ansprüchen 1 bis 5 zur Anwendung als Fibrosuppressiva.

9. Verbindungen nach den Ansprüchen 1 bis 5 zur Inhibierung der Prolylhydroxylase.

10. Verbindungen nach den Ansprüchen 1 bis 5 zur Anwendung als Inhibitoren der Kollagenbiosynthese.

11. Arzneimittel, enthaltend mindestens eine Verbindung gemäß Anspruch 1 und ggf. einen pharmazeutisch verträglichen Träger.

12. Verbindungen gemäß Anspruch 1 zur Anwendung in der Behandlung von Störungen des Stoffwechsels von Collagen und collagenähnlichen Stoffen.

13. Verbindungen gemäß Anspruch 1 zur Anwendung in der Behandlung von fibrotischen Erkrankungen.

14. Verfahren zur Herstellung von Arzneimitteln zur Behandlung von fibrotischen Erkrankungen, dadurch gekennzeichnet, daß das Arzneimittel eine Verbindung gemäß Anspruch 1 enthält.

## Claims

1. A sulfonamidocarbonylpyridine-2-carboxamide or its pyridine-N-oxide of the formula I 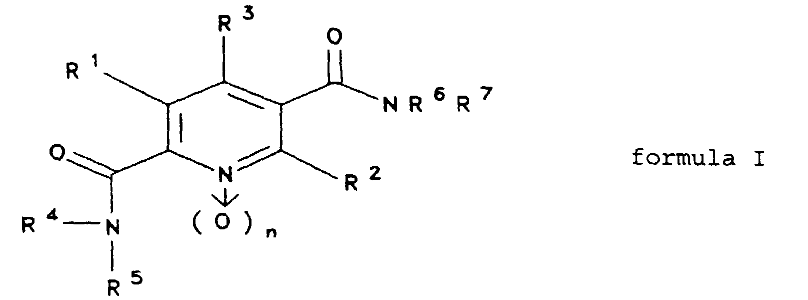 in which
R¹ is hydroxyl or (C₁-C₆)-alkoxy,
R² and R³ are identical or different and are hydrogen, unsubstituted or substituted (C₁-C₆)-alkyl, (C₁-C₆)-alkoxy, halogen, in -particular fluorine, chlorine or bromine, nitrile, hydroxyl or amino,
R⁶ is hydrogen, (C₁-C₆)-alkyl or an N-protective group such as (C₁-C₈)-alkanoyl, (C₁-C₆)-alkylcarbamoyl, (C₁-C₆)-alkoxycarbonyl, benzyloxycarbonyl, (C₁-C₁₀) -acyloxy- (C₁-C₆)-alkyl, preferably (C₁-C₁₀)-alkanoyloxy-(C₁-C₆)-alkyl, benzoyloxy-(C₁-C₆)-alkyl, benzyloxycarbonyloxy-(C₁-C₆)-alkyl or (C₁-C₆)-alkoxycarbonyloxy-(C₁-C₆)-alkyl, a 1-, 2-, 3- or 4-valent physiologically utilizable cation, in particular Na^{⊕}, K^{⊕}, Mg^{2⊕}, Ca^{2⊕} , Al^{3⊕} or an ammonium ion, optionally substituted 1-3 times by (C₁-C₈)-alkyl, (C₁-C₈)-hydroxyalkyl, (C₁-C₄)-alkoxy-(C₁-C₈)-alkyl, phenyl, benzyl or (C₁-C₈)-alkyl which can be substituted 1 to 3 times by hydroxyl or (C₁-C₄)-alkoxy, or a cation of a basic amino acid derivative,
R⁷ is a radical of the formula II, excluding -SO₂H,
-Y-[C-U]ᵣ-D-W (II)
in which
Y is -SO₂- or -CO-,
C is a bond or
a branched or unbranched aliphatic (C₁-C₁₆)-alkanediyl or cycloaliphatic (C₃-C₁₀)-alkanediyl radical or a branched or unbranched (C₂-C₁₆)-alkenediyl or cycloalkenediyl radical, or a (C₂-C₁₆)-alkynediyl radical or a (C₂-C₁₆)-alkenynediyl radical, which in each case can contain one or more C-C multiple bonds,
U is a bond or
hydrogen or
a radical from the series of the following heteroatom groups -CO-, -O(CO)-, -(CO)-O-, -(CO)NR-, -NR(CO)-, -O-, -SO-, -SO₂-, -NR, in which R is (C₁-C₃)-alkyl, C₁-C₈-alkanoyl, (C₇-C₁₆)-alkanoyl, (C₆-C₁₂)-aroyl or hydrogen,
r is 1, 2, 3 or 4,
D is a bond or hydrogen or a branched or unbranched aliphatic (C₁-C₁₀)-alkanediyl radical, or a branched or unbranched (C₁-C₁₀)-alkenediyl radical, a (C₂-C₁₀)-alkynediyl radical or a (C₂-C₁₀)-alkenynediyl radical, which in each case can contain one or more C-C multiple bonds,
W is a bond or
hydrogen or
a (C₃-C₁₀) cycloaliphatic alkyl, alkenyl, alkynyl or alkenynyl radical or
a (C₆-C₁₆)-aryl radical or a 5- or 6-membered heteroaryl radical, where at least one of the variables C or D or W is not a bond and U is only a heteroatom group if C is not a bond or if D and/or W are not a bond and
C and/or W, if these are not a bond or hydrogen for their part preferably are substituted,
R⁴ is hydrogen, (C₁-C₆)-alkyl, phenyl or phenyl-(C₁-C₄)-alkyl,
R⁵ is an unsubstituted or substituted, branched or unbranched, aliphatic (C₁-C₈)-alkyl radical which carries an acidic group and furthermore can carry one or more substituents, and
n is 0 or 1

2. A compound as claimed in claim 1 according to the formula I 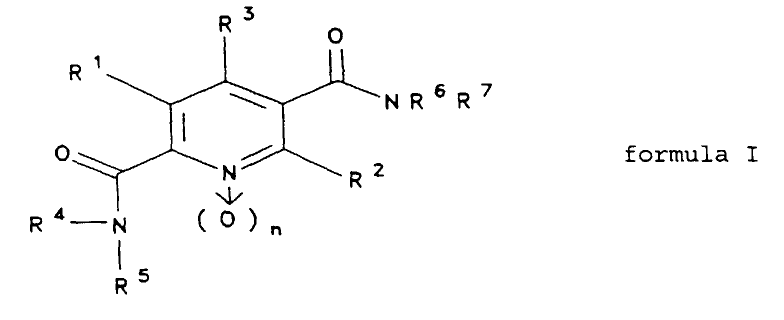 in which
R¹ is hydroxyl or (C₁-C₆)-alkoxy,
R² and R³ are identical or different and are hydrogen, unsubstituted or substituted (C₁-C₆)-alkyl, (C₁-C₆)-alkoxy, halogen, in particular fluorine, chlorine or bromine, nitrile, hydroxyl or amino,
R⁶ is hydrogen, (C₁-C₆)-alkyl or an N-protective group such as (C₁-C₈)-alkanoyl, (C₁-C₆) -alkylcarbamoyl, (C₁-C₆)-alkoxycarbonyl, benzyloxycarbonyl, (C₁-C₁₀)-acyloxy-(C₁-C₆)-alkyl, preferably (C₁-C₁₀)-alkanoyloxy-(C₁-C₆)-alkyl, benzoyloxy-(C₁-C₆)-alkyl, benzyloxycarbonyloxy-(C₁-C₆)-alkyl or (C₁-C₆-alkoxycarbonyloxy-(C₁-C₆)-alkyl, a 1-, 2-, 3- or 4-valent physiologically utilizable cation, in particular Na^{⊕}, K^{⊕}, Mg^{2⊕}, Ca^{2⊕}, Al^{3⊕} or an ammonium ion, optionally substituted 1-3 times by (C₁-C₈)-alkyl, (C₁-C₈)-hydroxyalkyl, (C₁-C₄)-alkoxy-(C₁-C₈)-alkyl, phenyl, benzyl or (C₁-C₈)-alkyl which can be substituted 1 to 3 times by hydroxyl or (C₁-C₄)-alkoxy, or a cation of a basic amino acid derivative,
R⁷ is a radical of the formula II, excluding -SO₂H,
-Y-[C-U]ᵣ-D-W (II)
in which
Y is -SO₂- or -CO-,
C is a bond or
a branched or unbranched aliphatic (C₁-C₁₆)-alkanediyl or cycloaliphatic (C₃-C₁₀)-alkanediyl radical or a branched or unbranched (C₂-C₁₆)-alkenediyl or cycloalkenediyl radical, or a (C₂-C₁₆)-alkynediyl radical or a (C₂-C₁₆)-alkenynediyl radical, which in each case can contain one or more C-C multiple bonds,
U is a bond or
hydrogen or
a radical from the series of the following heteroatom groups -CO-, -O(CO)-, -(CO)-O-, -(CO)NR-, -NR(CO)-, -O-, -SO-, -SO₂-, -NR, in which R is (C₁-C₃)-alkyl, (C₁-C₈)-alkanoyl, (C₇-C₁₆)-aralkanoyl, (C₆-C₁₂)-aroyl or hydrogen,
r is 1, 2, 3 or 4,
D is a bond or hydrogen or
a branched or unbranched (C₁-C₁₀)-alkanediyl radical, or
a branched or unbranched (C₁-C₁₀)-alkenediyl radical, a (C₂-C₁₀)-alkynediyl radical or a (C₂-C₁₀)-alkenynediyl radical, which in each case can contain one or more C-C multiple bonds,
W is a bond or
hydrogen or
a (C₃-C₁₀) cycloaliphatic alkyl, alkenyl, alkynyl or alkenynyl radical or
a (C₆-C₁₆)-aryl radical or a 5- or 6-membered heteroaryl radical, where at least one of the variables C or D or W is not a bond and U is only a heteroatom group if C is not a bond or if D and/or W are not a bond and
C and/or W, if these are not a bond or hydrogen, for their part preferably are substituted by a combination of up to 5 identical or different substituents from the series hydroxyl, halogen, cyano, trifluoromethyl, nitro, carboxyl, (C₁-C₁₂)-alkyl, (C₃-C₈)-cycloalkyl, (C₆-C₁₂)-aryl, (C₇-C₁₆)-aralkyl, (C₃-C₁₂)-alkenyl, (C₃-C₁₂)-alkynyl, (C₁-C₁₂)-alkoxy, (C₁-C₁₂)-alkoxy-(C₁-C₁₂) -alkyl, (C₁-C₁₂)-alkoxy-(C₁-C₁₂)-alkoxy, (C₆-C₁₂)-aryloxy, (C₇-C₁₆)-aralkyloxy, (C₁-C₈)-hydroxy-alkyl, -O-[CH₂]ₓC_{f}H_{(2f+1-g)}F_{g}, -OCF₂Cl, -OCF₂-CHFCl,
(C₁-C₁₂)-alkylcarbonyl, (C₃-C₈)-cycloalkylcarbonyl, (C₆-C₁₂)-arylcarbonyl, (C₇-C₁₆)-aralkylcarbonyl, cinnamoyl, (C₃-C₁₂)-alkenylcarbonyl, (C₃-C₁₂)-alkynylcarbonyl,
(C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₃-C₁₂)-alkenyloxycarbonyl, (C₃-C₁₂)-alkynyloxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₃-C₁₂)-alkenylcarbonyloxy, (C₃-C₁₂)-alkynylcarbonyloxy,
(C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₃-C₁₂)-alkenyloxycarbonyloxy, (C₃-C₁₂)-alkynyloxycarbonyloxy,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N-(C₆-C₁₆)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₀)-alkoxy-(C₁-C₁₀)-alkyl)carbamoyl, N-((C₆-C₁₆)-aryloxy-(C₁-C₁₀)-alkyl)-carbamoyl, N-((C₇-C₁₆)-aralkyloxy-(C₁-C₁₀)-alkyl)carbamoyl, N-(C₁-C₁₀)-alkyl-N-((C₁-C₁₀)-alkoxy-(C₁-C₁₀)-alkyl)carbamoyl, N-(C₁-C₁₀)-alkyl-N-((C₆-C₁₆)-aryloxy-(C₁-C₁₀)-alkyl)carbamoyl, N-(C₁-C₁₀)-alkyl-N-((C₇-C₁₆)-aralkyloxy-(C₁-C₁₀)-alkyl)carbamoyl,
carbamoyloxy, N-(C₁-C₁₂)-alkylcarbamoyloxy, N,N-di-(C₁-C₁₂)-alkylcarbamoyloxy, N-(C₃-C₈)-cycloalkylcarbamoyloxy, N-(C₆-C₁₆)-arylcarbamoyloxy, N-(C₇-C₁₆)-aralkylcarbamoyloxy, N-(C₁-C₁₀)-alkyl-N-(C₆-C₁₂)-arylcarbamoyloxy, N-(C₁-C₁₀)-alkyl-N-(C₇-C₁₆)-aralkylcarbamoyloxy, N-((C₁-C₁₀)-alkoxy-(C₁-C₁₀)-alkyl)-carbamoyloxy, N-((C₆-C₁₆)-aryloxy-(C₁-C₁₀)-alkyl)-carbamoyloxy, N-((C₇-C₁₆)-aralkyloxy-(C₁-C₁₀)-alkyl)-carbamoyloxy, N-(C₁-C₁₀)-alkyl-N-((C₁-C₁₀)-alkoxy-(C₁-C₁₀)-alkyl) carbamoyloxy, N-(C₁-C₁₀)-alkyl-N-((C₆-C₁₆)-aryloxy-(C₁-C₁₀)-alkyl)carbamoyloxy, N-(C₁-C₁₀)-alkyl-N-((C₇-C₁₆)-aralkyloxy-(C₁-C₁₀)-alkyl)carbamoyloxy, amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-(C₁-C₁₀)-alkyl-(C₇-C₁₀)-aralkylamino, N-(C₁-C₁₀)-alkyl-N-(C₆-C₁₂)-arylamino, (C₁-C₁₂)-alkoxyamino, (C₁-C₁₂)-alkoxy-(C₁-C₁₀)-alkylamino,
(C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino,
(C₁-C₁₂)-alkanoylamino-(C₁-C₈)-alkyl, (C₃-C₈)-cycloalkanoylamino-(C₁-C₈)-alkyl, (C₆-C₁₆)-aroylamino-(C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di-(C₁-C₁₀)-alkylamino-(C₁-C₁₀)-alkyl, (C₃-C₈)-cycloalkylamino-(C₁-C₁₀)-alkyl,
(C₁-C₁₂)-alkylmercapto, (C₁-C₁₂)-alkylsulfinyl, (C₁-C₁₂)-alkylsulfonyl, (C₆-C₁₆)-arylmercapto, (C₆-C₁₆)-arylsulfinyl, (C₆-C₁₆)-arylsulfonyl, (C₇-C₁₆)-aralkyl- mercapto, (C₇-C₁₆)-aralkylsulfinyl, (C₇-C₁₆)-aralkylsulfonyl,
sulfamoyl, N-(C₁-C₁₀)-alkylsulfamoyl, N,N-di-(C₁-C₁₀)-alkylsulfamoyl, (C₃-C₈)-cycloalkylsulfamoyl, N-(C₆-C₁₆)-arylsulfamoyl, N-(C₇-C₁₆)-aralkylsulfamoyl, N-(C₁-C₁₀)-alkyl-N-(C₆-C₁₆)-arylsulfamoyl, N-(C₁-C₁₀)-alkyl-N-(C₇-C₁₆)-aralkylsulfamoyl,
(C₁-C₁₀)-alkylsulfonamido, N-((C₁-C₁₀)-alkyl)-(C₁-C₁₀)- alkylsulfonamido, (C₇-C₁₆)-aralkylsulfonamido, N-((C₁-C₁₀)-alkyl-(C₇-C₁₆)-aralkylsulfonamido,
where the radicals which contain an aryl radical can for their part be substituted on the aryl by 1, 2, 3, 4 or 5 identical or different substituents from the series hydroxyl, halogen, cyano, trifluoromethyl, nitro, carboxyl, (C₁-C₁₂)-alkyl, (C₃-C₈)-cycloalkyl, (C₆-C₁₂)-aryl, (C₇-C₁₆)-aralkyl, (C₃-C₁₂)-alkenyl, (C₃-C₁₂)-alkynyl, (C₁-C₁₂)-alkoxy, (C₁-C₁₂)-alkoxy -(C₁-C₁₂)-alkyl, (C₁-C₁₂)-alkoxy-(C₁-C₁₂)-alkoxy, (C₆-C₁₂)-aryloxy, (C₇-C₁₆)-aralkyloxy, (C₁-C₈)-hydroxyalkyl, -O-[CH₂]ₓC_{f}H_{(2f + 1 - g)}F_{g}, -OCF₂Cl, -OCF₂-CHFCl,
(C₁-C₁₂)-alkylcarbonyl, (C₃-C₈)-cycloalkylcarbonyl, (C₆-C₁₂)-arylcarbonyl, (C₇-C₁₆)-aralkylcarbonyl, cinnamoyl, (C₃-C₁₂)-alkenylcarbonyl, (C₃-C₁₂)-alkynylcarbonyl,
(C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₃-C₁₂)-alkenyloxycarbonyl, (C₃-C₁₂)-alkynyloxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₃-C₁₂)-alkenylcarbonyloxy, (C₃-C₁₂)-alkynylcarbonyloxy,
(C₁-C₁₂)-alkoxycarbonyloxy, (C₁-C₁₂)-alkoxy-(C₁-C₁₂)-alkoxycarbonyloxy, (C₆-C₁₂)-aryloxycarbonyloxy, (C₇-C₁₆)-aralkyloxycarbonyloxy, (C₃-C₈)-cycloalkoxycarbonyloxy, (C₃-C₁₂)-alkenyloxycarbonyloxy, (C₃-C₁₂)-alkynyloxycarbonyloxy,
carbamoyl, N-(C₁-C₁₂)-alkylcarbamoyl, hydroxy-(C₁-C₄)-alkylcarbamoyl, acyloxy-(C₁-C₄)-alkylcarbamoyl, N,N-di-(C₁-C₁₂)-alkylcarbamoyl, N-(C₃-C₈)-cycloalkylcarbamoyl, N-(C₆-C₁₆)-aryl- carbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₀)-alkoxy-(C₁-C₁₀)-alkyl)carbamoyl, N-((C₆-C₁₆)-aryloxy-(C₁-C₁₀)-alkyl)carbamoyl, N-((C₇-C₁₆)-aralkyloxy-(C₁-C₁₀)-alkyl)carbamoyl, N-(C₁-C₁₀)-alkyl-N-((C₁-C₁₀)-alkoxy-(C₁-C₁₀)-alkyl)carbamoyl, N-(C₁-C₁₀)-alkyl-N-((C₆-C₁₆)-aryloxy-(C₁-C₁₀)-alkyl)carbamoyl, N-(C₁-C₁₀)-alkyl-N-((C₇-C₁₆)-aralkyloxy-(C₁-C₁₀)-alkyl)carbamoyl,
carbamoyloxy, N-(C₁-C₁₂)-alkylcarbamoyloxy, N,N-di-(C₁-C₁₂)-alkylcarbamoyloxy, N-(C₃-C₈)-cyclo-alkylcarbamoyloxy, N-(C₆-C₁₆)-aryl- carbamoyloxy, N-(C₇-C₁₆)-aralkylcarbamoyloxy, N-(C₁-C₁₀)-alkyl-N-(C₆-C₁₂)arylcarbamoyloxy, N-(C₁-C₁₀)-alkyl-N-(C₇-C₁₆)-aralkylcarbamoyloxy N-((C₁-C₁₀)-alkoxy-(C₁-C₁₀)-alkyl)carbamoyloxy, N-((C₆-C₁₆)-aryloxy-(C₁-C₁₀)-alkyl)carbamoyloxy, N-((C₇-C₁₆)-aralkyloxy-(C₁-C₁₀)-alkyl)carbamoyloxy, N-(C₁-C₁₀)-alkyl-N-((C₁-C₁₀)-alkoxy-(C₁-C₁₀)-alkyl)carbamoyloxy, N-(C₁-C₁₀)-alkyl-N-((C₆-C₁₆)-aryloxy-(C₁-C₁₀)-alkyl)carbamoyloxy, N-(C₁-C₁₀)-alkyl-N-((C₇-C₁₆)-aralkyloxy-(C₁-C₁₀)-alkyl)carbamoyloxy,
amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-(C₁-C₁₀)-alkyl-N-(C₇-C₁₆)-aralkylamino, N-(C₁-C₁₀)-alkyl-N-(C₆-C₁₂)-arylamino, (C₁-C₁₂)-alkoxyamino, (C₁-C₁₂)-alkoxy-N-(C₁-C₁₀)-alkylamino,
(C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino,
(C₁-C₁₂)-alkanoylamino-(C₁-C₈)-alkyl, (C₃-C₈)-cycloalkanoylamino-(C₁-C₈)-alkyl, (C₆-C₁₆)-aroylamino-(C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)alkyl, N,N-di-(C₁-C₁₀)-alkylamino-(C₁-C₁₀)alkyl, (C₃-C₈)-cycloalkylamino-(C₁-C₁₀)alkyl,
(C₁-C₁₂)-alkylmercapto,(C₁-C₁₂)-alkylsulfinyl, (C₁-C₁₂)-alkylsulfonyl, (C₆-C₁₆)-arylmercapto, (C₆-C₁₆)-arylsulfinyl, (C₆-C₁₆)-arylsulfonyl, (C₇-C₁₆)-aralkylmercapto, (C₇-C₁₆)-aralkylsulfinyl, (C₇-C₁₆)-aralkylsulfonyl,
sulfamoyl, N-(C₁-C₁₀)-alkylsulfamoyl, N,N-di-(C₁-C₁₀)-alkylsulfamoyl, (C₃-C₈)-cycloalkylsulfamoyl, N-(C₆-C₁₆)-arylsulfamoyl, N-(C₇-C₁₆)-aralkylsulfamoyl, N-(C₁-C₁₀)-alkyl-N-(C₆-C₁₆)-arylsulfamoyl, N-(C₁-C₁₀)-alkyl-N-(C₇-C₁₆)-aralkyl- sulfamoyl,
(C₁-C₁₀)-alkylsulfonamido, N-((C₁-C₁₀)-alkyl)-(C₁-C₁₀)-alkylsulfonamido, (C₇-C₁₆)-aralkylsulfonamido, N-((C₁-C₁₀)-alkyl)-(C₇-C₁₆)-aralkyl-sulfonamido,
R⁴ is hydrogen, (C₁-C₆)-alkyl, phenyl, phenyl-(C₁-C₄)-alkyl,
R⁵ is an unsubstitued or substituted branched or unbranched, aliphatic (C₁-C₈)-alkyl radical which carries a carboxyl group which is substituted one or more times, preferably once or twice, by
hydroxyl, flourine, chlorine, hydroxy-(C₁-C₄)-alkyl, (C₁-C₁₂)-alkyl, (C₃-C₈)-cycloalkyl, (C₆-C₁₂)-aryl, (C₇-C₁₆)-aralkyl, (C₁-C₆)-alkoxy, (C₆-C₁₂)-aryloxy, (C₇-C₁₆)-aralkyloxy, -O-[CH₂]ₓC_{f}H_{(2f + 1 - g)}F_{g},
where the radicals which contain an aryl radical can for their part be substituted on the aryl by 1, 2, 3, 4 or 5 identical or different substituents from the series hydroxyl, halogen, cyano, trifluoromethyl, nitro, carboxyl, (C₁-C₆)-alkyl, (C₃-C₈)-cycloalkyl, (C₁-C₆)-alkoxy,
and
n is 0 or 1,
f is 1 to 8, preferably 1 to 5,
g is 0, 1 to (2f+1) and
x is 0 to 8, preferably 0 to 1.

3. A compound as claimed in claim 1 or 2 according to the formula (I),
in which
R¹ is hydroxyl or (C₁-C₆)-alkoxy,
R² and R³ are hydrogen,
R⁶ is hydrogen, (C₁-C₆)-alkyl or an N-protective group such as (C₁-C₈)-alkanoyl, (C₁-C₆)-alkylcarbamoyl, (C₁-C₆)-alkoxycarbonyl, benzyloxycarbonyl, (C₁-C₁₀)-acyloxy-(C₁-C₆)-alkyl, preferably (C₁-C₁₀)-alkanoyloxy-(C₁-C₆)-alkyl, benzoyloxy-(C₁-C₆)-alkyl, benzyloxycarbonyl-oxy-(C₁-C₆)-alkyl or (C₁-C₆)-alkoxycarbonyloxy-(C₁-C₆)-alkyl, a 1-, 2-, 3- or 4-valent physiologically utilizable cation, in particular Na^{⊕}, K^{⊕}, Mg^{2⊕}, Ca^{2⊕}, Al^{3⊕} or an ammonium ion, optionally substituted 1-3 times by (C₁-C₈)-alkyl, (C₁-C₈)-hydroxyalkyl, (C₁-C₄)-alkoxy-(C₁-C₈)-alkyl, phenyl, benzyl or (C₁-C₈)-alkyl which can be substituted 1 to 3 times by hydroxyl or (C₁-C₄)-alkoxy, or a cation of a basic amino acid derivative,
R⁷ is a radical of the formula II, excluding -SO₂H,
-Y-[C-U]ᵣ-D-W (II)
in which
Y is -SO₂-,
C is a bond, or
a branched or unbranched aliphatic (C₁-C₁₆)-alkanediyl radical,
U is a bond or
hydrogen or -O-,
r is 1,
D is a bond or
hydrogen or
a branched or unbranched aliphatic (C₁-C₄)-alkanediyl radical,
W is a bond or
hydrogen or
a (C₃-C₁₀)-cycloaliphatic alkyl, alkenyl, alkynyl or alkenynyl radical or
a (C₆-C₁₆)-aryl radical or a 5- or 6-membered heteroaryl radical, where at least one of the variables C or D or W is not a bond and U is only a heteroatom group if C is not a bond or if D and/or W are not a bond and
C, D and/or W, if these are not a bond or hydrogen, for their part preferably are substituted by a combination of up to 5 identical or different substituents from the series
hydroxyl, halogen, cyano, trifluoromethyl, nitro, carboxyl, (C₁-C₁₂)-alkyl, (C₃-C₈)-cycloalkyl, (C₆-C₁₂)-aryl, (C₇-C₁₆)-aralkyl, (C₃-C₁₂)-alkenyl, (C₃-C₁₂)-alkynyl, (C₁-C₁₂)-alkoxy, (C₁-C₁₂)-alkoxy-(C₁-C₁₂)-alkyl, (C₁-C₁₂)-alkoxy-(C₁-C₁₂)-alkoxy, (C₆-C₁₂)-aryloxy, (C₇-C₁₆)-aralkyloxy, (C₁-C₈)-hydroxyalkyl, -O-[CH₂]ₓC_{f}H_{(2f+1-g})F_{g}, -OCF₂Cl, -OCF₂-CHFCl,
(C₁-C₁₂)-alkylcarbonyl, (C₃-C₈)-cycloalkylcarbonyl, (C₆-C₁₂)-arylcarbonyl, (C₇-C₁₆)-aralkylcarbonyl, cinnamoyl, (C₃-C₁₂)-alkenylcarbonyl, (C₃-C₁₂)-alkynylcarbonyl,
(C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₃-C₁₂)-alkenyloxycarbonyl, (C₃-C₁₂)-alkynyloxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₃-C₁₂)-alkenylcarbonyloxy, (C₃-C₁₂)-alkynyl-carbonyloxy,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N-(C₆-C₁₆)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₀)-alkoxy-(C₁-C₁₀)-alkyl)carbamoyl, N-((C₆-C₁₆)-aryloxy-(C₁-C₁₀)-alkyl)carbamoyl, N-((C₇-C₁₆)-aralkyloxy-(C₁-C₁₀)-alkyl)carbamoyl, N-(C₁-C₁₀)-alkyl-N-((C₁-C₁₀)-alkoxy-(C₁-C₁₀)-alkyl)carbamoyl, N-(C₁-C₁₀)-alkyl-N-((C₆-C₁₆)-aryloxy-(C₁-C₁₀)-alkyl)carbamoyl, N-(C₁-C₁₀)-alkyl-N-((C₇-C₁₆)-aralkyloxy-(C₁-C₁₀)-alkyl)carbamoyl,
amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-(C₁-C₅)-alkyl-N-(C₇-C₁₀)-aralkylamino, N-(C₁-C₅)-alkyl-N-(C₆-C₁₂)-arylamino, (C₁-C₁₂)-alkoxyamino, (C₁-C₁₂)-alkoxy-N-(C₁-C₁₀)-alkylamino,
(C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino,
(C₁-C₁₂)-alkanoylamino-(C₁-C₈)-alkyl, (C₃-C₈)-cycloalkanoylamino-(C₁-C₈)-alkyl, (C₆-C₁₆)-aroylamino-(C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di-(C₁-C₁₀)-alkylamino-(C₁-C₁₀)-alkyl, (C₃-C₈)-cycloalkylamino-(C₁-C₁₀)-alkyl,
(C₁-C₁₂)-alkylmercapto, (C₁-C₁₂)-alkylsulfinyl, (C₁-C₁₂)-alkylsulfonyl, (C₆-C₁₆)-arylmercapto, (C₆-C₁₆)-arylsulfinyl, (C₆-C₁₆)-arylsulfonyl, (C₇-C₁₆)-aralkyl-mercapto, (C₇-C₁₆)-aralkylsulfinyl, (C₇-C₁₆)-aralkylsulfonyl,
where the radicals which contain an aryl radical for their part can be substituted on the aryl by 1, 2, 3, 4 or 5 identical or different substituents from the series hydrogen, hydroxyl, halogen, cyano, trifluoromethyl, nitro, carboxyl, (C₁-C₁₂)-alkyl, (C₃-C₈)-cycloalkyl, (C₆-C₁₂)-aryl, (C₇-C₁₆)-aralkyl, (C₃-C₁₂)-alkenyl, (C₃-C₁₂)-alkynyl, (C₁-C₁₂)-alkoxy, (C₁-C₁₂)-alkoxy-(C₁-C₁₂)-alkyl, (C₁-C₁₂)-alkoxy-(C₁-C₁₂)-alkoxy, (C₆-C₁₂)-aryloxy, (C₇-C₁₆)-aralkyloxy, (C₁-C₈)-hydroxyalkyl, -O-[CH₂]ₓC_{f}H_{(2f+1-g)}F_{g}, -OCF₂Cl, -OCF₂-CHFCl,
(C₁-C₁₂)-alkylcarbonyl, (C₃-C₈)-cycloalkylcarbonyl, (C₆-C₁₂)-arylcarbonyl, (C₇-C₁₆)-aralkylcarbonyl, cinnamoyl, (C₃-C₁₂)-alkenylcarbonyl, (C₃-C₁₂)-alkynylcarbonyl,
(C₁-C₁₂)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₃-C₁₂)-alkenyloxycarbonyl, (C₃-C₁₂)-alkynyloxycarbonyl,
(C₁-C₁₂)-alkylcarbonyloxy, (C₃-C₈)-cycloalkylcarbonyloxy, (C₆-C₁₂)-arylcarbonyloxy, (C₇-C₁₆)-aralkylcarbonyloxy, cinnamoyloxy, (C₃-C₁₂)-alkenylcarbonyloxy, (C₃-C₁₂)-alkynylcarbonyloxy,
carbamoyl, N-(C₁-C₁₂)-alkylcarbamoyl, hydroxy-(C₁-C₄)-alkylcarbamoyl, acyloxy-(C₁-C₄)-alkylcarbamoyl, N,N-di-(C₁-C₁₂)-alkylcarbamoyl, N-(C₃-C₈)-cycloalkylcarbamoyl, N-(C₆-C₁₆)-arylcarbamoyl, N-(C₇-C₁₆)-aralkylcarbamoyl, N-(C₁-C₁₀)-alkyl-N-(C₆-C₁₆)-arylcarbamoyl, N-(C₁-C₁₀)-alkyl-N-(C₇-C₁₆)-aralkylcarbamoyl, N-((C₁-C₁₀)-alkoxy-(C₁-C₁₀)-alkyl)carbamoyl, N-((C₆-C₁₆)-aryloxy-(C₁-C₁₀)-alkyl)carbamoyl, N-((C₇-C₁₆)-aralkyloxy-(C₁-C₁₀)-alkyl)carbamoyl, N-(C₁-C₁₀)-alkyl-N-((C₁-C₁₀)-alkoxy-(C₁-C₁₀)-alkyl)carbamoyl, N-(C₁-C₁₀)-alkyl-N-((C₆-C₁₆)-aryloxy-(C₁-C₁₀)-alkyl )carbamoyl, N-(C₁-C₁₀)-alkyl-N-((C₇-C₁₆)-aralkyloxy-(C₁-C₁₀)-alkyl)carbamoyl,
amino, (C₁-C₁₂)-alkylamino, di-(C₁-C₁₂)-alkylamino, (C₃-C₈)-cycloalkylamino, (C₃-C₁₂)-alkenylamino, (C₃-C₁₂)-alkynylamino, N-(C₆-C₁₂)-arylamino, N-(C₇-C₁₁)-aralkylamino, N-(C₁-C₅)-alkyl-(C₇-C₁₀)-aralkylamino, N-(C₁-C₅)-alkyl-(C₆-C₁₂)-arylamino, (C₁-C₁₂)-alkoxyamino, (C₁-C₁₂)-alkoxy-N-(C₁-C₁₀)-alkylamino,
(C₁-C₁₂)-alkanoylamino, (C₃-C₈)-cycloalkanoylamino, (C₆-C₁₂)-aroylamino, (C₇-C₁₆)-aralkanoylamino, (C₁-C₁₂)-alkanoyl-N-(C₁-C₁₀)-alkylamino, (C₃-C₈)-cycloalkanoyl-N-(C₁-C₁₀)-alkylamino, (C₆-C₁₂)-aroyl-N-(C₁-C₁₀)-alkylamino, (C₇-C₁₁)-aralkanoyl-N-(C₁-C₁₀)-alkylamino,
(C₁-C₁₂)-alkanoylamino-(C₁-C₈)-alkyl, (C₃-C₈)-cycloalkanoylamino-(C₁-C₈)-alkyl, (C₆-C₁₆)-aroylamino-(C₁-C₈)-alkyl, (C₇-C₁₆)-aralkanoylamino-(C₁-C₈)-alkyl, amino-(C₁-C₁₀)-alkyl, N-(C₁-C₁₀)-alkylamino-(C₁-C₁₀)-alkyl, N,N-di-(C₁-C₁₀)-alkylamino-(C₁-C₁₀)-alkyl, (C₃-C₈)-cycloalkylamino-(C₁-C₁₀)-alkyl,
(C₁-C₁₂)-alkylmercapto, (C₁-C₁₂)-alkylsulfinyl, (C₁-C₁₂)-alkylsulfonyl, (C₆-C₁₆)-arylmercapto, (C₆-C₁₆)-arylsulfinyl, (C₆-C₁₆)-arylsulfonyl, (C₇-C₁₆)-aralkyl-mercapto, (C₇-C₁₆)-aralkylsulfinyl, (C₇-C₁₆)-aralkylsulfonyl,
R⁴ is hydrogen or (C₁-C₄)-alkyl,
R⁵ is a branched or unbranched (C₁-C₄)-alkyl radical which carries a carboxyl group and which is substituted one or two times by
hydroxyl, fluorine, chlorine, (C₁-C₆)-alkyl, (C₃-C₈)-cycloalkyl, phenyl or benzyl, and
n is 0 or 1,
f is 1 to 5,
g is 0, 1 to (2f+1) and
x is 0 or 1.

4. A compound as claimed in one or more of claims 1 to 3 according to the formula I, in which
R¹ is hydroxyl or (C₁-C₆)-alkoxy,
R² and R³ are hydrogen,
R⁶ is hydrogen or a 1- or 2-valent physiologically utilizable cation, in particular Na^{⊕}, K^{⊕}, Mg^{2⊕} or Ca^{2⊕} or an ammonium ion,
R⁷ is a radical of the formula II, excluding -SO₂H,
-Y-[C-U]ᵣ-D-W (II),
in which
Y is -SO₂-,
C is a bond or
a (C₁-C₆)-alkanediyl radical,
U is a bond,
r is 1,
D is a bond or
hydrogen,
W is hydrogen or a phenyl radical, where at least one of the variables C or D or W is not a bond and
C, D and/or W can be substituted by fluorine, chlorine, trifluoromethyl, (C₁-C₁₆)-alkyl, (C₃-C₈)-cycloalkyl, benzyl, phenyl, (C₁-C₆)-alkoxy, phenoxy or -O-[CH₂]ₓC_{f}H_{(2f+1g})F_{g},
carbamoyl, N-(C₁-C₁₀)-alkylcarbamoyl, N,N-di-(C₁-C₈)-alkylcarbamoyl, N-(C₃-C₈)-cycloalkylcarbamoyl, N-phenylcarbamoyl, N-(C₇-C₁₁)-phenylalkylcarbamoyl, N-(C₁-C₈)-alkyl-N-(C₆-C₁₆)-phenylcarbamoyl,N-(C₁-C₈)-alkyl-N-(C₇-C₁₁)-phenylalkylcarbamoyl, N-((C₁-C₁₀)-alkoxy-(C₁-C₁₀)-alkylcarbamoyl, N-phenoxy-(C₁-C₈)-alkylcarbamoyl, N-((C₇-C₁₆)-phenylalkyloxy-(C₁-C₈)-alkylcarbamoyl, N-(C₁-C₈)-alkyl-N-((C₁-C₆)-alkoxy-(C₁-C₈)-alkyl)carbamoyl, N-(C₁-C₈)-alkyl-N-phenoxy-(C₁-C₈)-alkylcarbamoyl, N-(C₁-C₈)-alkyl-N-((C₇-C₁₆)-aralkyloxy-(C₁-C₁₀)-alkylcarbamoyl,
(C₁-C₁₀)-alkanoylamino, (C₃-C₈)-cycloalkanoylamino, phenylamino, (C₇-C₁₁)-phenylalkanoylamino, (C₁-C₈)-alkanoyl-N-(C₁-C₁₀)-alkylamino, (C₃-C₈)-cycloalkanoyl-N-(C₁-C₆)-alkylamino, benzoyl-N-(C₁-C₆)-alkylamino, (C₇-C₁₁)-phenyl-alkanoyl-N-(C₁-C₁₀)-alkylamino,
(C₁-C₁₀)-alkanoylamino-(C₁-C₄)-alkyl, (C₃-C₈)-cycloalkanoylamino-(C₁-C₄)-alkyl, phenoylamino-(C₁-C₆)-alkyl, (C₇-C₁₁)-phenylalkanoylamino-(C₁-C₆)-alkyl,
where the radicals which contain an aryl radical can for their part be substituted on the aryl by 1, 2, 3, 4 or 5 identical or different substituents from the series hydroxyl, fluorine, chlorine, trifluoromethyl, carboxyl, (C₁-C₄)-alkoxy, benzyloxy, phenoxy,
(C₁-C₁₂)-alkoxycarbonyl, (C₃-C₈)cycloalkylcarbonyl, phenoxycarbonyl, (C₇-C₁₁)-phenylalkoxycarbonyl,
(C₁-C₁₂)-alkylcarbonyloxy, (C₃-C₈)-cycloalkylcarbonyloxy, benzyloxy, (C₇-C₁₁)-phenylalkylcarbonyloxy,
carbamoyl, N-(C₁-C₆)-alkylcarbamoyl, N,N-di-(C₁-C₆)-alkylcarbamoyl, N-(C₃-C₈)-cycloalkylcarbamoyl, N-phenylcarbamoyl, N-(C₇-C₁₁)-phenylalkylcarbamoyl, hydroxy-(C₁-C₄)-alkylcarbamoyl, acyloxy-(C₁-C₄)-alkylcarbamoyl,
carbamoyloxy, N-(C₁-C₆)-alkylcarbamoyloxy, N,N-di-(C₁-C₆)-alkylcarbamoyloxy, N-(C₃-C₈)-cyclo-alkylcarbamoyloxy,
R⁴ is hydrogen,
R⁵ is a methyl group which is substituted by carboxyl and has a further substituent from the series
hydroxyl, hydroxy-(C₁-C₄)-alkyl, phenyl, benzyl, (C₁-C₄)-alkoxy, (C₁-C₅)-alkyl, benzyloxy or phenoxy and
n is 0 or 1,
f is 1 to 5,
g is 0, 1 to (2f+1) and
x is 0 or 1.

5. A compound as claimed in one or more of claims 1 to 4 of the formula I, in which
R¹ is hydroxyl or (C₁-C₆)-alkoxy,
R² and R³ are hydrogen,
R⁶ is hydrogen or a 1- or 2-valent physiologically utilizable cation, in particular Na^{⊕}, K^{⊕}, Mg^{2⊕} or Ca^{2⊕} or an ammonium ion, in particular H₃N^{⊕} C (CH₂OH)₃,
R⁷ is a radical of the formula II, excluding -SO₂H,
-Y-[C-U]ᵣ-D-W (II),
in which
Y is -SO₂-,
C is a bond or
(C₁-C₁₆)-alkanediyl,
U is a bond,
r is 1,
D is a bond or hydrogen,
W is a phenyl radical, where W can be substituted once or twice by fluorine, chlorine, (C₁-C₆)-alkyl, (C₇-C₁₆)-aralkyl, phenyl or (C₁-C₆)-alkoxy, and W is additionally substituted once by phenyl, phenoxy, -O-[CH₂]ₓC_{f}H_{(2f+1-g)}F_{g},
carbamoyl, N-(C₁-C₁₀)-alkylcarbamoyl, N,N-di-(C₁-C₈)-alkylcarbamoyl, N-(C₃-C₈)-cycloalkylcarbamoyl, N-phenylcarbamoyl, N-(C₇-C₁₁)-phenylalkylcarbamoyl, N-((C₁-C₄)-alkoxy-(C₁-C₄)-alkyl)-carbamoyl, N-phenoxy-(C₁-C₄)-alkylcarbamoyl,
(C₁-C₁₀)-alkanoylamino-(C₁-C₂)-alkyl, (C₃-C₈)-cycloalkanoylamino-(C₁-C₂)-alkyl, benzoylamino-(C₁-C₂)-alkyl or (C₇-C₁₁)-phenylalkanoylamino-(C₁-C₂)-alkyl,
where the radicals which contain an aryl radical can for their part be substituted on the aryl by 1, 2, 3, 4 or 5 identical or different substituents from the series hydroxyl, carboxyl, (C₁-C₄)-alkoxy, phenoxy, benzyloxy, fluorine, chlorine, trifluoromethyl,
carbamoyl, N-(C₁-C₆)-alkylcarbamoyl, N,N-di-(C₁-C₆)-alkylcarbamoyl, N-(C₃-C₈)-cycloalkylcarbamoyl, N-phenylcarbamoyl, N-(C₇-C₁₁)-phenalkylcarbamoyl,
R⁴ is hydrogen,
R⁵ is a methyl group which is substituted by carboxyl, and
n is 0,
f is 1 to 5,
g is 0, 1 to (2f+1) and
x is 0 or 1.

6. A process for the preparation of compounds as claimed in one or more of claims 1 to 5, in which according to formula I R¹ to R⁷ have the meaning as claimed in claims 1 to 5, which comprises reacting a pyridine-2-carboxylic acid ester carboxylate of the formula 7 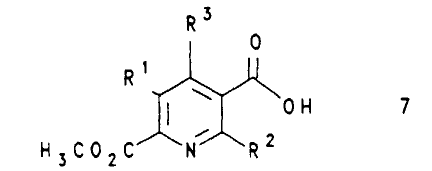 with a sulfonamide derivative of the formula 8
R⁶HN-R⁷ 8
and reacting the compound resulting therefrom with an amine of the formula 5
HNR⁴R⁵ 5
to give the compound of the formula Ia' 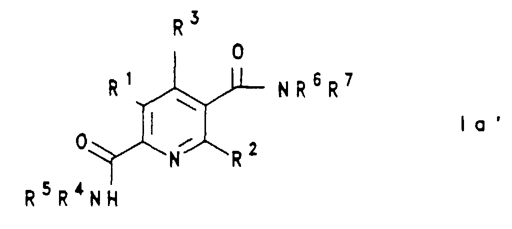 an oxidation optionally following to give the pyridine-N-oxide according to formula Ib'.

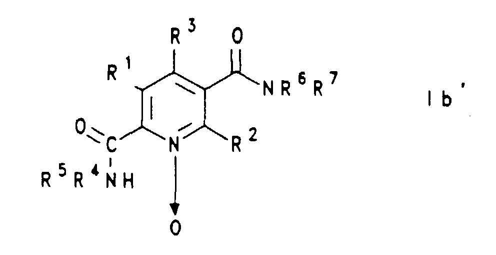

7. A compound as claimed in claims 1 to 5 for use against fibrotic disorders.

8. A compound as claimed in claims 1 to 5 for use as a fibrosuppressant.

9. A compound as claimed in claims 1 to 5 for the inhibition of proline hydroxylase.

10. A compound as claimed in claims 1 to 5 for use as an inhibitor of collagen biosynthesis.

11. A pharmaceutical containing at least one compound as claimed in claim 1 and optionally a pharmaceutically tolerable vehicle.

12. A compound as claimed in claim 1 for use in the treatment of disorders of the metabolism of collagen and collagen-like substances.

13. A compound as claimed in claim 1 for use in the treatment of fibrotic disorders.

14. A process for the production of pharmaceuticals for the treatment of fibrotic disorders, wherein the pharmaceutical contains a compound as claimed in claim 1.

## Revendications

1. Sulfonamidocarbonylpyridine-2-carboxamides ainsi que leurs N-oxydes de pyridine de formule générale I 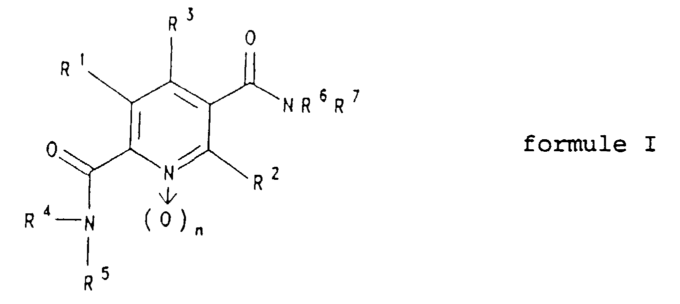 dans laquelle
R¹ représente un groupe hydroxy ou alcoxy en C₁-C₆,
R² et R³ sont identiques ou différents et représentent un atome d'hydrogène, un groupe alkyle en C₁-C₆ substitué ou non substitué, un groupe alcoxy en C₁-C₆, un atome d'halogène, en particulier de fluor, chlore ou brome, le groupe nitrile, hydroxy, amino,
R⁶ représente un atome d'hydrogène ou un groupe alkyle en C₁-C₆ ou un groupe protecteur d'azote tel qu'un groupe alcanoyle en C₁-C₈, alkyl(C₁-C₆)-carbamoyle, alcoxy(C₁-C₆)-carbonyle, benzyloxycarbonyle, acyloxy(C₁-C₁₀)-alkyle(C₁-C₆), de préférence un groupe alcanoyloxy-(C₁-C₁₀)-alkyle(C₁-C₆), benzoyloxy-alkyle(C₁-C₆), benzyloxycarbonyloxy-alkyle(C₁-C₆) ou alcoxy(C₁-C₆)-carbonyloxy-alkyle(C₁-C₆), un cation mono-, di- tri- ou tétravalent physiologiquement utilisable, en particulier Na^{⊕}, K^{⊕}, Mg^{2⊕}, Ca^{2⊕}, Al^{3⊕} ou un ion ammonium, éventuellement 1 à 3 fois substitué par un ou des radicaux alkyle en C₁-C₈, hydroxyalkyle en C₁-C₈, alcoxy(C₁-C₄)-alkyle(C₁-C₈), phényle, benzyle, ou un groupe alkyle en C₁-C₈ qui peut être 1 à 3 fois substitué par un ou des radicaux hydroxy ou alcoxy en C₁-C₄, ou un cation d'un dérivé d'aminoacide basique,
R⁷ représente un radical de formule II, à l'exception de -SO₂H,
-Y-[C-U]ᵣ-D-W (II)
dans laquelle
Y représente -SO₂- ou -CO-,
C représente une liaison ou
un radical alcanediyle en C₁-C₁₆ aliphatique, ramifié ou non ramifié, ou un radical alcanediyle en C₃-C₁₀ cycloaliphatique, ou un radical alcènediyle en C₂-C₁₆ ramifié ou non ramifié ou cycloalcènediyle, ou un radical alcynediyle en C₂-C₁₆ ou un radical alcénynediyle en C₂-C₁₆, pouvant comporter chacun une ou plusieurs liaisons multiples C-C,
U représente une liaison ou
un atome d'hydrogène ou
un radical choisi parmi les groupements d'hétéroatomes suivants: -CO-, -O(CO)-, -(CO)-O-, -(CO)NR-, -NR(CO)-, -O-, -SO-, -SO₂-, -NR, R représentant un groupe alkyle en C₁-C₃, alcanoyle en C₁-C₈, aralcanoyle en C₇-C₁₆, aroyle en C₆-C₁₂, ou un atome d'hydrogène,
r est 1, 2, 3 ou 4,
D représente une liaison ou un atome d'hydrogène ou un radical alcanediyle en C₁-C₁₀ aliphatique, ramifié ou non ramifié, ou
un radical alcènediyle en C₁-C₁₀ ramifié ou non ramifié,
un radical alcynediyle en C₂-C₁₀ ou un radical alcénynediyle en C₂-C₁₀, qui peuvent comporter chacun une ou plusieurs liaisons multiples C-C,
W représente une liaison ou
un atome d'hydrogène ou
un radical alkyle, alcényle, alcynyle ou alcénynyle cycloaliphatique en C₃-C₁₀, ou
un radical aryle en C₆-C₁₆ ou un radical hétéroaryle à 5-6 chaînons, au moins l'une des variables C ou D ou W ne représentant pas une liaison et U ne représentant alors un groupement d'hétéroatomes que lorsque C ne représente pas une liaison, ou lorsque D et/ou W ne représentent pas une liaison, et
C et/ou W, lorsqu'ils ne représentent pas une liaison ni un atome d'hydrogène, sont pour leur part de préférence substitués,
R⁴ représente un atome d'hydrogène ou un groupe alkyle en C₁-C₆, phényle, phénylalkyle(C₁-C₄),
R⁵ représente un radical alkyle aliphatique en C₁-C₈ substitué ou non substitué, ramifié ou non ramifié, qui porte un groupe acide, et peut porter en outre un ou plusieurs substituants, et
n représente 0 ou 1.

2. Composés selon la revendication 1, de formule générale 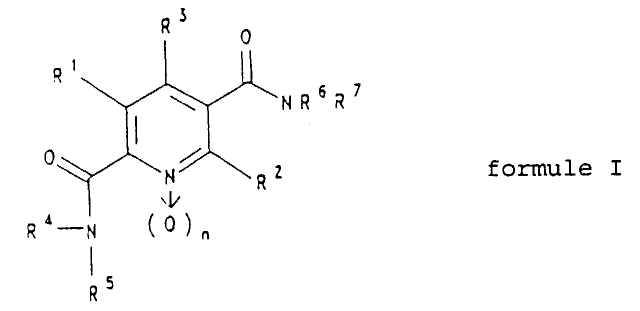 dans laquelle
R¹ représente un groupe hydroxy ou alcoxy en C₁-C₆,
R² et R³ sont identiques ou différents et représentent un atome d'hydrogène, un groupe alkyle en C₁-C₆ substitué ou non substitué, un groupe alcoxy en C₁-C₆, un atome d'halogène, en particulier de fluor, chlore ou brome, le groupe nitrile, hydroxy, amino,
R⁶ représente un atome d'hydrogène ou un groupe alkyle en C₁-C₆ ou un groupe protecteur d'azote tel qu'un groupe alcanoyle en C₁-C₈, alkyl(C₁-C₆)-carbamoyle, alcoxy(C₁-C₆)-carbonyle, benzyloxycarbonyle, acyloxy(C₁-C₁₀)-alkyle(C₁-C₆), de préférence un groupe alcanoyloxy(C₁-C₁₀)-alkyle(C₁-C₆), benzoyloxy-alkyle(C₁-C₆), benzyloxycarbonyloxy-alkyle(C₁-C₆) ou alcoxy(C₁-C₆)-carbonyloxy-alkyle(C₁-C₆), un cation mono-, di- tri- ou tétravalent physiologiquement utilisable, en particulier Na^{⊕}, K^{⊕}, Mg^{2⊕} , Ca^{2⊕}, Al^{3⊕} ou un ion ammonium, éventuellement 1 à 3 fois substitué par un ou des radicaux alkyle en C₁-C₈, hydroxyalkyle en C₁-C₈, alcoxy(C₁-C₄)-alkyle(C₁-C₈), phényle, benzyle, ou un groupe alkyle en C₁-C₈ qui peut être 1 à 3 fois substitué par un ou des radicaux hydroxy ou alcoxy en C₁-C₄, ou un cation d'un dérivé d'aminoacide basique,
R⁷ représente un radical de formule II, à l'exception de -SO₂H,
-Y-[C-U]ᵣ-D-W (II)
dans laquelle
Y représente -SO₂- ou -CO-,
C représente une liaison ou
un radical alcanediyle en C₁-C₁₆ aliphatique, ramifié ou non ramifié, ou un radical alcanediyle en C₃-C₁₀ cycloaliphatique, ou un radical alcènediyle en C₂-C₁₆ ramifié ou non ramifié ou cycloalcènediyle, ou un radical alcynediyle en C₂-C₁₆ ou un radical alcénynediyle en C₂-C₁₆, pouvant comporter chacun une ou plusieurs liaisons multiples C-C,
U représente une liaison ou
un atome d'hydrogène ou
un radical choisi parmi les groupements d'hétéroatomes suivants -CO-, -O(CO)-, -(CO)-O-, -(CO)NR-, -NR(CO)-, -O-, -SO-, -SO₂-, -NR, R représentant un groupe alkyle en C₁-C₃, alcanoyle en C₁-C₈, aralcanoyle en C₇-C₁₆, aroyle en C₆-C₁₂, ou un atome d'hydrogène,
r est 1, 2, 3 ou 4,
D représente une liaison ou un atome d'hydrogène ou un radical alcanediyle en C₁-C₁₀ aliphatique, ramifié ou non ramifié, ou
un radical alcènediyle en C₁-C₁₀ ramifié ou non ramifié,
un radical alcynediyle en C₂-C₁₀ ou un radical alcénynediyle en C₂-C₁₀, qui peuvent comporter chacun une ou plusieurs liaisons multiples C-C,
W représente une liaison ou
un atome d'hydrogène ou
un radical alkyle, alcényle, alcynyle ou alcénynyle cycloaliphatique en C₃-C₁₀, ou
un radical aryle en C₆-C₁₆ ou un radical hétéroaryle à 5-6 chaînons, au moins l'une des variables C ou D ou W ne représentant pas une liaison et U ne représentant alors un groupement d'hétéroatomes que lorsque C ne représente pas une liaison, ou lorsque D et/ou W ne représentent pas une liaison, et
C et/ou W, lorsqu'ils ne représentent pas une liaison ou un atome d'hydrogène, sont pour leur part de préférence substitués par un ensemble de jusqu'à 5 substituants identiques ou différents, choisis parmi des atomes d'halogène et des groupes hydroxy, cyano, trifluorométhyle, nitro, carboxy, alkyle en C₁-C₁₂, cycloalkyle en C₃-C₈, aryle en C₆-C₁₂, aralkyle en C₇-C₁₆, alcényle en C₃-C₁₂, alcynyle en C₃-C₁₂, alcoxy en C₁-C₁₂, alcoxy(C₁-C₁₂)-alkyle(C₁-C₁₂), alcoxy(C₁-C₁₂)-alcoxy(C₁-C₁₂), aryloxy en C₆-C₁₂, aralkyloxy en C₇-C₁₆, hydroxyalkyle en C₁-C₈, -O-[CH₂]ₓC_{f}H_{(2f+1-g)}F_{g}, -OCF₂Cl, -OCF₂-CHFCl,
alkyl(C₁-C₁₂)-carbonyle, cycloalkyl(C₃-C₈)-carbonyle, aryl(C₆-C₁₂)-carbonyle, aralkyl(C₇-C₁₆)-carbonyle, cinnamoyle, alcényl(C₃-C₁₂)-carbonyle, alcynyl(C₃-C₁₂)-carbonyle,
alcoxy(C₁-C₁₂)-carbonyle, alcoxy(C₁-C₁₂)-alcoxy(C₁-C₁₂)-carbonyle, aryloxy(C₆-C₁₂)-carbonyle, aralcoxy(C₇-C₁₆)-carbonyle, cycloalcoxy(C₃-C₈)-carbonyle, alcényloxy(C₃-C₁₂)-carbonyle, alcynyloxy(C₃-C₁₂)-carbonyle,
alkyl(C₁-C₁₂)-carbonyloxy, cycloalkyl(C₃-C₈)-carbonyloxy, aryl(C₆-C₁₂)-carbonyloxy, aralkyl(C₇-C₁₆)-carbonyloxy, cinnamoyloxy, alcényl(C₃-C₁₂)-carbonyloxy, alcynyl(C₃-C₁₂)-carbonyloxy,
alcoxy(C₁-C₁₂)-carbonyloxy, alcoxy(C₁-C₁₂)-alcoxy(C₁-C₁₂)-carbonyloxy, aryloxy(C₆-C₁₂)-carbonyloxy, aralkyloxy(C₇-C₁₆)-carbonyloxy, cycloalcoxy(C₃-C₈)-carbonyloxy, alcényloxy(C₃-C₁₂)-carbonyloxy, alcynyloxy(C₃-C₁₂)-carbonyloxy,
carbamoyle, N-alkyl(C₁-C₁₂)-carbamoyle, N,N-dialkyl(C₁-C₁₂)-carbamoyle, N-cycloalkyl(C₃-C₈)-carbamoyle, N-aryl(C₆-C₁₆)-carbamoyle, N-aralkyl(C₇-C₁₆)-carbamoyle, N-alkyl(C₁-C₁₀)-N-aryl(C₆-C₁₆)-carbamoyle, N-alkyl(C₁-C₁₀)-N-aralkyl(C₇-C₁₆)-carbamoyle, N-[alcoxy(C₁-C₁₀)-alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, N-[alkyl(C₁-C₁₀)-N-[alcoxy(C₁-C₁₀)-alkyl(C₁-C₁₀)]carbamoyle, N-[alkyl(C₁-C₁₀)-N-[aryloxy(C₆-C₁₆)-alkyl(C₁-C₁₀)]carbamoyle, N-[alkyl(C₁-C₁₀)-N-[aralkyloxy(C₇-C₁₆)-alkyl(C₁-C₁₀)]carbamoyle,
carbamoyloxy, N-alkyl(C₁-C₁₂)-carbamoyloxy, N,N-dialkyl(C₁-C₁₂)-carbamoyloxy, N-cycloalkyl(C₃-C₈)carbamoyloxy, N-aryl(C₆-C₁₆)-carbamoyloxy, N-aralkyl(C₇-C₁₆)-carbamoyloxy, N-alkyl(C₁-C₁₀)-N-aryl(C₆-C₁₂)-carbamoyloxy, N-alkyl(C₁-C₁₀)-N-aralkyl(C₇-C₁₆)-carbamoyloxy, N-[alcoxy(C₁-C₁₀)-alkyl(C₁-C₁₀)]carbamoyloxy,
N-[aryloxy(C₆-C₁₆)-alkyl(C₁-C₁₀)]carbamoyloxy, N-[aralkyloxy(C₇-C₁₆)-alkyl(C₁-C₁₀)]carbamoyioxy, N-[alkyl(C₁-C₁₀)-N-[alcoxy(C₁-C₁₀)]-alkyl(C₁-C₁₀)]carbamoyloxy, N-[alkyl(C₁-C₁₀)-N-[aryloxy(C₆-C₁₆)]-alkyl(C₁-C₁₀)]carbamoyloxy, N-[alkyl(C₁-C₁₀)-N-[aralkyloxy(C₇-C₁₆)]-alkyl(C₁-C₁₀)]-carbamoyloxy,
amino, alkyl(C₁-C₁₂)amino, dialkyl(C₁-C₁₂)amino, cycloalkyl(C₃-C₈)amino, alcényl(C₃-C₁₂)amino, alcynyl(C₃-C₁₂)amino, N-aryl(C₆-C₁₂)amino, N-aralkyl(C₇-C₁₁)amino, N-alkyl(C₁-C₁₀)-aralkyl(C₇-C₁₀)amino, N-alkyl(C₁-C₁₀)-N-aryl(C₆-C₁₂)amino, alcoxy(C₁-C₁₂)amino, alcoxy(C₁-C₁₂)-alkyl(C₁-C₁₀)amino,
alcanoyl(C₁-C₁₂)amino, cycloalcanoyl(C₃-C₈)amino, aroyl(C₆-C₁₂)amino, aralcanoyl(C₇-C₁₆)amino, alcanoyl(C₁-C₁₂)-N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino, aralcanoyl(C₇-C₁₁)-N-alkyl(C₁-C₁₀)amino,
alcanoyl(C₁-C₁₂)amino-alkyle(C₁-C₈), cycloalcanoyl(C₃-C₈)amino-alkyle(C₁-C₈), aroyl(C₆-C₁₆)amino-alkyle(C₁-C₈), aralcanoyl(C₇-C₁₆)amino-alkyle(C₁-C₈), amino-alkyle en C₁-C₁₀, N-alkyl(C₁-C₁₀)amino-alkyle(C₁-C₁₀), N,N-dialkyl(C₁-C₁₀)amino-alkyle(C₁-C₁₀), cycloalkyl(C₃-C₈)amino-alkyle(C₁-C₁₀),
alkyl(C₁-C₁₂)mercapto, alkyl(C₁-C₁₂)sulfinyle, alkyl(C₁-C₁₂)sulfonyle, aryl(C₆-C₁₆)mercapto, aryl(C₆-C₁₆)sulfinyle, aryl(C₆-C₁₆)sulfonyle, aralkyl(C₇-C₁₆)mercapto, aralkyl(C₇-C₁₆)sulfinyle, aralkyl(C₇-C₁₆)sulfonyle,
sulfamoyle, N-alkyl(C₁-C₁₀)sulfamoyle, N,N-dialkyl(C₁-C₁₀)sulfamoyle, cycloalkyl(C₃-C₈)sulfamoyle, N-aryl(C₆-C₁₆)sulfamoyle, N-aralkyl(C₇-C₁₆)sulfamoyle, N-alkyl(C₁-C₁₀)-N-aryl(C₆-C₁₆)sulfamoyie, N-alkyl(C₁-C₁₀)-N-aralkyl(C₇-C₁₆)sulfamoyle,
alkyl(C₁-C₁₀)sulfonamido, N-[alkyl(C₁-C₁₀)]-alkyl(C₁-C₁₀)sulfonamido,
aralkyl(C₇-C₁₆)sulfonamido, N-[alkyl(C₁-C₁₀)]-aralkyl(C₇-C₁₆)sulfonamido,
les radicaux qui contiennent un fragment aryle pouvant pour leur part être substitués sur le fragment aryle par 1, 2, 3, 4 ou 5 substituants identiques ou différents choisis parmi des atomes d'halogène et des groupes hydroxy, cyano, trifluorométhyle, nitro, carboxy, alkyle en C₁-C₁₂, cycloalkyle en C₃-C₈, aryle en C₆-C₁₂, aralkyle en C₇-C₁₆, alcényle en C₃-C₁₂, alcynyle en C₃-C₁₂, alcoxy en C₁-C₁₂, alcoxy(C₁-C₁₂)alkyle(C₁-C₁₂), alcoxy(C₁-C₁₂)-alcoxy(C₁-C₁₂), aryloxy en C₆-C₁₂, aralkyloxy en C₇-C₁₆, hydroxyalkyle en C₁-C₈, -O-[CH₂]ₓC_{f}H_{(2f+1-g)}F_{g}, -OCF₂Cl, -OCF₂-CHFCl,
alkyl(C₁-C₁₂)-carbonyle, cycloalkyl(C₃-C₈)-carbonyle, aryl(C₆-C₁₂)-carbonyle, aralkyl(C₇-C₁₆)-carbonyle, cinnamoyle, alcényl(C₃-C₁₂)-carbonyle, alcynyl(C₃-C₁₂)-carbonyle,
alcoxy(C₁-C₁₂)-carbonyle, alcoxy(C₁-C₁₂)-alcoxy(C₁-C₁₂)-carbonyle, aryloxy(C₆-C₁₂)-carbonyle, aralcoxy(C₇-C₁₆)-carbonyle, cycloalcoxy(C₃-C₈)-carbonyle, alcényloxy(C₃-C₁₂)-carbonyle, alcynyloxy(C₃-C₁₂)-carbonyle,
alkyl(C₁-C₁₂)-carbonyloxy, cycloalkyl(C₃-C₈)-carbonyloxy, aryl(C₆-C₁₂)-carbonyloxy, aralkyl(C₇-C₁₆)-carbonyloxy, cinnamoyloxy, alcényl(C₃-C₁₂)-carbonyloxy, alcynyl(C₃-C₁₂)-carbonyloxy,
alcoxy(C₁-C₁₂)-carbonyloxy, alcoxy(C₁-C₁₂)-alcoxy(C₁-C₁₂)-carbonyloxy, aryloxy(C₆-C₁₂)-carbonyloxy, aralkyloxy(C₇-C₁₆)-carbonyloxy, cycloalcoxy(C₃-C₈)-carbonyloxy, alcényloxy(C₃-C₁₂)-carbonyloxy, alcynyloxy(C₃-C₁₂)-carbonyloxy,
carbamoyle, N-alkyl(C₁-C₁₂)-carbamoyle, hydroxyalkyl(C₁-C₄)carbamoyle, acyloxy-[alkyl(C₁-C₄)]carbamoyle, N,N-dialkyl(C₁-C₁₂)-carbamoyle, N-cycloalkyl(C₃-C₈)carbamoyle, N-aryl(C₆-C₁₆)-carbamoyle, N-aralkyl(C₇-C₁₆)carbamoyle, N-alkyl(C₁-C₁₀)-N-aryl(C₆-C₁₆)carbamoyle, N-alkyl(C₁-C₁₀)-N-aralkyl(C₇-C₁₆)carbamoyle, N-[alcoxy(C₁-C₁₀)-alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, N-[alkyl(C₁-C₁₀)]-N-[alcoxy(C₁-C₁₀)-alkyl(C₁-C₁₀)]carbamoyle, N-[alkyl(C₁-C₁₀)]-N-[aryloxy(C₆-C₁₆)-alkyl(C₁-C₁₀)]carbamoyle, N-[alkyl(C₁-C₁₀)-N-[aralkyloxy(C₇-C₁₆)-alkyl(C₁-C₁₀)]carbamoyle,
carbamoyloxy, N-alkyl(C₁-C₁₂)-carbamoyloxy, N,N-dialkyl(C₁-C₁₂)-carbamoyloxy, N-cycloalkyl(C₃-C₈)carbamoyloxy, N-aryl(C₆-C₁₆)-carbamoyloxy, N-aralkyl(C₇-C₁₆)-carbamoyloxy, N-alkyl(C₁-C₁₀)-N-aryl(C₆-C₁₂)-carbamoyloxy, N-alkyl(C₁-C₁₀)-N-aralkyl(C₇-C₁₆)carbamoyloxy, N-[alcoxy(C₁-C₁₀)-alkyl(C₁-C₁₀)]carbamoyloxy, N-[aryloxy(C₆-C₁₆)-alkyl(C₁-C₁₀)]carbamoyloxy, N-[aralkyloxy(C₇-C₁₆)-alkyl(C₁-C₁₀)]carbamoyloxy, N-[alkyl(C₁-C₁₀)]-N-[alcoxy(C₁-C₁₀)-alkyl(C₁-C₁₀)]carbamoyloxy, N-[alkyl(C₁-C₁₀)]-N-[aryloxy(C₆-C₁₆)-alkyl(C₁-C₁₀)]carbamoyloxy, N-[alkyl(C₁-C₁₀)]-N-[aralkyloxy(C₇-C₁₆)-alkyl(C₁-C₁₀)]-carbamoyloxy,
amino, alkyl(C₁-C₁₂)amino, dialkyl(C₁-C₁₂)amino, cycloalkyl(C₃-C₈)amino, alcényl(C₃-C₁₂)amino, alcynyl-(C₃-C₁₂)amino, N-aryl(C₆-C₁₂)amino, N-aralkyl(C₇-C₁₁)amino, N-alkyl(C₁-C₁₀)-N-aralkyl(C₇-C₁₆)amino, N-alkyl(C₁-C₁₀)-N-aryl(C₆-C₁₂)amino, alcoxy(C₁-C₁₂)amino, alcoxy(C₁-C₁₂)-N-alkyl(C₁-C₁₀)amino,
alcanoyl(C₁-C₁₂)amino, cycloalcanoyl(C₃-C₈)amino, aroyl(C₆-C₁₂)amino, aralcanoyl(C₇-C₁₆)amino, alcanoyl(C₁-C₁₂)-N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino, aralcanoyl(C₇-C₁₁)-N-alkyl(C₁-C₁₀)amino,
alcanoyl(C₁-C₁₂)amino-alkyle(C₁-C₈), cycloalcanoyl(C₃-C₈)amino-alkyle(C₁-C₈), aroyl(C₆-C₁₆)amino-alkyle(C₁-C₈), aralcanoyl(C₇-C₁₆)amino-alkyle(C₁-C₈), aminoalkyle en C₁-C₁₀, N-alkyl(C₁-C₁₀)amino-alkyle(C₁-C₁₀), N,N-dialkyl(C₁-C₁₀)amino-alkyle(C₁-C₁₀), cycloalkyl(C₃-C₈)amino-alkyle(C₁-C₁₀),
alkyl(C₁-C₁₂)mercapto, alkyl(C₁-C₁₂)sulfinyle, alkyl(C₁-C₁₂)sulfonyle, aryl(C₆-C₁₆)mercapto, aryl(C₆-C₁₆)sulfinyle, aryl(C₆-C₁₆)sulfonyle, aralkyl(C₇-C₁₆)mercapto, aralkyl(C₇-C₁₆)sulfinyle, aralkyl(C₇-C₁₆)sulfonyle,
sulfamoyle, N-alkyl(C₁-C₁₀)sulfamoyle, N,N-dialkyl(C₁-C₁₀)sulfamoyle, cycloalkyl(C₃-C₈)sulfamoyle, N-aryl(C₆-C₁₆)sulfamoyle, N-aralkyl(C₇-C₁₆)sulfamoyle, N-alkyl(C₁-C₁₀)-N-aryl(C₆-C₁₆)sulfamoyle, N-alkyl(C₁-C₁₀)-N-aralkyl(C₇-C₁₆)sulfamoyle,
alkyl(C₁-C₁₀)sulfonamido, N-[alkyl(C₁-C₁₀)]-alkyl(C₁-C₁₀)sulfonamido, aralkyl(C₇-C₁₆)sulfonamido, N-[alkyl(C₁-C₁₀)]-aralkyl(C₇-C₁₆)sulfonamido,
R⁴ est un atome d'hydrogène ou un groupe alkyle en C₁-C₆, phényle, phényl-alkyle(C₁-C₄),
R⁵ représente un radical alkyle aliphatique en C₁-C₈ substitué ou non substitué, ramifié ou non ramifié, qui porte un groupe carboxy, et qui est substitué une ou plusieurs fois, de préférence une ou deux fois, par un ou des atomes de fluor ou de chlore ou groupes hydroxy, hydroxyalkyle en C₁-C₄, alkyle en C₁-C₁₂, cycloalkyle en C₃-C₈, aryle en C₆-C₁₂, aralkyle en C₇-C₁₆, alcoxy en C₁-C₆, aryloxy en C₆-C₁₂, aralkyloxy en C₇-C₁₆, -O-[CH₂]ₓC_{f}H_{(2f+1-g)}F_{g},
les radicaux qui contiennent un fragment aryle pouvant pour leur part être substitués sur le fragment aryle par 1, 2, 3, 4 ou 5 substituants identiques ou différents, choisis parmi des atomes d'halogène et des groupes hydroxy, cyano, trifluorométhyle, nitro, carboxy, alkyle en C₁-C₆, cycloalkyle en C₃-C₈, alcoxy en C₁-C₆, et
n est 0 ou 1,
f va de 1 à 8, de préférence de 1 à 5,
g est 0 ou va de 1 à (2f + 1) et
x va de 0 à 8, et de préférence est 0 ou 1.

3. Composés conformes à la revendication 1 ou 2, selon la formule générale I, dans lesquels
R¹ représente un groupe hydroxy ou alcoxy en C₁-C₆,
R² et R³ représentent des atomes d'hydrogène,
R⁶ représente un atome d'hydrogène ou un groupe alkyle en C₁-C₆ ou un groupe protecteur d'azote tel qu'un groupe alcanoyle en C₁-C₈, alkyl(C₁-C₆)-carbamoyle, alcoxy(C₁-C₆)-carbonyle, benzyloxycarbonyle, acyloxy(C₁-C₁₀)alkyle(C₁-C₆), de préférence un groupe alcanoyloxy(C₁-C₁₀)-alkyle(C₁-C₆), benzoyloxy-alkyle(C₁-C₆), benzyloxycarbonyloxy-alkyle(C₁-C₆) ou alcoxy(C₁-C₆)-carbonyloxy-alkyle(C₁-C₆), un cation mono-, di- tri- ou tétravalent physiologiquement utilisable, en particulier Na^{⊕}, K^{⊕}, Mg^{2⊕} , Ca^{2⊕}, Al^{3⊕} ou un ion ammonium, éventuellement 1 à 3 fois substitué par un ou des radicaux alkyle en C₁-C₈, hydroxyalkyle en C₁-C₈, alcoxy(C₁-C₄)-alkyle(C₁-C₈), phényle, benzyle, ou un groupe alkyle en C₁-C₈ qui peut être 1 à 3 fois substitué par un ou des radicaux hydroxy ou alcoxy en C₁-C₄, ou un cation d'un dérivé d'aminoacide basique,
R⁷ représente un radical de formule II, à l'exception de -SO₂H,
-Y-[C-U]ᵣ-D-W (II)
dans laquelle
Y représente -SO₂-,
C représente une liaison ou
un radical alcanediyle en C₁-C₁₆ aliphatique, ramifié ou non ramifié,
U représente une liaison ou
un atome d'hydrogène ou -O-,
r est égal à 1,
D représente une liaison ou un atome d'hydrogène ou un radical alcanediyle en C₁-C₄ aliphatique, ramifié ou non ramifié,
W représente une liaison ou
un atome d'hydrogène ou
un radical alkyle, alcényle, alcynyle ou alcénynyle cycloaliphatique en C₃-C₁₀, ou
un radical aryle en C₆-C₁₆ ou un radical hétéroaryle à 5-6 chaînons, au moins l'une des variables C ou D ou W ne représentant pas une liaison et U ne représentant alors un groupement d'hétéroatomes que lorsque C ne représente pas une liaison, ou lorsque D et/ou W ne représentent pas une liaison, et
C, D et/ou W, lorsqu'ils ne représentent pas une liaison ou un atome d'hydrogène, sont pour leur part de préférence substitués par un ensemble de jusqu'à 5 substituants identiques ou différents, choisis parmi des atomes d'halogène et des groupes hydroxy, cyano, trifluorométhyle, nitro, carboxy, alkyle en C₁-C₁₂, cycloalkyle en C₃-C₈, aryle en C₆-C₁₂, aralkyle en C₇-C₁₆, alcényle en C₃-C₁₂, alcynyle en C₃-C₁₂, alcoxy en C₁-C₁₂, alcoxy(C₁-C₁₂)-alkyle(C₁-C₁₂), alcoxy(C₁-C₁₂)-alcoxy(C₁-C₁₂), aryloxy en C₆-C₁₂, aralkyloxy en C₇-C₁₆, hydroxyalkyle en C₁-C₈, -O-[CH₂]ₓC_{f}H_{(2f+1-g)}F_{g}, -OCF₂Cl, -OCF₂-CHFCl,
alkyl(C₁-C₁₂)-carbonyle, cycloalkyl(C₃-C₈)-carbonyle, aryl(C₆-C₁₂)-carbonyle, aralkyl(C₇-C₁₆)-carbonyle, cinnamoyle, alcényl(C₃-C₁₂)-carbonyle, alcynyl(C₃-C₁₂)-carbonyle,
alcoxy(C₁-C₁₂)-carbonyle, alcoxy(C₁-C₁₂)-alcoxy(C₁-C₁₂)-carbonyle, aryloxy(C₆-C₁₂)-carbonyle, aralcoxy(C₇-C₁₆)-carbonyle, cycloalcoxy(C₃-C₈)-carbonyle, alcényloxy(C₃-C₁₂)-carbonyle, alcynyloxy(C₃-C₁₂)-carbonyle,
alkyl(C₁-C₁₂)-carbonyloxy, cycloalkyl(C₃-C₈)-carbonyloxy, aryl(C₆-C₁₂)-carbonyloxy, aralkyl(C₇-C₁₆)-carbonyloxy, cinnamoyloxy, alcényl(C₃-C₁₂)-carbonyloxy, alcynyl(C₃-C₁₂)-carbonyloxy,
carbamoyle, N-alkyl(C₁-C₁₂)-carbamoyle, N,N-dialkyl(C₁-C₁₂)-carbamoyle, N-cycloalkyl(C₃-C₈)-carbamoyle, N-aryl(C₆-C₁₆)-carbamoyle, N-aralkyl(C₇-C₁₆)-carbamoyle, N-alkyl(C₁-C₁₀)-N-aryl(C₆-C₁₆)-carbamoyle, N-alkyl(C₁-C₁₀)-N-aralkyl(C₇-C₁₆)-carbamoyle, N-[alcoxy(C₁-C₁₀)-alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, N-[alkyl(C₁-C₁₀)-N-[alcoxy(C₁-C₁₀)-alkyl(C₁-C₁₀)]carbamoyle, N-[alkyl(C₁-C₁₀)]-N-[aryloxy(C₆-C₁₆)-alkyl(C₁-C₁₀)]carbamoyle, N-[alkyl(C₁-C₁₀)]-N-[aralkyloxy(C₇-C₁₆)-alkyl(C₁-C₁₀)]carbamoyle,
amino, alkyl(C₁-C₁₂)amino, dialkyl(C₁-C₁₂)amino, cycloalkyl(C₃-C₈)amino, alcényl(C₃-C₁₂)amino, alcynyl(C₃-C₁₂)amino, N-aryl(C₆-C₁₂)amino, N-aralkyl(C₇-C₁₁)amino, N-alkyl(C₁-C₅)-N-aralkyl(C₇-C₁₀)amino, N-alkyl(C₁-C₅)-N-aryl(C₆-C₁₂)amino, alcoxy(C₁-C₁₂)amino, alcoxy(C₁-C₁₂)-N-alkyl(C₁-C₁₀)amino,
alcanoyl(C₁-C₁₂)amino, cycloalcanoyl(C₃-C₈)amino, aroyl(C₆-C₁₂)amino, aralcanoyl(C₇-C₁₆)amino, alcanoyl(C₁-C₁₂)-N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino, aralcanoyl(C₇-C₁₁)-N-alkyl(C₁-C₁₀)amino,
alcanoyl(C₁-C₁₂)amino-alkyle(C₁-C₈), cycloalcanoyl(C₃-C₈)amino-alkyle(C₁-C₈), aroyl(C₆-C₁₆)amino-alkyle(C₁-C₈), aralcanoyl(C₇-C₁₆)amino-alkyle(C₁-C₈), amino-alkyle en C₁-C₁₀, N-alkyl(C₁-C₁₀)amino-alkyle(C₁-C₁₀), N,N-dialkyl(C₁-C₁₀)amino-alkyle(C₁-C₁₀), cycloalkyl(C₃-C₈)amino-alkyle(C₁-C₁₀),
alkyl(C₁-C₁₂)mercapto, alkyl(C₁-C₁₂)sulfinyle, alkyl(C₁-C₁₂)sulfonyle, aryl(C₆-C₁₆)mercapto, aryl(C₆-C₁₆)sulfinyle, aryl(C₆-C₁₆)sulfonyle, aralkyl(C₇-C₁₆)mercapto, aralkyl(C₇-C₁₆)sulfinyle, aralkyl(C₇-C₁₆)sulfonyle,
les radicaux qui contiennent un fragment aryle pouvant pour leur part être substitués sur le fragment aryle par 1, 2, 3, 4 ou 5 substituants identiques ou différents choisis parmi des atomes d'hydrogène et d'halogène et des groupes hydroxy, cyano, trifluorométhyle, nitro, carboxy, alkyle en C₁-C₁₂, cycloalkyle en C₃-C₈, aryle en C₆-C₁₂, aralkyle en C₇-C₁₆, alcényle en C₃-C₁₂, alcynyle en C₃-C₁₂, alcoxy en C₁-C₁₂, alcoxy(C₁-C₁₂)-alkyle(C₁-C₁₂), alcoxy(C₁-C₁₂)-alcoxy(C₁-C₁₂), aryloxy en C₆-C₁₂, aralkyloxy en C₇-C₁₆, hydroxyalkyle en C₁-C₈, -O-[CH₂]ₓC_{f}H_{(2f+1-g)}F_{g}, -OCF₂Cl, -OCF₂-CHFCl,
alkyl(C₁-C₁₂)-carbonyle, cycloalkyl(C₃-C₈)-carbonyle, aryl(C₆-C₁₂)-carbonyle, aralkyl(C₇-C₁₆)-carbonyle, cinnamoyle, alcényl(C₃-C₁₂)-carbonyle, alcynyl(C₃-C₁₂)-carbonyle,
alcoxy(C₁-C₁₂)-carbonyle, alcoxy(C₁-C₁₂)-alcoxy(C₁-C₁₂)-carbonyle, aryloxy(C₆-C₁₂)-carbonyle, aralcoxy(C₇-C₁₆)-carbonyle, cycloalcoxy(C₃-C₈)-carbonyle, alcényloxy(C₃-C₁₂)-carbonyle, alcynyloxy(C₃-C₁₂)-carbonyle,
alkyl(C₁-C₁₂)-carbonyloxy, cycloalkyl(C₃-C₈)-carbonyloxy, aryl(C₆-C₁₂)-carbonyloxy, aralkyl(C₇-C₁₆)-carbonyloxy, cinnamoyloxy, alcényl(C₃-C₁₂)-carbonyloxy, alcynyl(C₃-C₁₂)-carbonyloxy,
carbamoyle, N-alkyl(C₁-C₁₂)-carbamoyle, hydroxyalkyl(C₁-C₄)carbamoyle, acyloxy-[alkyl(C₁-C₄)]carbamoyle, N,N-dialkyl(C₁-C₁₂)-carbamoyle, N-cycloalkyl(C₃-C₈)carbamoyle, N-aryl(C₆-C₁₆)-carbamoyle, N-aralkyl(C₇-C₁₆)carbamoyle, N-alkyl(C₁-C₁₀)-N-aryl(C₆-C₁₆)carbamoyle, N-alkyl(C₁-C₁₀)-N-aralkyl(C₇-C₁₆)carbamoyle, N-[alcoxy(C₁-C₁₀)-alkyl(C₁-C₁₀)]carbamoyle, N-[aryloxy(C₆-C₁₆)-alkyl(C₁-C₁₀)]carbamoyle, N-[aralkyloxy(C₇-C₁₆)-alkyl(C₁-C₁₀)]carbamoyle, N-[alkyl(C₁-C₁₀)]-N-[alcoxy(C₁-C₁₀)-alkyl(C₁-C₁₀)]carbamoyle, N-[alkyl(C₁-C₁₀)]-N-[aryloxy(C₆-C₁₆)-alkyl(C₁-C₁₀)]carbamoyle, N-[alkyl(C₁-C₁₀)]-N-[aralkyloxy(C₇-C₁₆)-alkyl(C₁-C₁₀)]carbamoyle,
amino, alkyl(C₁-C₁₂)amino, dialkyl(C₁-C₁₂)amino, cycloalkyl(C₃-C₈) amino, alcényl(C₃-C₁₂)amino, alcynyl(C₃-C₁₂)amino, N-aryl(C₆-C₁₂)amino, N-aralkyl(C₇-C₁₁)amino, N-alkyl(C₁-C₅)-aralkyl(C₇-C₁₀)amino, N-alkyl(C₁-C₅)-aryl(C₆-C₁₂)amino, alcoxy(C₁-C₁₂)amino, alcoxy(C₁-C₁₂)-N-alkyl (C₁-C₁₀)amino,
alcanoyl(C₁-C₁₂)amino, cycloalcanoyl(C₃-C₈)amino, aroyl(C₆-C₁₂)amino, aralcanoyl(C₇-C₁₆)amino, alcanoyl(C₁-C₁₂)-N-alkyl(C₁-C₁₀)amino, cycloalcanoyl(C₃-C₈)-N-alkyl(C₁-C₁₀)amino, aroyl(C₆-C₁₂)-N-alkyl(C₁-C₁₀)amino, aralcanoyl(C₇-C₁₁)-N-alkyl(C₁-C₁₀)amino,
alcanoyl(C₁-C₁₂)amino-alkyle(C₁-C₈), cycloalcanoyl(C₃-C₈)amino-alkyle(C₁-C₈), aroyl(C₆-C₁₆)amino-alkyle(C₁-C₈), aralcanoyl(C₇-C₁₆)amino-alkyle(C₁-C₈), amino-alkyle en C₁-C₁₀, N-alkyl(C₁-C₁₀)amino-alkyle(C₁-C₁₀), N,N-dialkyl(C₁-C₁₀)amino-alkyle(C₁-C₁₀), cycloalkyl(C₃-C₈)amino-alkyle(C₁-C₁₀),
alkyl(C₁-C₁₂)mercapto, alkyl(C₁-C₁₂)sulfinyle, alkyl(C₁-C₁₂)sulfonyle, aryl(C₆-C₁₆)mercapto, aryl(C₆-C₁₆)sulfinyle, aryl(C₆-C₁₆)sulfonyle, aralkyl(C₇-C₁₆)mercapto, aralkyl(C₇-C₁₆)sulfinyle, aralkyl(C₇-C₁₆)sulfonyle,
R⁴ représente un atome d'hydrogène ou un groupe alkyle en C₁-C₄,
R⁵ représente un radical alkyle en C₁-C₄ ramifié ou non ramifié, qui porte un groupe carboxy et qui est une ou deux fois substitué par un ou des atomes de fluor ou de chlore ou groupes hydroxy, alkyle en C₁-C₆, cycloalkyle en C₃-C₈, phényle, benzyle, et
n est 0 ou 1,
f va de 1 à 5,
g est 0 ou va de 1 à (2f + 1) et
x est 0 ou 1.

4. Composés conformes à une ou plusieurs des revendications 1 à 3, selon la formule générale I, dans lesquels
R¹ représente un groupe hydroxy ou alcoxy en C₁-C₆,
R² et R³ représentent des atomes d'hydrogène,
R⁶ représente un atome d'hydrogène ou un cation monovalent ou divalent, physiologiquement utilisable, en particulier Na^{⊕}, K^{⊕}, Mg^{2⊕} ou Ca^{2⊕}, ou un ion ammonium,
R⁷ représente un radical de formule II, à l'exception de -SO₂H,
-Y-[C-U]ᵣ-D-W (II)
dans laquelle
Y représente -SO₂-,
C représente une liaison ou
un radical alcanediyle en C₁-C₆,
U représente une liaison,
r est égal à 1,
D représente une liaison ou un atome d'hydrogène,
W représente un atome d'hydrogène ou le radical phényle, au moins l'une des variables C, D et W ne représentant pas une liaison, et
C, D et/ou W peuvent être substitués par un atome de fluor ou de chlore ou par un groupe trifluorométhyle, alkyle en C₁-C₁₆, cycloalkyle en C₃-C₈, benzyle, phényle, alcoxy en C₁-C₆, phénoxy ou -O-[CH₂]ₓC_{f}H_{(2f+1-g)}F_{g},
carbamoyle, N-alkyl(C₁-C₁₀)-carbamoyle, N,N-dialkyl(C₁-C₈)-carbamoyle, N-cycloalkyl(C₃-C₈)-carbamoyle, N-phénylcarbamoyle, N-phénylalkyl(C₇-C₁₁)-carbamoyle, N-alkyl(C₁-C₈)-N-phényl(C₆-C₁₆)-carbamoyle, N-alkyl(C₁-C₈)-N-phénylalkyl(C₇-C₁₁)-carbamoyle, N-[alcoxy(C₁-C₁₀)-alkyl(C₁-C₁₀)carbamoyle, N-phénoxy-alkyl(C₁-C₈)carbamoyle, N-[phénylalkyloxy(C₇-C₁₆)-alkyl(C₁-C₈) carbamoyle, N-alkyl(C₁-C₈)-N-[alcoxy(C₁-C₆)-alkyl(C₁-C₈)]carbamoyle, N-alkyl(C₁-C₈)-N-[phénoxy-alkyl(C₁-C₈)]carbamoyle, N-alkyl(C₁-C₈)-N-[aralkyloxy(C₇-C₁₆)-alkyl(C₁-C₁₀)]carbamoyle,
alcanoyl(C₁-C₁₀)amino, cycloalcanoyl(C₃-C₈)amino, phénylamino, phénylalcanoyl(C₇-C₁₁)amino, alcanoyl(C₁-C₈)-N-alkyl(C₁-C₁₀)amino, cycloalcanoyl(C₃-C₈)-N-alkyl-(C₁-C₆)amino, benzoyl-N-alkyl(C₁-C₆)amino, phénylalcanoyl(C₇-C₁₁)-N-alkyl(C₁-C₁₀)amino,
alcanoyl(C₁-C₁₀)amino-alkyle(C₁-C₄), cycloalcanoyl(C₃-C₈)amino-alkyle(C₁-C₄), phénylamino-alkyle(C₁-C₆), phénylalcanoyl(C₇-C₁₁)amino-alkyle(C₁-C₆),
les radicaux qui contiennent un fragment aryle pouvant pour leur part être substitués sur le fragment aryle par 1, 2, 3, 4 ou 5 substituants identiques ou différents, choisis parmi des atomes de fluor et de chlore et des groupes hydroxy, trifluorométhyle, carboxy, alcoxy en C₁-C₄, benzyloxy, phénoxy,
alcoxy(C₁-C₁₂)-carbonyle, cycloalkyl(C₃-C₈)-carbonyle phénoxycarbonyle, phénylalcoxy(C₇-C₁₁)-carbonyle,
alkyl(C₁-C₁₂)-carbonyloxy, cycloalkyl(C₃-C₈)-carbonyloxy, benzoyloxy, phénylalkyl(C₇-C₁₁)-carbonyloxy,
carbamoyle, N-alkyl(C₁-C₆)-carbamoyle, N,N-dialkyl(C₁-C₆)-carbamoyle, N-cycloalkyl(C₃-C₈)-carbamoyle, N-phénylcarbamoyle, N-phénylalkyl(C₇-C₁₁)-carbamoyle, hydroxyalkyl(C₁-C₄)-carbamoyle, acyloxyalkyl(C₁-C₄)-carbamoyle,
carbamoyloxy, N-alkyl(C₁-C₆)-carbamoyloxy, N,N-dialkyl(C₁-C₆)-carbamoyloxy, N-cycloalkyl(C₃-C₈)-carbamoyloxy,
R⁴ représente un atome d'hydrogène,
R⁵ représente un groupe méthyle qui est substitué par le groupe carboxy et porte un autre substituant choisi parmi des groupes hydroxy, hydroxyalkyle en C₁-C₄, phényle, benzyle, alcoxy en C₁-C₄, alkyle en C₁-C₅, benzyloxy, phénoxy, et
n est 0 ou 1,
f va de 1 à 5,
g est 0 ou va de 1 à (2f + 1) et
x est 0 ou 1.

5. Composés selon une ou plusieurs des revendications 1 à 4, de formule générale I, dans lesquels
R¹ représente un groupe hydroxy ou alcoxy en C₁-C₆,
R² et R³ représentent des atomes d'hydrogène,
R⁶ représente un atome d'hydrogène ou un cation monovalent ou divalent, physiologiquement utilisable, en particulier Na^{⊕}, K^{⊕}, Mg^{2⊕} ou Ca^{2⊕}, ou un ion ammonium, en particulier H₃N^{⊕}C(CH₂OH)₃,
R⁷ représente un radical de formule II, à l'exception de -SO₂H,
-Y-[C-U]ᵣ-D-W (II)
dans laquelle
Y représente -SO₂-,
C représente une liaison ou
un radical alcanediyle en C₁-C₁₆,
U représente une liaison,
r est égal à 1,
D représente une liaison ou un atome d'hydrogène,
W représente un radical phényle, W pouvant être deux fois substitué par un ou des atomes de fluor ou de chlore ou groupes alkyle en C₁-C₆, aralkyle en C₇-C₁₆, phényle, alcoxy en C₁-C₆, et W étant en outre mono-substitué par un groupe phényle, phénoxy, O-[CH₂]ₓC_{f}H_{(2f+1-g)}F_{g},
carbamoyle, N-alkyl(C₁-C₁₀)-carbamoyle, N,N-dialkyl(C₁-C₈)-carbamoyle, N-cycloalkyl(C₃-C₈)-carbamoyle, N-phénylcarbamoyle, N-phénylalkyl(C₇-C₁₁)-carbamoyle, N-[alcoxy(C₁-C₄)-alkyl(C₁-C₄)]-carbamoyle, N-phénoxyalkyl(C₁-C₄)]-carbamoyle,
alcanoyl(C₁-C₁₀)amino-alkyle(C₁-C₂), cycloalcanoyl(C₃-C₈)amino-alkyle (C₁-C₂), benzoylamino-alkyle(C₁-C₂) ou phénylalcanoyl(C₇-C₁₁)amino-alkyle(C₁-C₂),
les radicaux qui contiennent un fragment aryle pouvant pour leur part être substitués sur le fragment aryle par 1, 2, 3, 4 ou 5 substituants identiques ou différents choisis parmi les atomes de fluor et de chlore et des groupes hydroxy, carboxy, alcoxy en C₁-C₄, phénoxy, benzyloxy, trifluorométhyle,
carbamoyle, N-alkyl(C₁-C₆)-carbamoyle, N,N-dialkyl(C₁-C₆)-carbamoyle, N-cycloalkyl(C₃-C₈)-carbamoyle, N-phénylcarbamoyle, N-phénylalkyl(C₇-C₁₁)-carbamoyle,
R⁴ représente un atome d'hydrogène,
R⁵ représente un groupe méthyle qui est substitué par le groupe carboxy, et
n est égal à 0,
f va de 1 à 5,
g est 0 ou va de 1 à (2f + 1) et
x est 0 ou 1.

6. Procédé pour la préparation des composés selon une ou plusieurs des revendications 1 à 5, dans lesquels, dans la formule I, R¹ à R⁷ ont les significations selon les revendications 1 à 5, caractérisé en ce que l'on fait réagir des esters d'acide pyridine-2-carboxylique-carboxylates de formule 7 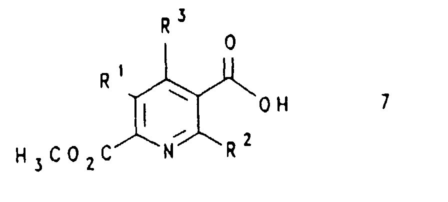 avec un dérivé de type sulfonamide de formule 8
R⁶HN-R⁷ 8
et on fait réagir les composés ainsi obtenus avec une amine de formule 5
HNR⁴R⁵ 5
pour aboutir aux composés de formule Ia' 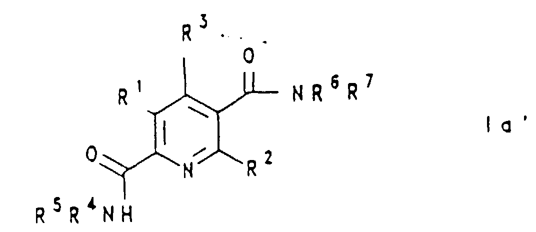 en effectuant éventuellement ensuite une oxydation pour aboutir au N-oxyde de pyridine de formule Ib'

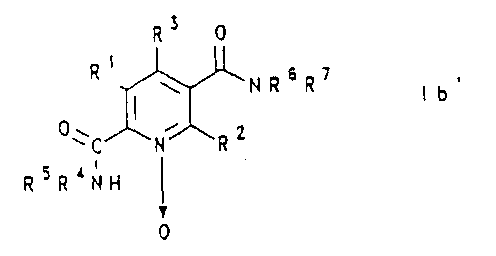

7. Composés selon les revendications 1 à 5, pour utilisation contre des maladies fibreuses.

8. Composés selon les revendications 1 à 5, pour utilisation en tant que fibrosuppresseurs.

9. Composés selon les revendications 1 à 5, pour l'inhibition de la prolylhydroxylase.

10. Composés selon les revendications 1 à 5, pour utilisation en tant qu'inhibiteurs de la biosynthèse du collagène.

11. Médicament, contenant au moins un composé selon la revendication 1 et éventuellement un véhicule pharmaceutiquement acceptable.

12. Composés selon la revendication 1, pour utilisation dans le traitement de troubles du métabolisme du collagène et de substances de type collagène.

13. Composés selon la revendication 1, pour utilisation dans le traitement de maladies fibreuses.

14. Procédé pour la fabrication de médicaments destinés au traitement de maladies fibreuses, caractérisé en ce que le médicament contient un composé selon la revendication 1.
